(19) Europäisches Patentamt / European Patent Office / Office européen des brevets

(11) **EP 4 343 772 A1**

(12) **EUROPEAN PATENT APPLICATION**
published in accordance with Art. 153(4) EPC

(43) Date of publication:
**27.03.2024 Bulletin 2024/13**

(21) Application number: **22804734.6**

(22) Date of filing: **18.05.2022**

(51) International Patent Classification (IPC):
**G16B 40/30** (2019.01)

(52) Cooperative Patent Classification (CPC):
**G16B 40/30; Y02A 90/10**

(86) International application number:
**PCT/JP2022/020744**

(87) International publication number:
**WO 2022/244824 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.05.2021 JP 2021084114**

(71) Applicant: **KARYDO THERAPEUTIX, INC.**
**Tokyo 102-0082 (JP)**

(72) Inventor: **SATO, Narutoku**
**Soraku-gun, Kyoto 619-0288 (JP)**

(74) Representative: **Maiwald GmbH**
**Elisenhof**
**Elisenstraße 3**
**80335 München (DE)**

(54) **METHOD, DEVICE, AND PROGRAM FOR SEARCHING FOR NEW DIAGNOSTIC BIOMARKER AND/OR THERAPEUTIC TARGET**

(57) A method of searching for novel diagnostic biomarkers and/or therapeutic targets, comprising performing a topic analysis using a topic model on an omics database derived from two or more biofeatures linked to information indicating a disease name, to estimate missing biofeatures for a particular disease, wherein the estimated missing biofeatures suggest the novel diagnostic biomarkers and/or therapeutic targets, is provided.

Fig. 4

```
                    ┌─────────┐
                    │  start  │
                    └─────────┘
                         │
                         ▼
   ┌──────────────────────────────────────────────┐
   │  accept a selection request for an omics       │   S11
   │  database and acquire a corresponding database │
   └──────────────────────────────────────────────┘
                         │
                         ▼
   ┌──────────────────────────────────────────────┐
   │  estimate missing biofeatures for a            │   S12
   │  particular disease                            │
   └──────────────────────────────────────────────┘
                         │
                         ▼
   ┌──────────────────────────────────────────────┐
   │  output the missing biofeatures                │   S13
   └──────────────────────────────────────────────┘
                         │
                         ▼
                    ┌─────────┐
                    │   end   │
                    └─────────┘
```

EP 4 343 772 A1

**Description**

Technical Field

Disclosed herein are a method, device and program of searching for novel diagnostic biomarkers and/or therapeutic targets.

Background Art

Computational methods for predicting potential biomarkers and/or therapeutic targets for human diseases have revolutionized medicine and drug development. One of such approaches is graph theory-based methods. This builds a network of diseases on the basis of a single type of disease-associated information, such as genetic influences on diseases, to allow for detecting genetic biomarkers according to the genetic relevance of the diseases. The same framework can also be used to predict novel therapeutic targets of diseases on the basis of other information, such as drug targets. Another approach is based on natural language processing models (Non-Patent Literatures 1 and 2). This method builds a model for searching for "likely" biomarkers and therapeutic targets by using descriptions of biological processes in numerous publications and other documents.

Citation List

Non Patent Literature

Non Patent Literature 1: Gyori, B.M., et al. (2017). Molecular systems biology 13, 954.
Non Patent Literature 2: Haggerty, R.A., and Purvis, J.E. (2017). Molecular systems biology 13, 958.

Summary of Invention

Technical Problem

The above approaches are common and, in some cases, effective in finding biomarkers and therapeutic targets. However, diseases are influenced by many factors, including genes, proteins, cell-cell interactions, lifestyle, environment, microbiota in the human body, and the like. Hence, it would be more useful to have a method that can identify latent disease-disease associations based on a plurality of disease-omics data. Such an approach may facilitate the discovery of biomarkers and/or therapeutic targets in the case where a plurality of disease-associated factors caused the disease. For example, a close disease-disease relationship based on genetics could be used to discover lifestyle biomarkers for the same diseases. A close disease-microbiota relationship may be translated into related drug targets.
An object of the present invention is to provide a framework for searching for biomarkers and/or therapeutic targets based on a plurality of types of disease-omics data.

Solution to Problem

The present invention may include following embodiments.

Item 1.

A method of searching for novel diagnostic biomarkers and/or therapeutic targets, comprising

performing a topic analysis using a topic model on an omics database derived from two or more biofeatures linked to information indicating a disease name to estimate missing biofeatures for a particular disease, wherein
the estimated missing biofeatures suggest the novel diagnostic biomarkers and/or therapeutic targets.

Item 2.

The method according to item 1, wherein the two or more biofeatures are selected from single nucleotide polymorphisms, microbiota, drug targets, transcriptome, proteome and disease biomarkers.

Item 3.

The method according to item 1, wherein the topic model is based on Latent Dirichlet Allocation model.

Item 4.

The method according to item 1, further comprising verifying the reliability of the extracted missing biofeatures.

Item 5.

A device of searching for novel diagnostic biomarkers and/or therapeutic targets provided with a control unit, wherein

the control unit performs a topic analysis using a topic model on an omics database derived from two or more biofeatures linked to information indicating a disease name to estimate missing biofeatures for a particular disease, wherein
the estimated missing biofeatures suggest the novel diagnostic biomarkers and/or therapeutic targets.

Item 6.

A program of searching for novel diagnostic biomarkers and/or therapeutic targets that, when executed by a computer, causes the computer to execute a step of performing a topic analysis using a topic model on an omics database derived from two or more biofeatures linked to information indicating a disease name to estimate missing biofeatures for a particular disease, wherein
the estimated missing biofeatures suggest the novel diagnostic biomarkers and/or therapeutic targets.

Item 7.

A method of using relationship between each disease and combination of tissue and gene corresponding to each disease listed in Listing 3 in the present description as a candidate for disease biomarkers and/or therapeutic targets.

Advantageous Effects of Invention

A framework for searching for novel diagnostic biomarkers and/or therapeutic targets is provided.

Brief Description of Drawings

FIG. 1 shows a configuration of a main database.
FIG. 2 shows a concept of a topic model.
FIG. 3 shows a hardware configuration of search device 10.
FIG. 4 shows a flow of processing in search program 1042.
FIG. 5 shows validation results of leave one-disease features out cross-validation.
FIG. 6-1 shows candidates for novel diagnostic biomarkers and/or therapeutic targets.
FIG. 6-2 shows candidates for the novel diagnostic biomarkers and/or therapeutic targets.
FIG. 6-3 shows candidates for the novel diagnostic biomarkers and/or therapeutic targets.
FIG. 6-4 shows candidates for the novel diagnostic biomarkers and/or therapeutic targets.
FIG. 6-5 shows candidates for the novel diagnostic biomarkers and/or therapeutic targets.
FIG. 6-6 shows candidates for the novel diagnostic biomarkers and/or therapeutic targets.

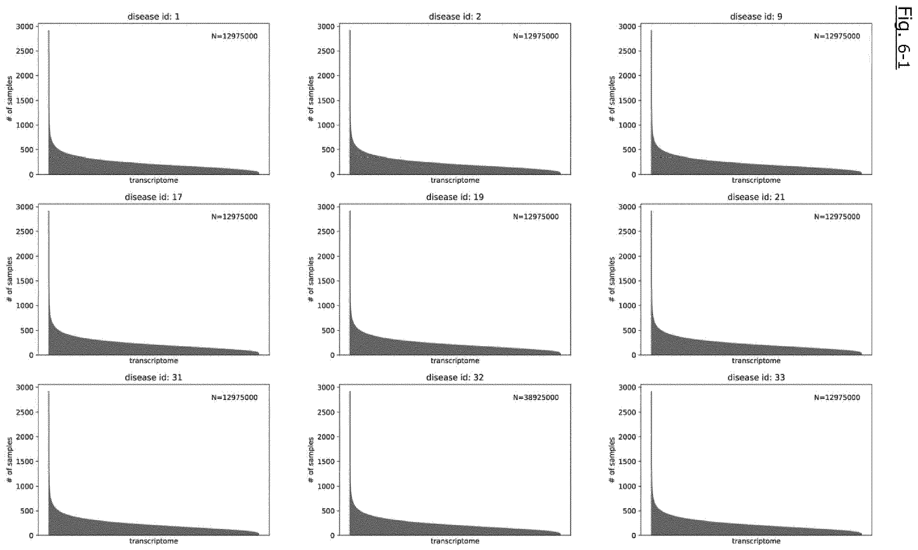

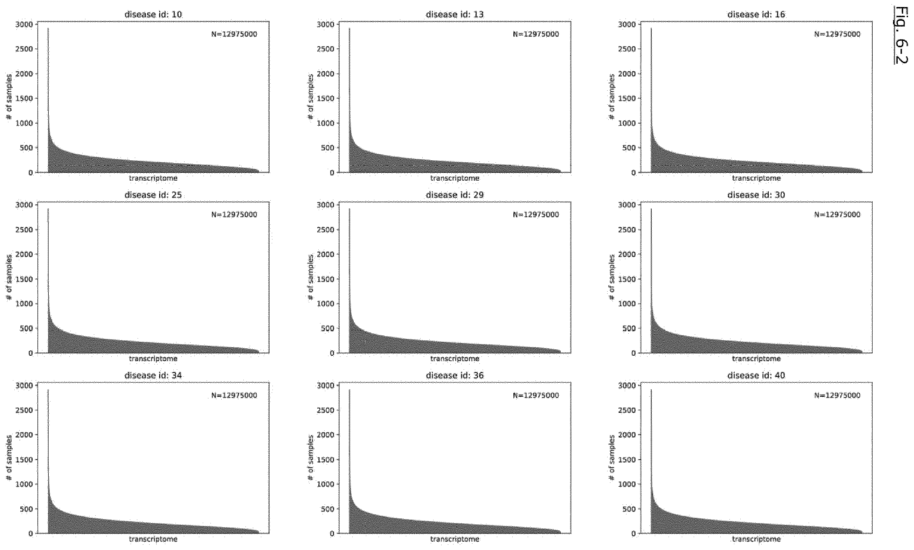

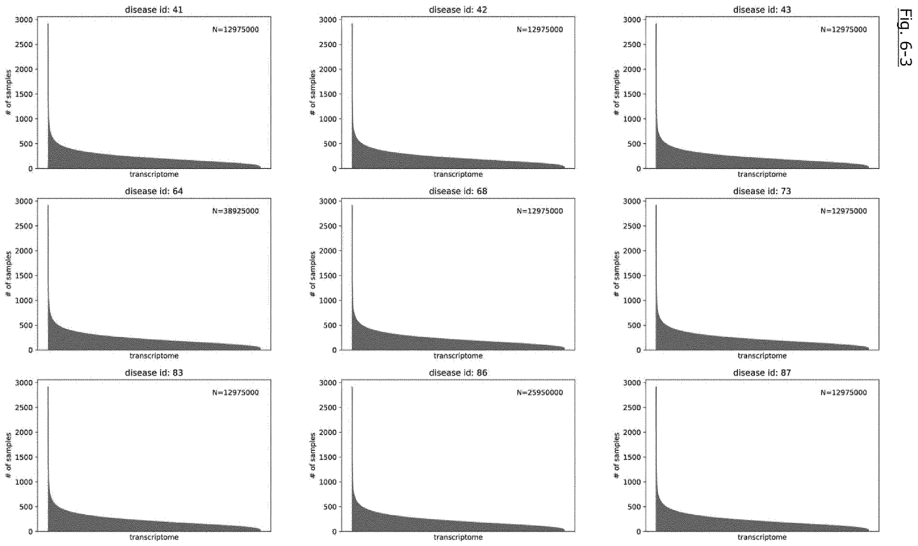

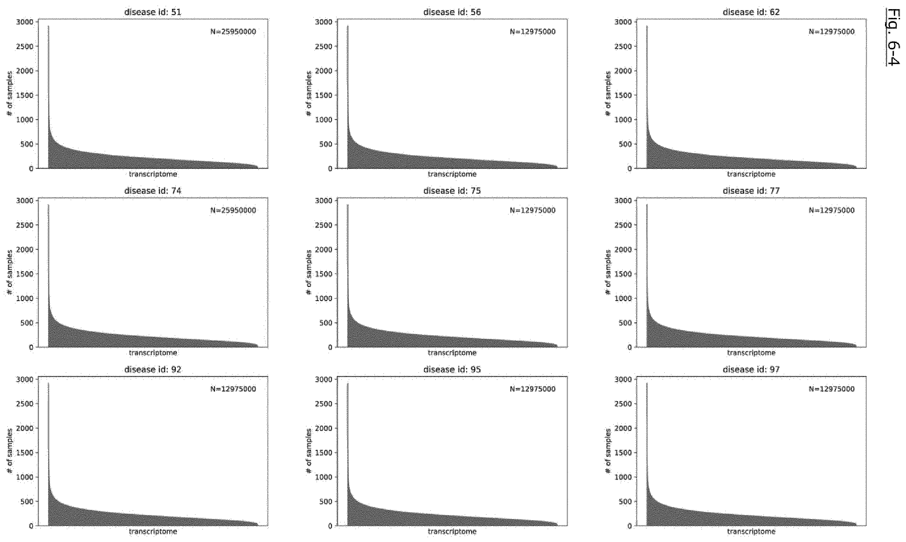

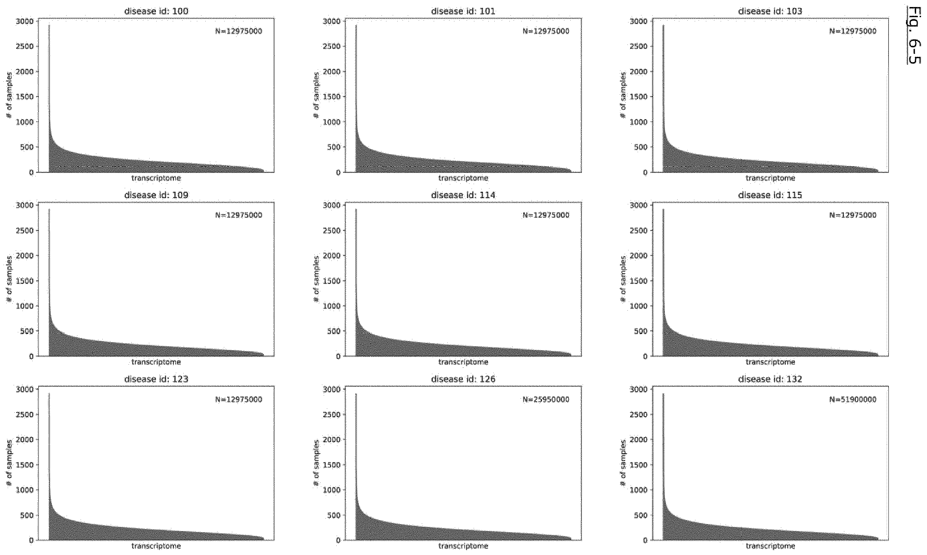

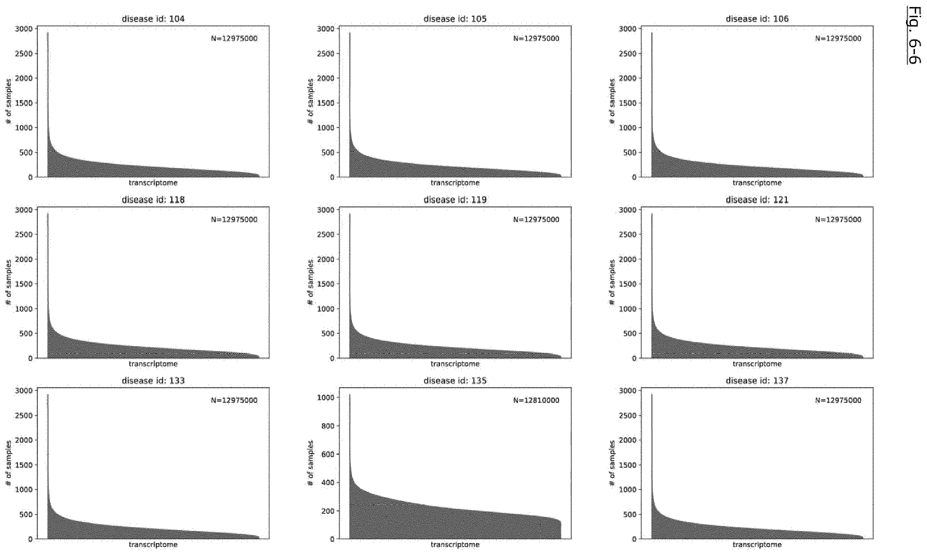

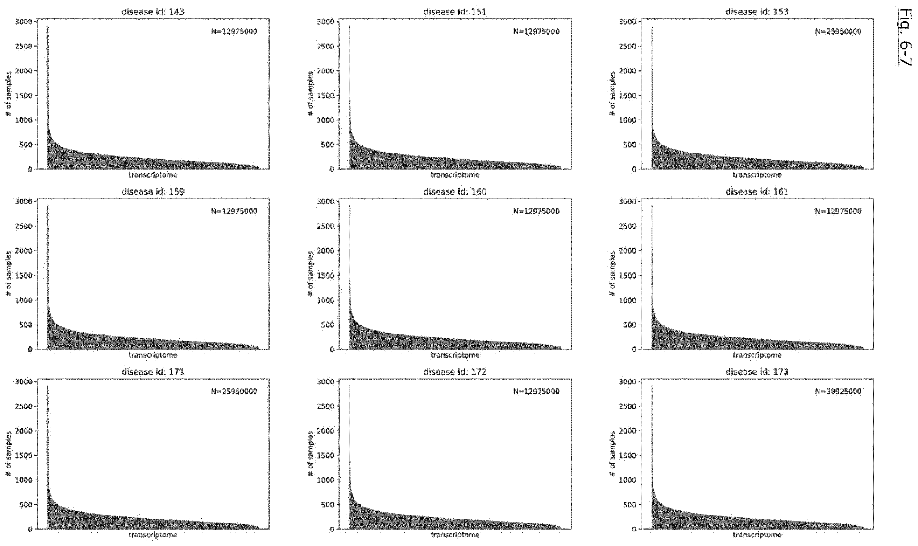

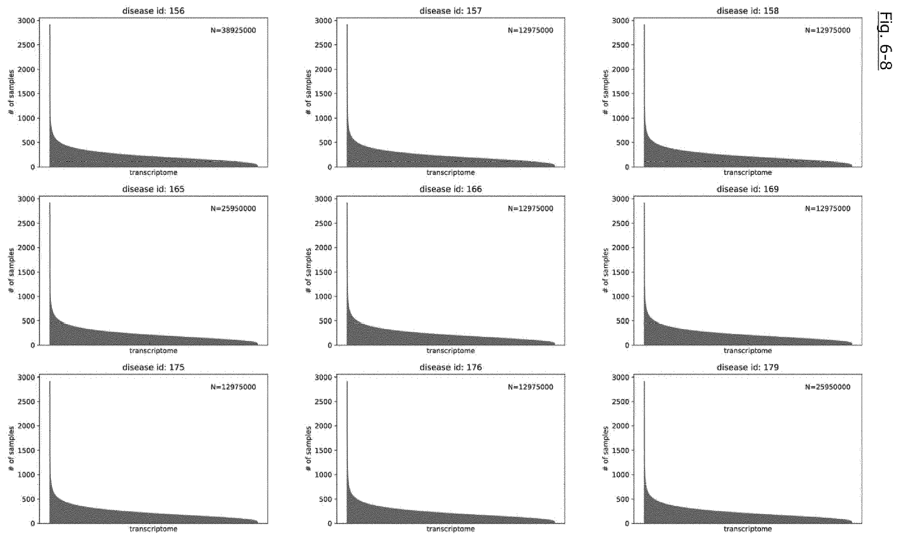

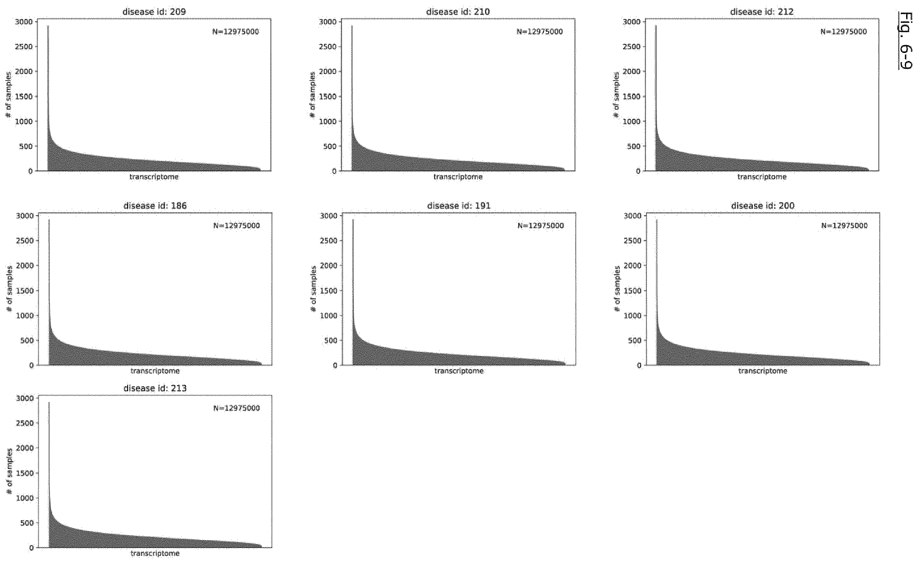

FIG. 6-7 shows candidates for the novel diagnostic biomarkers and/or therapeutic targets.
FIG. 6-8 shows candidates for the novel diagnostic biomarkers and/or therapeutic targets.
FIG. 6-9 shows candidates for the novel diagnostic biomarkers and/or therapeutic targets.

Description of Embodiments

1. Search of novel diagnostic biomarkers and/or therapeutic targets

One embodiment of the present invention relates to search of novel diagnostic biomarkers and/or therapeutic targets.

1-1. Main database

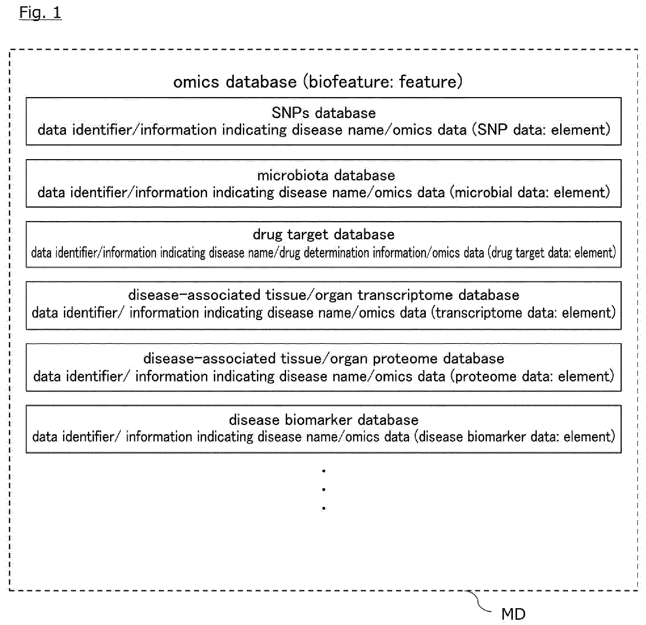

In this embodiment, main database MD is first prepared. The main database MD stores omics databases derived from different biofeatures, as shown in FIG. 1. The biofeatures are intended to be biological indicators associated with living organisms. An omics data is intended to be an individual omics data belonging to each biofeature. That is, the term 'biofeature' encompasses and describes certain biological indicators as features, as well as may include the individual omics data (the elements that make up the feature) that make up the feature. The language in which the database is structured is not limited, but is preferably English. However, the omics data corresponding to information indicating a disease name may be singular or plural. Hereafter, this definition is invoked herein.

The biofeature, for example, may be single nucleotide polymorphisms (SNPs), microbiota, drug target, transcriptome, proteome, disease biomarker transcriptome, proteome, disease biomarkers, or the like. In FIG. 1, illustratively, SNPs database, Microbiota database, drug target database, disease-associated tissue/organ transcriptome database, disease-associated tissue/organ proteome database, and disease biomarker database are shown.

Here, the individual omics data contained in each database is linked to the information indicating the disease names. In addition, the information indicating the disease name is assigned a data identifier to identify the information indicating the individual disease.

The information indicating the disease name includes information that can at least identify the disease name. The information that can identify the disease name may include the disease name itself, an abbreviation, the disease name and its type or stage, and so on. The term "disease" used in the present description may also include symptoms in addition to those defined as a disease.

For example, the SNPs database stores one data unit or a plurality of data units, wherein the one data unit is a series of data linking the information indicating the disease name to which the data identifier is assigned and the omics data, i.e., SNP data or SNPs data, linked to the information indicating the disease name, and the plurality of data units correspond to information indicating different disease names. The SNP data is, for example, accession number (SNP_GENE_IDS*: Entrez Gene ID), rs number, or chromosome number associated with the rs number. Disease-associated SNPs database can be obtained, for example, from the GWAS Catalog (https://www.ebi.ac.uk/gwas/docs/file-downloads) under "All associations" (v1.0. 2).

For example, the microbiota database stores one data unit or a plurality of data units, wherein the one data unit is a series of data linking the information indicating the disease name to which the data identifier is assigned, and the omics data, i.e., microbial data, linked to the information indicating the disease name, and the plurality of data units correspond to information indicating different disease names. The microbial data is, for example, the name of a microorganism and the like. Disease-associated microbiota database can be obtained, for example, from Disbiome (version on 11th of November 2020, https://disbiome.ugent.be/home) (Ref: Janssens et al., 2018, Disbiome database: linking the microbiome to disease. BMC Microbiol 18, 50.).

For example, the drug target database stores one or a plurality of data units, wherein the one data unit is a series of data linked to the information indicating the disease name to which the data identifier is assigned, drug determination information linked to the information indicating the disease name, and the omics data, i.e., drug target data, linked to the drug determination information, and the plurality of data units correspond to information indicating different disease names. The drug determination information is, for example, a drug name, a compound name, and the like. The drug target data is, for example, the name of a target molecule, and the like (protein name, cytokine name, chemokine name, and the like). In the drug target database, by first, "therapeutic-targets (i.e., diseases) information for drugs from SIDER (version 4.1) (http://sideeffects.embl.de)", for example, is downloaded, then "drug-targets from DrugBank (https://go.drugbank.com/)" is searched to associate the drug determination information linked to the information indicating the disease name with the drug target data linked to the drug determination information. This can be done, for example, using web-scraping on Python (version 3.8.1).

For example, a disease-associated tissue/organ transcriptome database stores one data unit or a plurality of data units, wherein the one data unit is a series of data linked to the information indicating the disease name to which the data identifier is assigned, and the omics data, i.e., transcriptome data, linked to the information indicating the disease name, and the plurality of data units correspond to information indicating different disease names. The transcriptome data is the name of an organ and/or tissue, and the transcriptome behavior data for each organ and/or tissue, i.e., various gene names and expression levels thereof, and the like.

The tissue/organ transcriptome database can be generated by the following procedure. For example, NCBI GEO DataSets (https://www.ncbi.nlm.nih.gov/gds) can be collected using a keyword. The keyword can be, for example, '"RNA-seq"[All Fields] AND "Homo sapiens"[organism] NOT "scRNA" [All Fields] NOT "Single cell" [All Fields] NOT "culture"[All Fields] NOT "cultured"[All Fields] NOT "cell line" [All Fields] NOT "treatment"[All Fields] NOT "single-nucleus"[All Fields] NOT "microRNA"[All Fields] NOT "iPSC"[All Fields] NOT "organoids"[All Fields] AND "expression profiling by high throughput sequencing"[DataSet Type] AND "SIDER's indication"[All Fields]'. In this case, only the input term "SIDER's indication" may be automatically converted into the above keywords. The "SIDER's indication" list is available for download from SIDER 4.1 (http://sideeffects.embl.de). After making the initial selection of GEO datasets described above automatically, GEO datasets containing both Human disease-associated bulk tissue/organ transcriptome data and control or healthy data in the same project are manually selected, and consequently, for example, 50 GEO datasets useful for this analysis are obtained. Examples of selected datasets are given in <List 1: GEO datasets> at the end of the present description.

Raw data for each selected GEO dataset can then also be generated. For example, the raw data for 17 GEO datasets (GSE107638, GSE112057, GSE112523, GSE114007, GSE117970, GSE121212, GSE124439, GSE131031, GSE131525, GSE133624, GSE134056, GSE138082, GSE138614, GSE146889, GSE42546, GSE80336, GSE80655) can be obtained from NCBI GEO DataSets websites. The raw data for the remaining 33 GEO datasets can be generated from FASTQ data. To briefly describe the generation of raw data from FASTQ data, at the first, the FASTQ data is downloaded from European Nucleotide Archive (ENA; https://www.ebi.ac.uk/ena/browser/home) via FTP downloading or the fast file-downloading software, Aspera Connect (https://www.ibm.com/aspera/connect/; version 3.11.1.58). Next, in order to generate BAM data, for example, the downloaded FASTQ data is mapped onto human genome (UCSC hg 38) with STAR (version 2.7.8a). The generated BAM data is converted into raw count data by, for example, the human gene GTF file (UCSC hg 38). Specifically, this data conversion was performed by using the software of Subread (version 2.0.1), where the 'featureCounts' command is used with specifying the option '-Q10' and, only in the case that the data is paired-end, additionally with specifying the options, '-p' and '-B'. The processing of STAR and Subread can be performed on Linux of CentOS6 or CentOS7. Since some raw count data is stored as split FASTQ data, a plurality of raw count data may be combined into one by GSM ID, if necessary.

Next, differentially expressing genes (DEGs) are extracted. The extraction of the DEGs can be performed with DESeq2 (version 1.30.1) (Ref: Love et al., 2014, Moderated estimation of fold change and dispersion for RNA-seq data with DESeq2. Genome biology 15, 550) on free software R (version 4.0.3). DESeq2 analysis is conducted for the respective GSE project. First, transcriptome data is classified into a control group obtained from healthy tissue or normal tissue parts and a disease group derived from lesion site of each tissue. This classification can be performed on sample information described in NCBI GEO datasets. Then, DESeq2 analysis is performed for each disease in each GSE project. To select DEGs, genes are typically filtered by an adjusted p-value of less than 0.0001. However, if filtering by the adjusted p-value of less than 0.0001 does not yield any DEGs, the adjusted p-value threshold may be increased to 0.0001, 0.001, 0.01, or 0.05 until at least one DEG is obtained. Furthermore, tissue/organ names are manually unified into a rough class.

The tissue/organ proteome database stores one data unit or a plurality of data units, wherein the one data unit is a series of data linking the information indicating the disease name to which the data identifier is assigned, and the omics data, i.e., proteome data, linked to the information indicating the disease name, and the plurality of data units correspond to information indicating different disease names. The proteome data is, for example, the name of an organ and/or tissue, and the proteome behavior data for each organ and/or tissue, and the like.

The disease biomarker database stores one data unit or a plurality of data units, wherein the one data unit is a series of data linking the information indicating the disease name to which the data identifier is assigned, and the omics data, i.e., disease biomarker data, linked to the information indicating the disease name, and the plurality of data units correspond to information indicating different disease names. The disease biomarker data is, for example, data linking a specific disease to a biomarker name and its behavior, and to an organ or tissue name if necessary.

Here, the information indicating the name of the disease linked to each omics data and the data identifier assigned to it are unified across all databases. For example, the data identifier can be a Unified Medical Language System (UMLS) ID or the actual disease name. Specifically,

Unified Medical Language System (UMLS) IDs are added to disease names to combine the disease names acquired from a group of a plurality of independent data sources. The UMLS ID annotation can be performed, for example, by using 'UMLS_AUI.extract_terminology ("ICD10")" function derived from python library PyMedTermino (version 0.3.3) (Ref: Lamy et al., 2015, Stud Health Technol Inform 210, 924-928). Prior to executing this function, replace the string "'s' in the disease name is replaced with a blank space. Following assigning the UMLS ID annotations, if the UMLS IDs have the same disease name, the UMLS IDs are combined with the string '|'. For disease names for which this UMLS ID annotation failed, the actual disease name in the dataset is used and only those with the exact matching name are combined.

1-2. Topic model

A topic model used in this embodiment is not limited as long as the information indicating the disease name and the omics data can be analyzed by linguistic analysis.

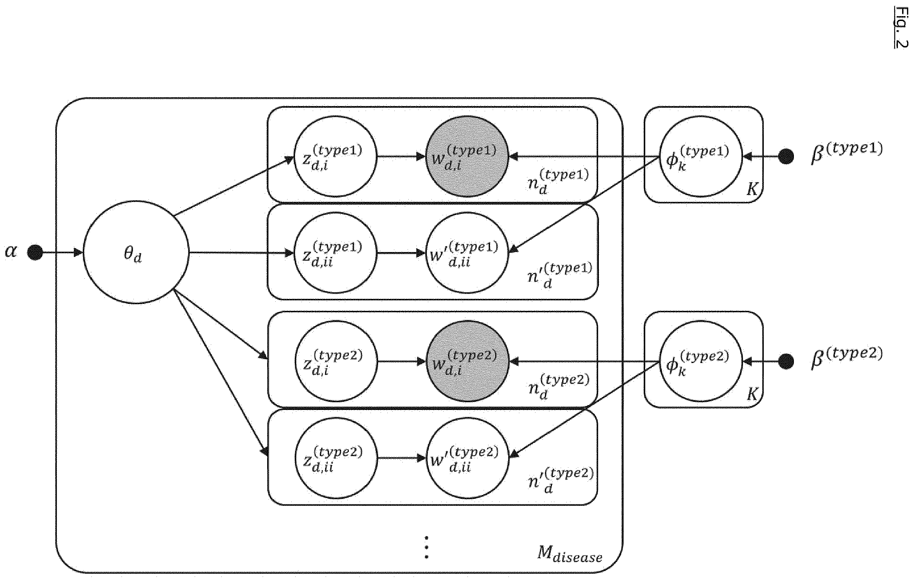

Here, an example of a model in which the Latent Dirichlet Allocation (LDA) model (Ref: Blei et al., 2003, Journal of Machine Learning Research 3, 993-1022), that is a conventional topic model for bag-of-words data, is extended to be able to classify diseases according to, for example, omics data derived from different biofeatures, is presented. This model is referred to herein as an extended LDA model. FIG. 2 shows the concept of the extended LDA model. In FIG. 2, empty circles represent random variables, grey circles represent observed/input data and black dots represent hyper-parameters.

In the extended LDA model, $w_{di}^{(type)}$ represents the i-th information of disease d acquired from (type) information. $z_{di}^{(type)}$ represents the topic number of $w_{di}^{(type)}$. $\theta_d$ represents the topic probability of disease d. $\phi_k^{(type)}$ represents the word probability of topic k in the (type) information. The total number of topic K is a hand-tuned parameter.

Wherein, the "type" is SNPs, drug-targets, microbiota, transcriptome, or the like. For example, the K can be set to "3". Examples of methods to determine the number of topics include Perplexity, community detection, and the like.

The generative model of proposed topic models was defined as following.

Mathematical formula 1:

$$p\left(w_{di}^{(type)}\middle| z_{di}^{(type)}, \boldsymbol{\phi}^{(type)}\right) = Multinomial\left(w_{di}^{(type)}\middle| \boldsymbol{\phi}^{(type)}\right)$$

$$= \prod_{v=1}^{V^{(type)}} \prod_{k=1}^{K} \phi_{vk}^{(type)\,\delta\left(z_{di}^{(type)}=k\right)\delta\left(w_{di}^{(type)}=v\right)}$$

$$p\left(z_{di}^{(type)}\middle| \boldsymbol{\theta}_d\right) = Multinomial\left(z_{di}^{(type)}\middle| \boldsymbol{\theta}_d\right) = \prod_{k=1}^{K} \theta_{dk}^{\delta\left(z_{di}^{(type)}=k\right)}$$

$$p(\boldsymbol{\theta}_d|\boldsymbol{\alpha}) = Dirchlet(\boldsymbol{\theta}_d|\boldsymbol{\alpha}) = \frac{\Gamma(\sum_k \alpha_k)}{\prod_k \Gamma(\alpha_k)} \prod_{k=1}^{K} \theta_{dk}^{\alpha_k-1}$$

$$p\left(\boldsymbol{\phi}_k^{(type)}\middle| \boldsymbol{\beta}^{(type)}\right) = Dirichlet\left(\boldsymbol{\phi}_k^{(type)}\middle| \boldsymbol{\beta}^{(type)}\right) = \frac{\Gamma\left(\sum_v \beta_v^{(type)}\right)}{\prod_v^{V^{(type)}} \Gamma\left(\beta_v^{(type)}\right)} \prod_{v=1}^{V^{(type)}} \phi_{kv}^{(type)\,\beta_v^{(type)}-1}$$

Wherein, $\boldsymbol{\alpha} \in \mathbb{R}^{+K}$ and $\boldsymbol{\beta}^{(type)} \in \mathbb{R}^{+V^{(type)}}$ are the hyperparameters and set to a vector with elements of 0.1. In addition, v is an index word, $V^{(type)}$ is all types of words in a (type) data score. $\Gamma(\cdot)$ is the Gamma function and $\delta(\cdot)$ is the Kronecker delta function.

Based on this model, the variables $z_{di}^{(type)}$, $\theta_d$ and $\phi_k^{(type)}$ are estimated. Some $w_{di}^{(type)}$, which are some (type) data sources using Gibbs sampling method, are also estimated based on the above model.

Conditional distributions are calculated based on, for example, Gibbs sampling method technique as described below.

Mathematical formula 2:

$$p\left(z_{di}^{(type)} = k \middle| w_{di}^{(type)} = v, \boldsymbol{\theta}_d, \boldsymbol{\phi}^{(type)}\right) = \frac{\theta_{dk}\phi_{kv}^{(type)}}{\sum_{k'} \theta_{dk'}\phi_{k'v}^{(type)}}$$

$$p\left(\boldsymbol{\theta}_d \middle| \mathbf{z}^{(type)}, \boldsymbol{\alpha}\right) = Dirichlet(\boldsymbol{\theta}_d | \boldsymbol{\alpha}') \propto \prod_{k=1}^{K} \theta_{dk}^{\alpha_k + \sum_t \sum_i \delta\left(z_{di}^{(type)} = k\right) - 1}$$

$$p\left(\boldsymbol{\phi}_k^{(type)} \middle| \mathbf{z}^{(type)}, \mathbf{w}^{(type)}, \boldsymbol{\beta}^{(type)}\right) = Dirichlet\left(\boldsymbol{\phi}_k^{(type)} \middle| \boldsymbol{\beta}^{(type)'}\right)$$

$$\propto \prod_v \phi_{kv}^{(type)\beta_k^{(type)} + \sum_d \sum_i \delta\left(z_{di}^{(type)} = k\right) \delta\left(w_{di}^{(type)} = v\right) - 1}$$

$$p\left(w_{di}^{(type)} = v \middle| z_{di}^{(type)} = k, \boldsymbol{\phi}^{(type)}\right) = \phi_{kv}^{(type)}$$

Here, from this conditional distribution, sampling is repeated for samples $z_{di}^{(type)}$, $\theta_d$, and $\boldsymbol{\phi}_k^{(type)}$, and $w_{di}^{(type)}$, until they all converge. For example, the number of iterations is set to 5,000, which is determined manually. The initial value of $\theta_d$ and $\boldsymbol{\phi}_k^{(type)}$ are set to uniform distribution. The initial value of $w_{di}^{(type)}$ is set to a randomly sampled word from $\boldsymbol{\phi}_k^{(type)}$. The total number of $w_{di}^{(type)}$ at disease d is determined by sampling from binomial distribution. The parameters of the binomial distribution can be estimated by maximum likelihood estimation method by using only observed data.

The estimated variables $z_{di}^{(type)}$, $\theta_d$ and $\boldsymbol{\phi}_k^{(type)}$ can be predicted by averaging sampled variables.

1-3. Search device

One embodiment of the present invention relates to a search device 10 for novel diagnostic biomarkers and/or therapeutic targets (hereinafter simply referred to as "search device 10").

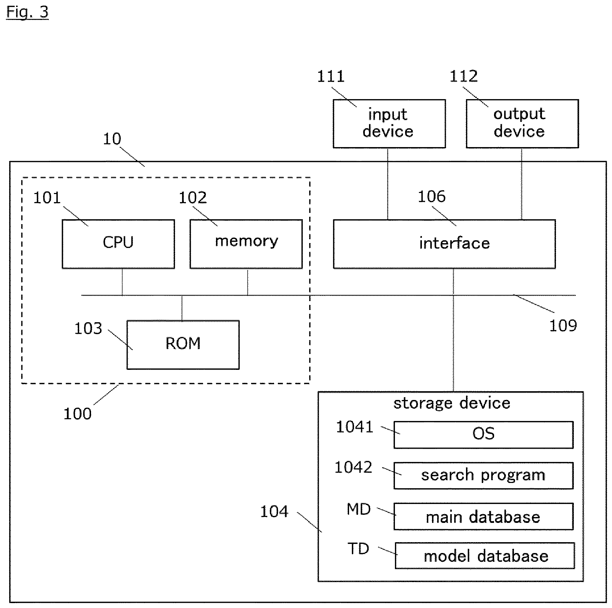

FIG. 3 shows a hardware configuration of the search device 10.

The search device 10 may be connected to an input device 111 and an output device 112.

In the search device 10, a processing unit (CPU) 101, memory 102, ROM (read only memory) 103, storage device 104, and interface 106 are connected to each other by a bus 109 so as to enable data communication. The processing unit 101, memory 102, and ROM 103 function as a control unit 100 of the search device 10.

The processing unit 101 is the CPU of the search device 10, also referred to as an arithmetic unit. The processing unit 101 may cooperate with a GPU or MPU. A computer functions as the search device 10 when the processing unit 101 executes a search program 1042 described below in cooperation with an operation system (OS) 1041 stored in the storage device 104 or ROM 103.

The ROM 103 stores the search program 1042 executed by the processing unit 101 and data used in it. The ROM 103 stores a boot program executed by the processing unit 101 when the search device 10 is started and programs and settings related to an operation of the hardware of the search device 10.

The storage device 104 non-volatilely stores the operation system (OS) 1041, the search program 1042 for novel diagnostic biomarkers and/or therapeutic targets (hereinafter simply referred to as "search program 1042") described below, the main database MD described in the above 1-1. and a model database TD storing the topic models described in the above 1-2.. The storage device 104 may also store analysis results.

The input device 111 is composed of a touch panel, keyboard, mouse, pen tablet, microphone, and the like, and provides text or voice input to the search device 10. The input device 111 may be connected from outside the search device 10 or may be integrated with the search device 10.

The output device 112 is composed of, for example, a display, printer, and the like.

The processing unit 101 may obtain an application software and various settings necessary to control the search device 10 via a network, instead of reading them from the ROM 103 or storage device 104. It is also possible for said application program to be stored in a storage device of a server computer on the network, and for the search device 10 to access this server computer and download the search program 1042, which is then stored in the ROM 103 or storage device 104.

An operating system providing a graphical user interface environment, for example, Windows® manufactured and sold by Microsoft Corporation in the United States, open source Linux, or the like, is installed in the ROM 103 or storage device 104. The search program runs on said operating system. Here, the search device 10 can be a personal computer or the like.

1-4. Processing in search program

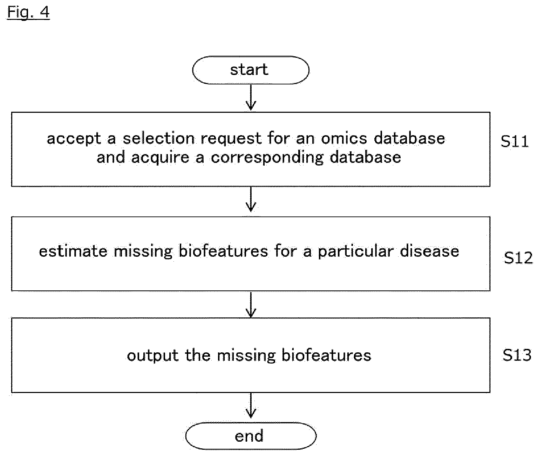

Referring to FIG. 4, the processing performed by the search program 1042 is described. The method of searching for novel diagnostic biomarkers and/or therapeutic targets is realized by the control unit 100 executing the search program 1042.

The control unit 100 accepts a request to start processing that is input by an operator from the input device 111, and starts processing.

In step S11, the control unit 100 accepts a selection request for the omics database input by the operator and acquires a corresponding omics database from the main database MD. At this time, the operator makes selection requests for at least two omics databases.

In step S12, the control unit 100 estimates missing biofeatures for the two or more omics databases acquired in the step S11 using the topic model stored in the model database TD. Here, "estimating" includes "extracting" biofeatures.

In step S13, the control unit 100 outputs the missing biofeatures together with information indicating a corresponding disease name, and terminates the processing.

Furthermore, the control unit 100 may perform a processing to verify the reliability of the extracted missing biofeatures. The verification processing can be performed, for example, by leave-one-disease features out cross-validation (which extracts all omics data for one disease and evaluates the proportion at which the missing omics data are extracted as missing biofeatures).

1-5. Recording media with search programs recorded thereon

One embodiment of the present invention relates to a program product, such as a media drive, that stores the search program 1042. That is, the search program 1042 can be stored in a media drive such as a hard disk, semiconductor memory element such as a flash memory, or an optical disk. The media drive can also be a computer such as a server device. The format of recording the program to the media drive is not limited as long as each device can read the program. It is preferred that the recording to the media drive is non-volatile. 2. Method of using relationship between each disease and combination of tissue and gene corresponding to each disease listed in Listing 3 as candidate for disease biomarker and/or therapeutic target

In the Listing 3 at the end of the present description, the genes, tissues and diseases extracted in the above 1. are shown. These genes are candidates for novel diagnostic biomarkers and/or therapeutic targets. Here, the candidate is intended to be data in a state of crude refinement that has not yet been verified for actual effectiveness. A diverse and astronomical variety of omics data is obtained from living organisms, from which a vast number of experiments (screening) must be repeated to arrive at a specific diagnostic biomarker and/or therapeutic target. The candidate is intended to be a molecule that provides stepping-stone information to simplify such screening.

The Listing 3 lists the data in order of (Disease ID for figure, UMLS ID, Tissue _ gene, count). The "Disease ID for figure" denotes the "Disease ID for figure" of the disease name "Disease" shown in Listing 2 at the end of the present description. The "UMLS ID" denotes the "UMLS ID" shown in Listing 1. The "Tissue _ gene" denotes a combination of newly extracted tissue and genes. Thus, the "Tissue_gene" combinations in the Listing 3 are combinations of diagnostic biomarker and/or therapeutic target and tissue as new candidates in each disease.

3. Verification of effectiveness

The LDA model is a topic model of bag-of-words data (Ref: Blei et al., 2003, Journal of Machine Learning Research 3, 993-1022). The conventional LDA model is a method that classifies sentences or documents as topics based on related words. Diseases are influenced by many different types of factors, including genes, microbiota, lifestyle, and the like. This is synonymous with describing diseases using terms such as genes, microbiota, lifestyle, and the like.

To address the multifactorial nature of human disease, the conventional LDA model was extended to classify diseases according to factors affecting a plurality of diseases. Herein, GWAS catalog (https://www.ebi.ac.uk/gwas/docs/file-downloads), SIDER (http://sideeffects.embl.de)/DrugBank (https://go.drugbank.com/), Disbiome (https://disbiome.ugent.be/home) (Janssens et al., 2018), or NCBI GEO DataSets (https://www.ncbi.nlm.nih.gov/gds) were used to classify diseases into three topics according to single nucleotide polymorphism (SNPs), drug targets (i.e., therapeutic targets of the disease), microbiota, and/or human disease-associated tissue/organ transcriptome data. The "number of topics" was determined by how many groups (topics) can be divided into to best represent each group (topic) specifically when the diseases were divided into groups (topics) according to the multiple biofeatures associated with each disease. Examples of methods for determining the number of topics include perplexity, community detection, and the like.

3-1. Performance evaluation of extended LDA model

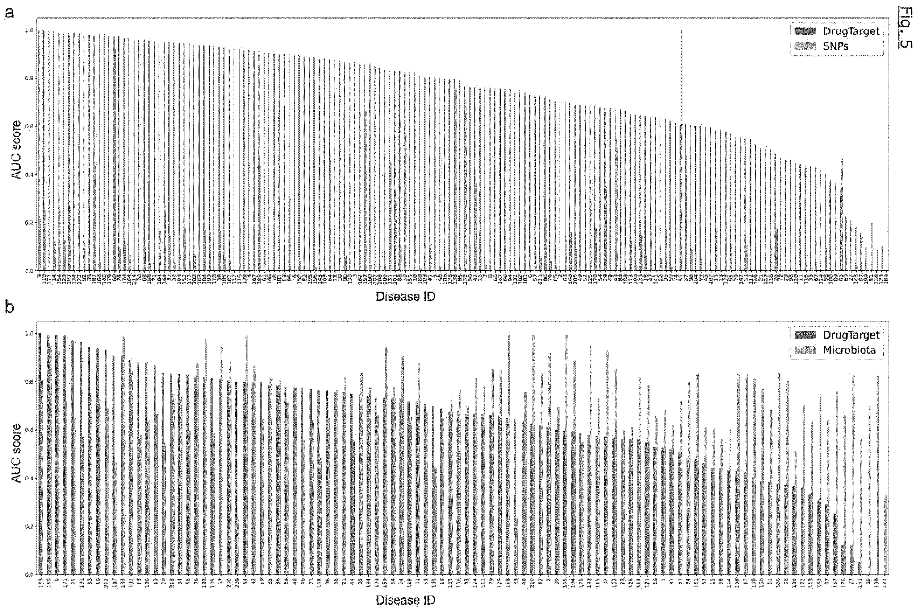

An extended LDA framework was used to predict disease biomarkers and/or therapeutic targets. For this purpose, performance in predicting the missing information for each disease was assessed by omitting certain types of disease-associated information and leaving the remaining information intact. Two models were evaluated by executing this "leave-one-disease features out cross-validation" scheme (FIG. 5, and Listing 2 at the end of the present description).

The first model is composed of the disease-associated biofeatures, i.e., SNPs and drug targets, and in this model, both SNPs and drug target biofeatures are tagged for 170 diseases with both biofeatures (i.e., the "type" in the description of the extended LDA model described above is "SNP and drug target"). The model includes 170 diseases with both SNPs and drug target information (FIG. 5, <Listing 2: Disease ID> at the end of the present description). All SNPs associated with one disease were excluded, all SNPs associated with the remaining 169 diseases were retained, and all drug target information was retained for all the 170 diseases. The generated model was then used to evaluate the performance of the model by predicting missing SNPs associated with the one disease mentioned above. This evaluation was repeated for all the 170 diseases.

In addition, all drug targets associated with one disease were excluded, all drug targets associated with the remaining 169 diseases were retained, and all SNPs information was retained for all 170 diseases. The generated model was then used to evaluate the performance of the model by predicting missing drug targets associated with the one disease mentioned above. This evaluation was repeated for all the 170 diseases.

The evaluation of the prediction performance of each disease was based on AUC scores.

The AUC scores were calculated as follows. Each biofeature was ranked according to sampling frequency with 1 being the highest and 0 being the lowest. This ranking score for each biofeature was compared to the score of 1 for known (observed) biofeatures and the score of 0 for all the other biofeatures (i.e., unobserved biofeatures). The AUC scores were calculated for each type of feature by comparing the scores of all sampled biofeature groups of each type.

FIG. 5a shows results. When predicting the missing drug targets, 104 diseases of the 170 diseases had AUC scores greater than 0.7 and 81 diseases had AUC scores greater than 0.8. In contrast, when predicting the missing SNPs, only 4 diseases had AUC scores greater than 0.7. This result was considered to suggest that the number of SNPs related to each disease was very high.

The same approach was then used to evaluate the performance of a second model, the microbiota/drug target model (i.e., the "type" in the above description of the extended LDA model is "microbiota and drug target") (FIG. 5b, Listing 2). The target diseases are 104 diseases with both microbiota and drug target biofeatures.

FIG. 5b shows results. In the second model, when predicting the missing microbiota, 61 diseases had AUC scores greater than 0.7 and 39 diseases had AUC scores greater than 0.8. When predicting the missing drug targets, 48 diseases had AUC scores greater than 0.7 and 24 diseases had AUC scores greater than 0.8.

These results indicate that the extended LDA model can predict omics data associated with missing diseases.

3-2. Prediction of putative biomarkers and therapeutic targets

The framework for predicting disease-associated biomarkers and/or therapeutic targets from the missing biofeatures predicted by the extended LDA model was applied to disease-associated tissue/organ transcriptome data. The drug target data, microbiota data, and disease-associated tissue/organ transcriptome data were combined and applied to the extended LDA model. Out of the 104 diseases with drug targets and microbiota features, 25 diseases were also tagged with tissue/organ transcriptome features. Thus, transcriptome information was missing for 79 diseases. That is, the missing transcriptome information for these 79 diseases is the transcriptome information newly associated with each disease. Therefore, it is suggested that the transcriptome corresponding to the missing transcriptome information may serve as a novel biomarker and/or therapeutic target for the above 79 diseases.

Analysis of the above 79 diseases identified numerous tissue/organ genes for each disease (FIG. 6, <Listing 3: Novel markers> at the end of the present description).

In particular, some tissue/organ genes showed significantly higher counts (1,018 to 2,911 counts) than other tissue/organ genes (FIG. 6, Listing 3). This approach identified about 1 to 20 tissue/organ genes highly associated with each of the 79 diseases (FIG. 6, Listing 3). Most of them were organs found throughout the body (blood, immune cells) (Listing 3). It was shown to be able to be used as the candidate for biomarkers and/or therapeutic targets for predicting diseases linked by word analysis with the extended LDA model.

Supporting information

<Listing 1: GEO datasets>

Data are listed in the order (UMLS ID, GSE, Ctrl_num,Disease_num, Disease, Tissue_origin, Tissue_rough_class, Note). The "Ctrl_num" is the number of controls, the "Disease_num" is the number of diseases, the "Disease" is the name of a disease, the "Tissue_origin" is the name of a tissue or organ, the "Tissue_rough_class" is the name of a tissue when manually classified, and the "Note" is a note.

(C0003165|C0014013|C0032273|C0032274|C0037116|C0041296|C0041300|C0041301|C004131
8|C0041321|C0041322|C0041324|C0041330|C0041333|C0152717|C0152861|C0152874|C01528
89|C0152915|C0153333|C0153334|C0156756|C0260341|C0260867|C0275716|C0275887|C0343
426|C0348102|C0348103|C0348104|C0348105|C0348106|C0348107|C0348109|C0348301|C034
8466|C0348513|C0348532|C0348539|C0348808|C0348809|C0348810|C0348811|C0348812|C03
48813|C0348825|C0348960|C0348961|C0348962|C0348963|C0348964|C0348966|C0348967|C0
348968|C0348969|C0477446|C0477650|C0477749|C0477767|C0477769|C0477773|C0494034|C
0494035|C0494036|C0494037|C0494132|C0494662|C0496581|C0496612|C0694497|C1331984|
C1331985|C1533626|C3203357,GSE148036,5,5,Tuberculosis,Lung,Lung,)
(C0003165|C0014013|C0032273|C0032274|C0037116|C0041296|C0041300|C0041301|C004131
8|C0041321|C0041322|C0041324|C0041330|C0041333|C0152717|C0152861|C0152874|C01528
89|C0152915|C0153333|C0153334|C0156756|C0260341|C0260867|C0275716|C0275887|C0343
426|C0348102|C0348103|C0348104|C0348105|C0348106|C0348107|C0348109|C0348301|C034
8466|C0348513|C0348532|C0348539|C0348808|C0348809|C0348810|C0348811|C0348812|C03
48813|C0348825|C0348960|C0348961|C0348962|C0348963|C0348964|C0348966|C0348967|C0
348968|C0348969|C0477446|C0477650|C0477749|C0477767|C0477769|C0477773|C0494034|C
0494035|C0494036|C0494037|C0494132|C0494662|C0496581|C0496612|C0694497|C1331984|
C1331985|C1533626|C3203357,GSE131031,4,20,Tuberculosis,ILC2,Lymphocyte,ILC2 from Lung)
(C0003165|C0014013|C0032273|C0032274|C0037116|C0041296|C0041300|C0041301|C004131
8|C0041321|C0041322|C0041324|C0041330|C0041333|C0152717|C0152861|C0152874|C01528
89|C0152915|C0153333|C0153334|C0156756|C0260341|C0260867|C0275716|C0275887|C0343
426|C0348102|C0348103|C0348104|C0348105|C0348106|C0348107|C0348109|C0348301|C034
8466|C0348513|C0348532|C0348539|C0348808|C0348809|C0348810|C0348811|C0348812|C03
48813|C0348825|C0348960|C0348961|C0348962|C0348963|C0348964|C0348966|C0348967|C0
348968|C0348969|C0477446|C0477650|C0477749|C0477767|C0477769|C0477773|C0494034|C
0494035|C0494036|C0494037|C0494132|C0494662|C0496581|C0496612|C0694497|C1331984|
C1331985|C1533626|C3203357,GSE114192,36,44,Tuberculosis (TB) and Intermediate hyperglycemia (IH),Whole_blood,Whole_blood,)
(C0003165|C0014013|C0032273|C0032274|C0037116|C0041296|C0041300|C0041301|C004131
8|C0041321|C0041322|C0041324|C0041330|C0041333|C0152717|C0152861|C0152874|C01528
89|C0152915|C0153333|C0153334|C0156756|C0260341|C0260867|C0275716|C0275887|C0343
426|C0348102|C0348103|C0348104|C0348105|C0348106|C0348107|C0348109|C0348301|C034
8466|C0348513|C0348532|C0348539|C0348808|C0348809|C0348810|C0348811|C0348812|C03
48813|C0348825|C0348960|C0348961|C0348962|C0348963|C0348964|C0348966|C0348967|C0
348968|C0348969|C0477446|C0477650|C0477749|C0477767|C0477769|C0477773|C0494034|C
0494035|C0494036|C0494037|C0494132|C0494662|C0496581|C0496612|C0694497|C1331984|
C1331985|C1533626|C3203357,GSE114192,36,61,Tuberculosis (TB) and Diabetes mellitus (DM),Whole_blood,Whole_blood,)

(C0003165|C0014013|C0032273|C0032274|C0037116|C0041296|C0041300|C0041301|C004131
8|C0041321|C0041322|C0041324|C0041330|C0041333|C0152717|C0152861|C0152874|C01528
89|C0152915|C0153333|C0153334|C0156756|C0260341|C0260867|C0275716|C0275887|C0343
426|C0348102|C0348103|C0348104|C0348105|C0348106|C0348107|C0348109|C0348301|C034
8466|C0348513|C0348532|C0348539|C0348808|C0348809|C0348810|C0348811|C0348812|C03
48813|C0348825|C0348960|C0348961|C0348962|C0348963|C0348964|C0348966|C0348967|C0
348968|C0348969|C0477446|C0477650|C0477749|C0477767|C0477769|C0477773|C0494034|C
0494035|C0494036|C0494037|C0494132|C0494662|C0496581|C0496612|C0694497|C1331984|
C1331985|C1533626|C3203357,GSE114192,36,46,Tuberculosis (TB),Whole_blood,Whole_blood,)

(C0003872|C0033860|C0263361|C0343052|C0343055|C0409673|C0477485,GSE54456,82,92,P soriasis,Skin,Skin,)

(C0003872|C0033860|C0263361|C0343052|C0343055|C0409673|C0477485,GSE41745,3,3,Psor iasis,Skin,Skin,)

(C0003872|C0033860|C0263361|C0343052|C0343055|C0409673|C0477485,GSE121212,38,28, Psoriasis,Skin,Skin,Healthy vs. AD lesional skin)

(C0003872|C0033860|C0263361|C0343052|C0343055|C0409673|C0477485,GSE121212,38,27, Psoriasis,Skin,Skin,Healthy vs. AD non-lesional skin)

(C0004096|C0023212|C0038218|C0155880|C0340139|C0348819|C0349268|C0478625|C049465
0|C0494659|C0494660|C0494678|C0496984|C0496998,GSE85567,28,57,Asthma,Airway epithelium,Airway epithelium,)

(C0004096|C0023212|C0038218|C0155880|C0340139|C0348819|C0349268|C0478625|C049465
0|C0494659|C0494660|C0494678|C0496984|C0496998,GSE109484,14,33,Asthma,Airway epithelium,Airway epithelium,)

(C0004352|C0011768|C0025362|C0236792|C0282512|C0338986|C0349348|C0869217,GSE305 73,2,2,Autism,Temporal cortex,Temporal_cortex,)

(C0005586|C0010598|C0029232|C0236765|C0349208|C0349210|C0349211|C0349212|C034921
3|C0349214|C0349215|C0349218|C0494396|C2939428,GSE80655,24,24,Bipolar disorder, anterior cingulate cortex (AnCg),anterior cingulate cortex (AnCg),)

(C0005586|C0010598|C0029232|C0236765|C0349208|C0349210|C0349211|C0349212|C034921
3|C0349214|C0349215|C0349218|C0494396|C2939428,GSE80336,18,18,Bipolar disorder,Dorsal striatum,Dorsal
striatum,)

(C0005586|C0010598|C0029232|C0236765|C0349208|C0349210|C0349211|C0349212|C034921
3|C0349214|C0349215|C0349218|C0494396|C2939428,GSE112523, 17,10,Bipolar disorder,Frontal cortex,Frontal_cortex,)

(C0005586|C0010598|C0029232|C0236765|C0349208|C0349210|C0349211|C0349212|C034921
3|C0349214|C0349215|C0349218|C0494396|C2939428,GSE80655,22,24,Bipolar disorder,nucleus accumbens
(nAcc),nucleus accumbens (nAcc),)

(C0005586|C0010598|C0029232|C0236765|C0349208|C0349210|C0349211|C0349212|C034921
3|C0349214|C0349215|C0349218|C0494396|C2939428,GSE80655,24,23,Bipolar disorder,dorsolateral prefrontal
cortex (DLPFC),Prefrontal_cortex,)

(C0005586|C0010598|C0029232|C0236765|C0349208|C0349210|C0349211|C0349212|C034921
3|C0349214|C0349215|C0349218|C0494396|C2939428,GSE42546,29,16,Bipolar disorder,dentate gyrus (DG)
granule cells in postmortem hippocampus,Hippocampus,)

(C0009324|C0010346|C0151971|C0348737|C0349268|C0400832|C0409696|C0451837,GSE112 057,12,15,Ulcerative colitis,Whole_blood,Whole_blood,)

(C0009324|C0010346|C0151971|C0348737|C0349268|C0400832|C0409696|C0451837,GSE836 87,8,1,Ulcerative Colitis, Small bowel, Small intestine,)

(C0009324|C0010346|C0151971|C0348737|C0349268|C0400832|C0409696|C0451837,GSE836 87,11,1,Ulcerative Colitis,Sigmoid,Sigmoid,)

(C0009324|C0010346|C0151971|C0348737|C0349268|C0400832|C0409696|C0451837,GSE836 87,17,5,Ulcerative Colitis,right colon,Colon,)

(C0009324|C0010346|C0151971|C0348737|C0349268|C0400832|C0409696|C0451837,GSE836 87,2,1,Ulcerative Colitis,Ileum,Ileum,)

(C0009324|C0010346|C0151971|C0348737|C0349268|C0400832|C0409696|C0451837,GSE836 87,5,21,Ulcerative Colitis,left colon,Colon,)

(C0009324|C0010346|C0151971|C0348737|C0349268|C0400832|C0409696|C0451837,GSE137 344,37,44,Ulcerative Colitis,Ileum,Ileum,)

(C0009324|C0010346|C0151971|C0348737|C0349268|C0400832|C0409696|C0451837,GSE836 87,2,3,Ulcerative Colitis,Rectum,Rectum,)

(C0010674|C0270246|C0348692|C0348815|C0348816|C0494350|C2939175,GSE136371,16,33, Cystic fibrosis,Whole_blood,Whole_blood,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE107638,27,23,Schizophrenia,Brain_Neuronal (from Brodmann area 46),Brain_Neuronal,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE107638,22,17,Schizophrenia,Brain_Oligodendrocyte (from Brodmann area 46),Brain_Oligodendrocyte,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE107638,4,3,Schizophrenia,Brain_Cytoplasmic_fraction (from Brodmann area 46),Brain_Cytoplasmic_fraction,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE112523,17,7,Schizophrenia,Frontal cortex,Frontal_cortex,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE138082,13,7,Schizophrenia,Hippocampus (CA1),Hippocampus,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE138082,13,7,Schizophrenia,Hippocampus (CA3),Hippocampus,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE42546,29,17,Schizophrenia,dentate gyrus (DG) granule cells in postmortem hippocampus,Hippocampus,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE80655,24,24,Schizophrenia,anterior cingulate cortex (AnCg),anterior cingulate cortex (AnCg),)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE80655,24,24,Schizophrenia,dorsolateral prefrontal cortex (DLPFC),Prefrontal_cortex,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE138082,13,7,Schizophrenia,Hippocampus (DG),Hippocampus,)

(C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,GSE80655,22,23,Schizophrenia,nucleus

accumbens (nAcc),nucleus accumbens (nAcc),)

(C0011615|C0494831,GSE65832,20,20,Atopic dermatitis,Skin,Skin,)

(C0011615|C0494831,GSE121212,38,27,Atopic Dermatitis,Skin,Skin,Healthy vs. AD non-lesional skin)

(C0011615|C0494831,GSE121212,38,21,Atopic Dermatitis,Skin,Skin,Healthy vs. AD lesional skin)

(C0011849|C0011854|C0011860|C0271641|C0348447|C0851208,GSE92724,6,4,Type 2 diabetes (T2D),Dermal blood endothelium,Dermal blood endothelium,)

(C0014058|C0026769|C0348564|C0349082|C0494470,GSE138614,25,16,multiple sclerosis,Brain (Active lesion),Brain white matter,)

(C0014058|C0026769|C0348564|C0349082|C0494470,GSE138614,25,17,multiple sclerosis,Brain (Chronic active lesion),Brain white matter,)

(C0014058|C0026769|C0348564|C0349082|C0494470,GSE138614,25,14,multiple sclerosis,Brain (Inactive lesion),Brain white matter,)

(C0014058|C0026769|C0348564|C0349082|C0494470,GSE138614,25,26,multiple sclerosis,Brain (Normal appearing white matter),Brain white matter,)

(C0014058|C0026769|C0348564|C0349082|C0494470,GSE77598,3,5,Multiple Sclerosis,Monocyte,Monocyte,)

(C0014175|C0014177|C0156344|C0156345|C0156346|C0156347|C0156348|C0341858|C0477778|C0495096,GSE 134056,22,16,Endometriosis,Endometrial tissue,Endometrial tissue,)

(C0022104|C0178283|C0338939|C0349268,GSE137344,37,1,Irritable bowel syndrome (IBS),Ileum,Ileum,)

(C0022408|C0038019|C0702154,GSE114007,18,20,Osteoarthritis (OA),Knee articular cartilage,Knee_articular cartilage,)

(C0024141|C0026848|C0263591|C0343426|C0348600|C0348641|C0349082|C0409974|C0409976|C0451753|C0477343|C0477411|C0477587|C0477884|C0494697|C0495047,GSE136731,4,7,S ystemic lupus erythematosus (SLE),cDC2 dendritic cells (CD5+),cDC2 dendritic cells,)

(C0024141|C0026848|C0263591|C0343426|C0348600|C0348641|C0349082|C0409974|C0409976|C0451753|C0477343|C0477411|C0477587|C0477884|C0494697|C0495047,GSE139358,11,11, Systemic lupus erythematosus (SLE),Normal density neutrophil,Neutrophil,)

(C0024141|C0026848|C0263591|C0343426|C0348600|C0348641|C0349082|C0409974|C0409976|C0451753|C0477343|C0477411|C0477587|C0477884|C0494697|C0495047,GSE136731,10,7, Systemic lupus erythematosus (SLE),cDC2 dendritic cells (CD5-CD163+CD14-),cDC2 dendritic cells,)

(C0024141|C0026848|C0263591|C0343426|C0348600|C0348641|C0349082|C0409974|C0409976|C0451753|C0477343|C0477411|C0477587|C0477884|C0494697|C0495047,GSE136731,2,1,S ystemic lupus erythematosus (SLE),cDC2 dendritic cells (CD5-CD163-),cDC2 dendritic cells,)

(C0024141|C0026848|C0263591|C0343426|C0348600|C0348641|C0349082|C0409974|C0409976|C0451753|C0477343|C0477411|C0477587|C0477884|C0494697|C0495047,GSE131525,9,9,S ystemic lupus erythematosus (SLE),CD8+ T cells,T_cells,)

(C0024141|C0026848|C0263591|C0343426|C0348600|C0348641|C0349082|C0409974|C0409976|C0451753|C0477343|C0477411|C0477587|C0477884|C0494697|C0495047,GSE122459,6,20, Systemic lupus erythematosus (SLE),PBMC,PBMC,)

(C0024141|C0026848|C0263591|C0343426|C0348600|C0348641|C0349082|C0409974|C0409976|C0451753|C0477343|C0477411|C0477587|C0477884|C0494697|C0495047,GSE131525,9,9,S ystemic lupus erythematosus (SLE),B cells,B_cells,)

(C0024141|C0026848|C0263591|C0343426|C0348600|C0348641|C0349082|C0409974|C0409976|C0451753|C0477343|C0477411|C0477587|C0477884|C0494697|C0495047,GSE 131525,9,9,S ystemic lupus erythematosus (SLE),Monocyte,Monocyte,)

(C0024141|C0026848|C0263591|C0343426|C0348600|C0348641|C0349082|C0409974|C0409976|C0451753|C0477343|C0477411|C0477587|C0477884|C0494697|C0495047,GSE72509,18,99, Systemic lupus erythematosus (SLE) (anti-ro: high / ISM high),Whole_blood,Whole_blood,)

(C0024141|C0026848|C0263591|C0343426|C0348600|C0348641|C0349082|C0409974|C0409976|C0451753|C0477343|C0477411|C0477587|C0477884|C0494697|C0495047,GSE 118256,8,9,S ystemic lupus erythematosus (SLE),Transitional 3 B cells (T3),Transitional 3 B cells (T3),)

(C0024141|C0026848|C0263591|C0343426|C0348600|C0348641|C0349082|C0409974|C0409976|C0451753|C0477343|C0477411|C0477587|C0477884|C0494697|C0495047,GSE 118256,8,9,S ystemic lupus erythematosus (SLE),Switched Memory B cells (SM),Switched Memory B cells (SM),)

(C0024141|C0026848|C0263591|C0343426|C0348600|C0348641|C0349082|C0409974|C0409976|C0451753|C0477343|C0477411|C0477587|C0477884|C0494697|C0495047,GSE 118256,6,9,S ystemic lupus erythematosus (SLE),Resting Naive Bcells,Resting Naive Bcells,)

(C0024141|C0026848|C0263591|C0343426|C0348600|C0348641|C0349082|C0409974|C0409976|C0451753|C0477343|C0477411|C0477587|C0477884|C0494697|C0495047,GSE118256,6,8,S ystemic lupus erythematosus (SLE),Double negative B cells,Double negative B cells,)

(C0024141|C0026848|C0263591|C0343426|C0348600|C0348641|C0349082|C0409974|C040997 6|C0451753|C0477343|C0477411|C0477587|C0477884|C0494697|C0495047,GSE118256,3,4,S ystemic lupus erythematosus (SLE),Antigen Secreting Cell (ASC),Antigen Secreting Cell (ASC),)

(C0024141|C0026848|C0263591|C0343426|C0348600|C0348641|C0349082|C0409974|C040997 6|C0451753|C0477343|C0477411|C0477587|C0477884|C0494697|C0495047,GSE118256,6,7,S ystemic lupus erythematosus (SLE),Activated Naive B cells,Activated Naive B cells,)

(C0024141|C0026848|C0263591|C0343426|C0348600|C0348641|C0349082|C0409974|C040997 61C04517531C04 773431C04 77 4111C04 775871C04 778841C04946971C049504 7 ,GSE 131525,9,9,S ystemic lupus erythematosus (SLE),CD4+ T cell,T_cells,)

(C0027651,GSE121842,2,2,Rectum cancer,Rectum,Rectum,Pericarcinoma vs. Cancer)

(C0027651,GSE97239,3,3,Bladder cancer,Bladder,Bladder,)

(C0027651,GSE133624,29,36,Bladder cancer,Bladder,Bladder,Paracancerous tissue vs. cancer tissue)

(C0028754|C0031880|C0154269|C0260823|C0332544|C0348480|C0348956|C0349252|C045181 9,GSE152991,11,20,Metabolically unhealthy Obese (MUO),Subcutaneous adipose tissue,Adipose tissue,vs. metabolically healthy lean (MHL) (a) MHL (n = 11, 6 women) had BMI 18.5-24.9 kg/m2, plasma TG concentration <150 mg/dL, fasting plasma glucose concentration < 100 mg/dL, 2-hour OGTT plasma glucose concentration <140 mg/dL, HbAlc $\leqq$ 5.6%, and IHTG content <4%; (b) MHO (n = 15, 13 women) had BMI 30-49.9 kg/m2, plasma TG concentration <150 mg/dL, fasting plasma glucose concentration <100 mg/dL, 2-hour OGTT plasma glucose concentration <140 mg/dL, HbAlc $\leqq$5.6%, and IHTG content <4%; and (c) MUO (n = 20, 15 women) had BMI 30-49.9 kg/m2, prediabetes (fasting plasma glucose concentration $\geqq$ 100 mg/dL, 2-hour OGTT plasma glucose concentration $\geqq$ 140 mg/dL, and/or HbAlc $\geqq$5.7%), and IHTG content $\geqq$5%.)

(C0028754|C0031880|C0154269|C0260823|C0332544|C0348480|C0348956|C0349252|C045181 9,GSE152991,11,14,Metabolically healthy Obese (MHO),Subcutaneous adipose tissue,Adipose tissue,vs. metabolically healthy lean (MHL) (a) MHL (n = 11, 6 women) had BMI 18.5-24.9 kg/m2, plasma TG concentration <150 mg/dL, fasting plasma glucose concentration <100 mg/dL, 2-hour OGTT plasma glucose concentration <140 mg/dL, HbAlc ^5.6%, and IHTG content <4%; (b) MHO (n = 15, 13 women) had BMI 30-49.9 kg/m2, plasma TG concentration <150 mg/dL, fasting plasma glucose concentration <100 mg/dL, 2-hour OGTT plasma glucose concentration <140 mg/dL, HbAlc ^5.6%, and IHTG content <4%; and (c) MUO (n = 20, 15 women) had BMI 30-49.9 kg/m2, prediabetes (fasting plasma glucose concentration $\geqq$ 100 mg/dL, 2-hour OGTT plasma glucose concentration $\geqq$ 140 mg/dL, and/or HbAlc $\geqq$ 5.7%), and IHTG content $\geqq$ 5%.)

(C0036220|C0153464|C0153519|C0153538|C0153560|C0153561|C0153562|C0153565|C027653 5|C0348355|C0348364,GSE100684,4,4,Kaposi sarcoma, Unknown, Unknown,)

(C0036421|C0349082|C0451842|C0477589|C0494697,GSE136731,4,4,Systemic sclerosis,cDC2 dendritic cells (CD5+),cDC2 dendritic cells,)

(C0036421|C0349082|C0451842|C0477589|C0494697,GSE136731,10,5,Systemic sclerosis,cDC2 dendritic cells (CD5-CD163+CD14-),cDC2 dendritic cells,)

(C0085084,GSE124439,4,37,ALS (Amyotrophic lateral sclerosis) spectrum MND (Motor neurone diseases),Motor cortex (lateral),Motor cortex,)

(C0085084,GSE124439,4,43,ALS (Amyotrophic lateral sclerosis) spectrum MND (Motor neurone diseases),Motor cortex (medial),Motor cortex,)

(C0085084,GSE124439,18,64,ALS (Amyotrophic lateral sclerosis) spectrum MND (Motor neurone diseases),Frontal cortex,Frontal cortex,)

(C0085084,GSE122649,12,4,Sporadic amyotrophic lateral sclerosis (sALS) and Frontotemporal dementia (FTD),Motor cortex,Motor cortex,)

(C0085084,GSE122649,12,22,Sporadic amyotrophic lateral sclerosis (sALS),Motor cortex,Motor cortex,)

(C0348703,GSE79544,7,16,Idiopathic Pulmonary Fibrosis (IPF),lung bronchoalveolar lavage(BAL) macrophage,Macrophage,)

(C0348703,GSE73189,5,4,Idiopathic Pulmonary Fibrosis (IPF),Lung,Lung,)

(C0348703,GSE138283,5,12,Idiopathic Pulmonary Fibrosis (IPF),Lung,Lung,)

(C0348703,GSE52463,7,8,Idiopathic Pulmonary Fibrosis (IPF),Lung,Lung,)

(breast cancer,GSE110114,3,10,Breast cancer (Luminal B type),Breast,Breast,)

(breast cancer,GSE117970,50,4,Breast cancer (DCIS; Ductal Carcinoma in Situ),Monocyte,Monocyte,)

(breast cancer,GSE117970,50,29,Breast cancer (Invasive breast cancer),Monocyte,Monocyte,)

(breast cancer,GSE124030,1,6,Breast cancer,Breast,Breast,)

(breast cancer,GSE117970,4,4,Breast cancer (Invasive breast cancer),Macrophage,Macrophage,)

(colorectal cancer,GSE50760,18,18,metastatic colorectal cancer to the liver (stage IV),Colorectum,Colorectum,)

(colorectal cancer,GSE109203,3,4,Colorectal tumor (poorly differentiated),Colorectal tissue,Colorectum,)

(colorectal cancer,GSE109203,3,4,Colorectal tumor (well differentiated),Colorectal tissue,Colorectum,)

(colorectal cancer,GSE109203,3,4,Colorectal tumor (moderately differentiated),Colorectal tissue,Colorectum,)

(diabetic nephropathy,GSE142025,9,6,Early Diabetic Nephropathy (DN),Kidney,Kidney,)

(endometrial cancer,GSE117970,50,4,Endometrial cancer (UPSC; Uterine papillary serous carcinoma),Monocyte,Monocyte,)

(endometrial cancer,GSE117970,5,1,Endometrial cancer (Carcinosarcoma),Macrophage,Macrophage,)

(endometrial cancer,GSE117970,5,5,Endometrial cancer (Endometrioid),Macrophage,Macrophage,)

(endometrial cancer,GSE56087,9,9,Endometrial tumor,Endometrial tissue,Endometrial tissue,Control: adjacent normal tissue)

(endometrial cancer,GSE146889,10,11,Endometrial tumor,Endometrial tissue,Endometrial tissue,MSI-H, likely Lynch due to MSH6 germline)

(endometrial cancer, GS E 146 8 8 9,3,4,Endometrial tumor,Endometrial tissue,Endometrial tissue,MSI-H, likely Lynch due to PMS2 germline)

(endometrial cancer,GSE146889,10,9,Endometrial tumor,Endometrial tissue,Endometrial tissue,MSI-H, MLH1 hypermethylation)

(endometrial cancer,GSE117970,50,5,Endometrial cancer (Endometrioid),Monocyte,Monocyte,)

(endometrial cancer,GSE117970,5,3,Endometrial cancer (UPSC; Uterine papillary serous carcinoma),Macrophage,Macrophage,)

(endometrial cancer,GSE146889,9,9,Endometrial tumor,Endometrial tissue,Endometrial tissue,MSS)

(endometrial cancer,GSE117970,50,1,Endometrial cancer (Carcinoma),Monocyte,Monocyte,)

<Listing 2: Disease ID>

Data are listed in the order (Disease ID for figure, UMLS ID, Disease, Drug Target, SNPs, Microbiota, Human Transcriptome, GSE).

(0,C0000727|C0002962|C0003862|C0008031|C0013428|C0018681|C0024031|C0029537|C0030193|C0030196|C0030319|C0031315|C0039494|C0040997|C0151827|C0154466|C0154729|C0156401|C0159066|C0184567|C0232286|C0232492|C0242429|C0349278|C0349295|C0392361|C0423729|C0452135|C0476481|C0478118|C0478119|C0478148|C0478488|C0478659|C0494408|C0494479|C0494993|C0495666|C0495669|C0495671|C0677061|C0995154|C1442976,pain,1,1,0,0, );(1,C0001144|C0001145|C0022548|C0029485|C0152249|C0263437|C0263442|C0494863|C0702166,acne,1,0,1,0,);(2,C0001339|C0153094|C0341465|C0341470|C0348760|C0348762,pancreatitis,1,1,1,0,);(3,C0001546,anxiety and major depressive disorder,0,1,0,0,);(3,C0001546,anxiety in major depressive disorder,0,1,0,0,);(3,C0001546,depressed mood,1,0,0,0,);(3,C0001546,subjective well-being,0,1,0,0,);(4,C0001546|C0001818|C0003469|C0013415|C0020604|C0027804|C0028084|C0028768|C0030319|C0031572|C0038436|C0236748|C0236816|C0236818|C0260822|C0270305|C0270549|C0338908|C0349194|C0349230|C0349231|C0349232|C0349233|C0349234|C0349257|C0349274|C0349290|C0349335|C0349337|C0478604|C0494432|C0525045|C1263846|C1527281,anxiety,1,1,0,0,);(5,C0001546|C0001818|C0003469|C0013415|C0020604|C0027804|C0028084|C0028768|C0030319|C0038436|C0236748|C0236816|C0236818|C0270305|C0270549|C0338908|C0349194|C0349231|C0349274|C0349290|C0349335|C0349337|C0494432|C1527281,anxiet Y disorder,1,1,0,0,);(6,C0001546|C0003431|C0014089|C0016142|C0029121|C0037448|C0149654|C0221520|C0339004|C0339009|C0339010|C0339017|C0349217|C0349335|C0349336|C0494432|C0579986|C0694453,conduct disorder,1,1,0,0,);(7,C0001546|C0027804|C0349268,worry,1,1,0,0,);(8,C0001818|C0030319|C0 236748|C0236816,panic disorder, 1,1,0,0,);(9,C0002171|C0477504,alopecia areata,1,1,1,0,);(10,C0003123|C0003125|C0338959|C0494440|C2267227,anorexia nervosa,1,1,1,0,);(11,C0003165|C0014013|C0032273|C0032274|C0037116|C0041296|C0041300|C0041301|C0041318|C0041321|C0041322|C0041324|C0041330|C0041333|C0152717|C0152861|C0152874|C0152889|C0152915|C0153333|C0153334|C0156756|C0260341|C0260867|C0275716|C0275887|C0343426|C0348102|C0348103|C0348104|C0348105|C0348106|C0348107|C0348109|C0348301|C0348466|C0348513|C0348532|C0348539|C0348808|C0348809|C0348810|C0348811|C0348812|C0348813|C0348825|C0348960|C0348961|C0348962|C0348963|C0348964|C0348966|C0348967|C0348968|C0348969|C0477446|C0477650|C0477749|C0477767|C0477769|C0477773|C0494034|C0494035|C0494036|C0494037|C0494132|C0494662|C0496581|C0496612|C0694497|C1331984|C1331985|C1533626|C3203357,tuberculosis,1,1,1,1,GSE148036, GSE131031);(11,C0003165|C0014013|C0032273|C0032274|C0037116|C0041296|C0041300|C0 041301|C0041318|C0041321|C0041322|C0041324|C0041330|C0041333|C0152717|C0152861|C0152874|C0152889|C0152915|C0153333|C0153334|C0156756|C0260341|C0260867|C0275716|C0275887|C0343426|C0348102|C0348103|C0348104|C0348105|C0348106|C0348107|C0348109|C0348301|C0348466|C0348513|C0348532|C0348539|C0348808|C0348809|C0348810|C0348811|C0348812|C0348813|C0348825|C0348960|C0348961|C0348962|C0348963|C0348964|C0348966|C0348967|C0348968|C0348969|C0477446|C0477650|C0477749|C0477767|C0477769|C0477773|C0494034|C0494035|C0494036|C0494037|C0494132|C0494662|C0496581|C0496612|C0694497|C1331984|C1331985|C1533626|C3203357,tuberculosis (tb),0,0,0,1,GSE114192);(11,C0003165|C0014013|C0032273|C0032274|C0037116|C0041296|C0041300|C0041301|C0041318|C0041321|C0041322|C0041324|C0041330|C0041333|C0152717|C0152861|C0152874|C0152889|C0152915|C0153333|C0153334|C0156756|C0260341|C0260867|C0275716|C0275887|C0343426|C0348102|C0348103|C0348104|C0348105|C0348106|C0348107|C0348109|C0348301|C0348466|C0348513|C0348532|C0348539|C0348808|C0348809|C0348810|C0348811|C0348812|C0348813|C0348825|C0348960|C0348961|C0348962|C0348963|C0348964|C0348966|C0348967|C0348968|C0348969|C0477446|C0477650|C0477749|C0477767|C0477769|C0477773|C0494034|C0494035|C0494036|C0494037|C0494132|C0494662|C0496581|C0496612|C0694497|C1331984|C1331985|C1533626|C3203357,tuberculosis (tb) and diabetes mellitus (dm),0,0,0,1,GSE114192);(11,C0003165|C0014013|C0032273|C0032274|C0037116|C0041296|C0041300|C0041301|C0041318|C0041321|C0041322|C0041324|C0041330|C0041333|C0152717|C0152861|C0152874|C0152889|C0152915|C0153333|C0153334|C0156756|C0260341|C0260867|C0275716|C0275887|C0343426|C0348102|C0348103|C0348104|C0348105|C0348106|C0348107|C0348109|C0348301|C0348466|C0348513|C0348532|C0348539|C0348808|C0348809|C0348810|C0348811|C0348812|C0348813|C0348825|C0348960|C0348961|C0348962|C0348963|C0348964|C0348966|C0348967|C0348968|C0348969|C0477446|C0477650|C0477749|C0477767|C0477769|C0477773|C0494034|C0494035|C0494036|C0494037|C0494132|C0494662|C0496581|C0496612|C0694497|C1331984|C1331985|C1533626|C3203357,tuberculosis (tb) and intermediate hyperglycemia (ih),0,0,0,1,GSE114192);(12,C0003431,frustration,1,0,0,0,);(12,C0003431,callous-unemotional behaviour,0,1,0,0,);(13,C0003615|C0031154|C0085693|C0152505|C0156095,appendicitis,1,1,1,0,);(14,C0003860|C0032533|C0343200|C0477585,giant cell arteritis,1,1,0,0,);(15,C0003872|C0033860|C0263361|C0343052|C0343055|C0409673|C0477485 ,psoriasis,1,1,1,1,GSE54456, GSE41745, GSE121212, GSE121212);(16,C0003873|C0015773|C0026848|C0029660|C0038013|C0409651|C0409652|C0477411|C0477541|C0477542|C0494896|C0494897|C0694497|C3714757,rheumatoid arthritis,1,1,1,0,);(17,C0003962|C0036528|C0152717|C0156180|C0477842|C0495216|C0495676 |C0495700,ascites,1,0,1,0,);(18,C0004096|C0023212|C0038218|C0155880|C0340139|C0348819|C0349268|C0478625|C0494650|C0494659|C0494660|C0494678|C0496984|C0496998,asthma,1, 1,1,1,GSE85567, GSE109484);(19,C0004153|C0007775|C0010054|C0017327|C0155733|C0155734|C0348648|C0494764|C0495064|C0495663|C3495604,atherosclerosis,1,0,1,0,);(20,C0004352|C0011768|C0025362|C0236792|C0282512|C0338986|C0349348|C0869217,autism,1,1,1,1,GSE30573);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,pneumonia,1,1,1,0,);(22,C0004698|C0149938|C0268701|C0268732|C0342611|C0348988|C0451767|C0451769|C0495053|C0694464|C0694525|C0848548|C0868872|C0869068,nephropathy,1,1,0,0, );(23,C0005586|C0010598|C0013415|C0020604|C0027804|C0030319|C0038436|C0154411|C0154412|C0154413|C0270268|C0338808|C0338908|C0339017|C0349212|C0349213|C0349216|C0349217|C0349218|C0349219|C0349231|C0349236|C0349290|C0392322|C0494396|C0494399|C0494400|C0494432|C0495315|C0496594|C0525045|C0869478,depression,1,1,0,0,);(24,C0005586|C0010598|C0029232|C0236765|C0349208|C0349210|C0349211|C0349212|C0349213|C0349214|C0349215|C0349218|C0494396|C2939428,bipolar disorder,1,1,1,1,GSE80655, GSE80336, GSE112523, GSE80655, GSE80655, GSE42546);(25,C0006267|C0152239|C0348809|C0348968|C0431530|C0494034|C0494659,bronchiectasis,1,0,1,0,);(26,C0006413|C0019829|C0021071|C0023434|C0023493|C0024301|C0024 305|C0024419|C0079744|C0079748|C0079758|C0079774|C0152266|C0152267|C0152268|C0153470|C0348387|C0348388|C0348389|C0348390|C0348394|C0348396|C0451750|C0452189|C0452192|C0494171|C0494172|C1264187|C1264188|C1264190|C1266194,lymphoma,1,1,0,0,);(27, C0007286,carpal tunnel syndrome,1,1,0,0,);(28,C0007820|C0011269|C0393560|C0393561|C0393562|C0478654|C04946 13, vascular dementia,1,1,0,0,);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C364 8968,hypertension,1,1,1,0,);(30,C0007860|C0014719|C0348904|C0477866|C0494054,cervicitis, 1,0,1,0,);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,pregnancy,1,0,1,0, );(32,C0008312,primary biliary cirrhosis,0,1,1,0,);(32,C0008312,primary biliary cholangitis,0,1,1,0,);(32,C0008312,biliary cirrhosis primary,1,0,0,0,);(33,C0008677|C0024117|C0264222|C0348686|C0348693|C0348817|C034881 8|C0494659,chronic obstructive pulmonary disease, 1,1,1,0,);(34,C0008684|C0155937|C0348160|C0494704|C0494706,gingivitis,1,0,1,0,); (35,C0008711|C0009443|C0018621|C0348688|C0348689|C0494650|C0494660|C2607914,allergic rhinitis,0,1,0,0,);(35,C0008711|C0009443|C0018621|C0348688|C0348689|C0494650|C0494660| C2607914,rhinitis allergic,1,0,0,0,);(36,C0008924|C0008925|C0158646|C0432090|C0432098|C0451896|C0451897 |C0477977|C0478022|C0495504|C0495539|C0495540|C0495541|C0495542|C0495543|C0495545|C0869081|C2981150,cleft palate,1,1,1,0,);(37,C0009088,cluster headache,1,1,0,0,);(38,C0009088|C0018681|C0033893|C0149931|C0349268|C0393745|C0475523|C0475524|C0475525|C0477374|C0494408|C0494479|C0495121|C0546983|C0869050|C0869051|C0995154|C1735856,headache,1,1,0,0,);(39,C0009324|C0010346|C0151971|C0348737|C0349268|C0400832|C0409696|C0451837,ulcerative colitis,0,1,1,1,GSE83687, GSE83687, GSE83687, GSE83687, GSE83687, GSE137344, GSE83687);(39,C0009324|C0010346|C0151971|C0348737|C0349268|C0400832|C0409696|C0451837,ulcerative colitis,0,1,1,1,GSE112057);(39,C0009324|C0010346|C0151971|C0348737|C0349268|C0400832|C0409696|C0451837,colitis ulcerative,1,0,0,0,);(40,C0009806|C0014089|C0278008,constipation,1,0,1,0,);(41,C0010054,coronary artery disease,1,1,1,0,);(42,C0010054|C0348590,coronary heart disease,1,1,1,0,);(43,C0010346|C0156146|C0156147|C0348736|C0409695|C0451836,crohn disease,1,1,1,0,);(44,C0010674|C0270246|C0348692|C0348815|C0348816|C0494350|C2939175, cystic fibrosis,1,0,1,1,GSE136371);(45,C0011168|C0032249|C0349249|C0495666,dysphagia,1,0,0,0,); (46,C0011251|C0016142|C0029225|C0029226|C0030477|C0036337|C0036339|C0036341|C0036344|C0036347|C0036349|C0036351|C0149654|C0221520|C0236818|C0270497|C0338650|C0338798|C0349192|C0349193|C0349194|C0349195|C0349196|C0349199|C0349200|C0349203|C0349243|C0349277|C0349709|C0392322|C0494408|C1263846|C1443045,schizophrenia,1,1,1,1,G SE107638, GSE107638, GSE107638, GSE112523, GSE138082, GSE138082, GSE42546, GSE80655, GSE80655, GSE138082, GSE80655);(47,C0011603|C0011616|C0043194|C0162820|C0162823|C0477474|C0494831|C09 36250,eczema,1,1,0,0,);(48,C0011615|C0494831,dermatitis atopic,1,0,0,0,);(48,C0011615|C0494831,atopicdermatitis,0,1,1,1,GSE121212, GSE121212);(48,C0011615|C0494831,atopic dermatitis,0,1,1,1,GSE65832);(49,C0011768|C0014544|C0020703|C0025362|C0027066|C00292 25|C0029233|C0036341|C0236795|C0236826|C0270850|C0282512|C0349082|C0475521|C0477 370|C0477371|C0478623|C0494471|C0494472|C0495698,epilepsy,1,1,0,0,);(50,C0011849|C001 1854|C0011860|C0032969|C0085207|C0158981|C0260526|C0260823|C0270221|C0271641|C03 48447|C0477716|C0478038|C0478546|C0494336|C0494882|C0495314|C0495663|C0495706|C0 701147|C0851207|C0851208|C0851209|C0851217,diabetes mellitus,1,1,0,0,);(51,C0011849|C0011854|C0011860|C0271641|C0348447|C0851207,type 1 diabetes,0,1,1,0,);(51,C0011849|C0011854|C0011860|C0271641|C0348447|C0851207, type 1 diabetes mellitus,1,0,0,0,);(52,C0011849|C0011854|C0011860|C0271641|C0348447|C0851208,type 2 diabetes,0,1,1,0,);(52,C0011849|C0011854|C0011860|C0271641|C0348447|C0851208,type 2 diabetes (t2d),0,0,0,1,GSE92724);(52,C0011849|C0011854|C0011860|C0271641|C0348447|C0851208,ty pe 2 diabetes mellitus,1,0,0,0,);(53,C0011884,diabetic retinopathy,1,1,0,0,);(54,C0012715|C0154286|C0162316|C0240066|C0476334|C0477300,iron deficiency,1,1,0,0,);(55,C0013295|C0151971,duodenal ulcer,1,1,0,0,);(56,C0013395|C0018834|C0232492,functional dyspepsia,1,0,1,0,);(57,C0013923|C0013924|C0029785|C0034065|C0042374|C0154685|C01557 31|C0155749|C0155755|C0155770|C0155773|C0155774|C0155775|C0155776|C0155777|C0156 194|C0157299|C0157535|C0340325|C0340579|C0340589|C0340712|C0342039|C0342044|C034 8650|C0348786|C0348787|C0348832|C0348876|C0393560|C0451674|C0451676|C0478488|C04 94450|C0494601|C0494609|C0494611|C0494619|C0494620|C0494623|C0494763|C0495064|C0 495184|C0495316,thrombosis,1,1,0,0,);(58,C0014058|C0026769|C0348564|C0349082|C049447 0,multiple sclerosis,1,1,1,1,GSE77598);(58,C0014058|C0026769|C0348564|C0349082|C0494470,multiple sclerosis,1,1,1,1,GSE138614, GSE138614, GSE138614);(59,C0014175|C0014177|C0156344|C0156345|C0156346|C0156347|C0156348|C0 341858|C0477778|C0495096,endometriosis,1,1,1,1,GSE134056);(60,C0014877|C0152210,esotr opia,1,1,0,0,);(61,C0015523,factor xi,0,1,0,0,);(61,C0015523,factor xi deficiency,1,0,0,0,);(62,C0015674|C0024528|C0027804|C0152145|C0236795|C0236816|C04518 80|C0477817|C0546983|C1270972,fatigue,l,0,l,0,);(63,C0017152|C0017154|C0017181|C00383 58|C0085695|C0156076|C0260823|C0267166|C0348729|C0348730|C0348738|C0348893|C0694 464|C2243087,gastritis,1,1,0,0,);(64,C0017154,gastritisatrophic,1,0,0,0,);(64,C0017154,atrophic gastritis,0,0,1,0,);(64,C0017154,gastric atrophy,0,1,0,0,);(65,C0017601|C0017606|C0017614|C0020302|C0036646|C0154777|C0339573 |C0348555|C0348557|C0348791|C0391997|C0431451|C0494532|C0494533|C0494534|C049453 5,glaucoma,1,1,0,0,);(66,C0017606,angle closure glaucoma,1,0,0,0,);(66,C0017606,glaucoma (primary angle closure),0,1,0,0,);(67,C0018099|C0034139|C0149896|C0409909|C0409910|C0409911|C047754 7|C0494334,gout,1,1,0,0,);(68,C0018681|C0149931|C0338480|C0338483|C0477373|C1735856, migraine,1,1,1,0,);(69,C0018798|C0018816|C0036439|C0477999,congenital heart malformation,0,1,0,0,);(69,C0018798|C0018816|C0036439|C0477999,heart malformation,1,0,0,0,);(70,C0018798|C0036439|C0477999,heart disease congenita1,1,0,0,0,);(70,C0018798|C0036439|C0477999,congenital heart disease,0,1,0,0,);(71,C0018800,cardiac hypertrophy,0,1,0,0,);(71,C0018800,cardiomegaly,1,0,0,0,);(72,C0019158|C0019187|C0019189| C0023895|C0042721|C0153075|C0153089|C0276252|C0276434|C0276609|C0276610|C027662 3|C0348185|C0348199|C0348200|C0348305|C0348752|C0400887|C0400895|C0400913|C04009 14|C0400918|C0411016|C0451704|C0451705|C0451709|C0451710|C0451711|C0451712|C0451 800|C0476551|C0476555|C0477538|C0477937|C0478483|C0489954|C0494092|C0494093|C049 4094|C0494782|C0494783|C0494784|C0494786|C0494787|C0494788|C0494789|C0494790|C04 94794|C0496128|C0524910|C0869060|C1290810|C2063407|C4721555,hepatitis,1,1,0,0,);(73,C0 019364|C0022568|C0022573|C0041322|C0155087|C0339239|C0348191|C0348526|C0348532|C 0348533|C0348985|C0494081|C0494524,keratitis,1,0,1,0,);(74,C0019693,acquired immunodeficiency syndrome,1,0,1,0,);(74,C0019693,aids,0,1,0,0,);(75,C0020179|C0349080,huntington disease, 1 ,0, 1,0,);(76,C0020458,excessive sweating,0,1,0,0,);(76,C0020458,night sweats,1,0,0,0,);(77,C0020517|C0034525|C0156153|C0156154|C0267166|C0348099|C0348101| C0348674|C0348676|C0348738|C0413022|C0494761|C0494762|C0494807|C0496106|C054682 2,gastroenteritis,1,0,1,0,);(78,C0020651|C0039070|C0476454|C0477359,orthostatic hypotension,1,1,0,0,);(79,C0020676|C0342149|C0342151|C0342173|C0342204|C0348442|C034 9082|C0477553|C0494270|C0494271|C0494272|C0494273|C0494364|C0495444|C0495634|C0869078|C3165526,hypothyroidism,1,1,0,0,);(80,C0021099|C0030807|C0030809|C0038165|C0085932|C0263312|C0263313|C0263314|C0263316|C0451938|C0477468|C5556023|C2363246,pemphigus,1,1,0,0,);(81,C0021192|C0023343|C0023346|C0023348|C0023351|C0275716|C0343457| C0343458|C0348121|C0348302|C0348513|C0477395|C0477639|C0494893,leprosy,1,1,0,0,);(82 ,C0021364|C0404584,infertility male,1,0,0,0,);(82,C0021364|C0404584,male infertility,0,1,0,0,);(83,C0021364|C0405581|C0476525,azoospermia,1,0,1,0,);(84,C0022104|C0178283|C0338939|C0349268,irritable bowel syndrome,1,1,1,0,);(84,C0022104|C0178283|C0338939|C0349268,irritable bowel syndrome (ibs),0,0,0,1,GSE137344);(85,C0022408|C0038019|C0702154,osteoarthritis (oa),0,0,0,1,GSE114007);(85,C0022408|C0038019|C0702154,osteoarthritis,1,1,1,0,);(86,C0022650,kidney stone,0,0,1,0,);(86,C0022650,nephrolithiasis,1,1,0,0,);(87,C0022661|C0041349|C0268731|C0348642|C0477312|C0495063|C0848548,chronic kidney disease,1,1,1,0,);(88,C0024141|C0026848|C0263591|C0343426|C0348600|C0348641|C0349082| C0409974|C0409976|C0451753|C0477343|C0477411|C0477587|C0477884|C0494697|C0495047,systemic lupus erythematosus,1,1,1,0,);(88,C0024141|C0026848|C0263591fC0343426|C0348600|C0348641|C0349082|C0409974|C0409976|C0451753|C0477343|C0477411|C0477587|C0477884|C0494697|C 0495047,systemic lupus erythematosus (sle),0,0,0,1,GSE136731, GSE139358, GSE136731, GSE136731, GSE131525, GSE122459, GSE131525, GSE131525, GSE118256, GSE118256, GSE118256, GSE118256, GSE118256, GSE118256, GSE 131525);(88,C0024141|C0026848|C0263591|C0343426|C0348600|C0348641|C0349082|C0409974|C0409976|C0451753|C0477343|C0477411|C0477587|C0477884|C0494697|C0495047,systemic lupus erythematosus (sle) (anti-ro: high / ism high),0,0,0,1,GSE72509);(89,C0024530|C0024535|C0024536|C0024537|C0152072|C0276837|C0348260|C0348262|C0348263|C0348264|C0348265|C0348986|C0348987|C0348988|C0456106|C0477713|C0477921|C0494108|C0869044,malaria,1,1,0,0,);(90,C0024623,gastric cancer,1,1,0,0,);(91,C0025362|C0029233|C0494371|C0494428|C1263846|C1270972,cognitive impairment,1,1,0,0,);(92,C0025517|C0263660|C0348791,metabolic syndrome,1,1,1,0,);(93,C0026267,mitral valve prolapse,1,1,0,0,);(94,C0026603,motion sickness,1,1,0,0,);(95,C0026896|C0154199|C0494501|C0495465,myasthenia gravis, 1,1,1,0,);(96,C0027430,nasal polyps, 1,1,0,0,);(97,C0027531|C0037929|C0411063|C0452049|C0452050|C0452051|C0478224| C0478257|C0694460|C1331979,spinal cord injury,1,0,1,0,);(98,C0027651,urinary bladder cancer,0,1,0,0,);(98,C0027651,tongue cancer,0,0,1,0,);(98,C0027651,squamous cell carcinoma,1,1,0,0,);(98,C0027651,small cell carcinoma, 1,0,0,0,);(98,C0027651,rectum cancer,0,0,0,1,GSE121842);(98,C0027651,pituitary adenoma,1,0,0,0,);(98,C0027651,phaeochromocytoma,1,0,0,0,);(98,C0027651,pancreatic carcinoma,1,0,0,0,);(98,C0027651,pancreatic cancer,0,1,1,0,);(98,C0027651,oral cancer,0,0,1,0,);(98,C0027651,oral cavity cancer,0,1,0,0,);(98,C0027651,metastatic carcinoma,1,0,0,0,);(98,C0027651,metastatic squamous cell carcinoma, 1,0,0,0,);(98,C0027651,adrenal adenoma, 1,0,0,0,);(98,C0027651,adrenal carcinoma,1,0,0,0,);(98,C0027651,anal cancer,1,0,0,0,);(98,C0027651,biliary tract cancer,0,0,1,0,);(98,C0027651,adenoma benign, 1,0,0,0,);(98,C0027651,bone cancer metastatic,1,0,0,0,);(98,C0027651,central nervous system leukaemia,1,0,0,0,);(98,C0027651,lip morphology,0,1,0,0,);(98,C0027651,bladder cancer, 1,1,1,1,GSE97239, GSE133624);(99,C0028754|C0031880|C0154269|C0260823|C0332544|C0348480|C0348956|C0349252|C0451819,metabolically healthy obese (mho),0,0,0,1,GSE152991);(99,C0028754|C0031880|C0154269|C0260823|C0332544|C0348480|C0348956|C0349252|C0451819,metabolically unhealthy obese (muo),0,0,0,1,GSE152991);(99,C0028754|C0031880|C0154269|C0260823|C0332544|C0348480|C0348956|C0349252|C0451819,obesity,1,1,1,0,);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,osteoporosis,1,1,1,0,);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,otitis media,1,1,1,0,);(102,C0030319,fear of pain,0,1,0,0,);(102,C0030319,fear of severe pain,0,1,0,0,);(102,C0030319,panic attack, 1 ,0,0,0,);(1 03,C0030567IC 1828079,parkinson disease, 1,1,1,0,);(104,C0030809,pemphigus vulgaris,1,1,1,0,);(105,C0031090|C1314006,periodontal disease, 1,0,1,0,);(106,C0035435,fibromyalgia,1,0,1,0,);(107,C0035436|C0264743|C0348684|C0 409580|C0409651|C0451910|C3536892,rheumatic fever, 1,1,0,0,);(108,C0035439|C0035440|C0155558|C0155711|C0348583|C0348584,rheumatic heart disease, 1,1,0,0,);(109,C0035854|C0477490|C0477510,rosacea,1,0,1,0,);(110,C0036202|C003620 3|C0036204|C0036205|C0348540|C0348753|C0348826|C0451646|C0451751|C0451845|C0477322|C0477329|C0477554|C0694513,sarcoidosis,1,1,0,0,);(111,C0036220|C0153464|C0153519|C0153538|C0153560|C0153561|C0153562|C0153565|C0276535|C0348355|C0348364,kaposi sarcoma,1,0,1,1,GSE100684);(112,C0036285|C0036689|C0477446,scarlet fever,1,1,0,0,);(113,C0036421|C0349082|C0451842|C0477589|C0494697,systemic sclerosis,1,1,1,1,GSE136731,GSE136731);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,sepsis,1,0,1,0,);(115,C0038013|C0348540|C0348715|C0409675|C0477606,ankyl osing spondylitis,1,1,1,0,);(116,C0038436|C0349255|C0494410|C0546983,insomnia,1,1,0,0,);(117,C0 038436|C0478140,suicidal ideation,1,1,0,0,);(118,C0040156|C0270222|C0477553|C0494276|C0494505,hyperthyroidism,1, 1,1,0,);(119,C0040592|C0153107|C0153108|C0348303,trachoma,1,0,1,0,);(120,C0040822|C027 0736|C0393519,essential tremor,1,1,0,0,);(121,C0041333|C0242172|C0348147|C0348149|C0348905|C0451784|C0451785|C0452162|C0452163|C0494056,pelvic inflammatory disease,1,0,1,0,);(122,C0042024,stress urinary incontinence, 1,1,0,0,);(123,C0042900|C0348510,vitiligo,1,1,1,0,);(124,C0085084,sporadic amyotrophic lateral sclerosis (sals),0,0,0,1,GSE122649);(124,C0085084,sporadic amyotrophic lateral sclerosis (sals) and frontotemporal dementia (ftd),0,0,0,1,GSE122649);(124,C0085084,als (amyotrophic lateral sclerosis) spectrum mnd (motor neurone diseases),0,0,0,1,GSE124439, GSE124439, GSE124439);(124,C0085084,amyotrophic lateral sclerosis,1,1,1,0,);(125,C0085096,intermittent claudication,1,0,0,0,);(125,C0085096,peripheral artery disease,0,1,0,0,);(126,C0085207|C0158915|C0477897|C1455664,diabetes (gestational),0,1,0,0,);(126,C0085207|C0158915|C0477897|C1455664,gestational diabetes,1,0,1,0,);(127,C0085215,premature ovarian failure,0,1,0,0,);(127,C0085215,primary ovarian failure,1,0,0,0,);(128,C0085437|C0477333|C0494441|C0694469,bacterial meningitis,0,1,0,0,);(128,C0085437|C0477333|C0494441|C0694469,meningitis bacterial,1,0,0,0,);(129,C0085655|C0494697|C0546839,polymyositis,1,0,0,0,);(130,C0154009|C 2937421,benign prostatic hyperplasia,1,1,0,0,);(130,C0154009|C2937421,benign prostatic hypertrophy,1,0,0,0,);(130,C0154009|C2937421,prostatic hypertrophy,1,0,0,0,);(131,C0155626|C0155668|C0155711|C0260469|C0340325|C0340331|C0340442|C0348589|C0348591|C0348592|C0348593|C0348862|C0348863|C0348864|C0348865|C0348866|C0348867|C0348876|C0348884|C0349074|C0476718|C0494577|C0494578|C0494579|C0494580|C3665604,myocardial infarction,1,1,0,0,);(132,C0155709,atrial fibrillation,1,1,1,0,);(132,C0155709,atrial fibrillation and flutter,1,0,0,0,);(132,C0155709,atrial fibrillation/atrial flutter,0,1,0,0,);(132,C0155709,atrial flutter,1,0,0,0,);(133,C0156258|C0178288|C0451641,urolithiasis,1,1,1,0,);(134,C0156273|C0478 034|C4317009,diverticulitis,1,1,0,0,);(135,C0178283,inflammatory bowel disease,1,1,1,0,);(136,C0178288|C0232867|C0269674|C0348859|C0451774|C0475548|C0477762|C0477811|C0494218|C0495723|C3648968,proteinuria,1,0,1,0,);(137,C0238990,lower respiratory tract infection,1,0,1,0,);(138,C0263361,plaque psoriasis, 1,0,0,0,);(138,C0263361,psoriasis vulgaris,0,1,0,0,);(139,C0263666|C0477588|C0494697|C3495559,dermatomyositis,1,0,0,0,);(140,C0265011|C0265012,abdominal aortic aneurysm, 1,1,0,0,);(141,C0266929|C0392492,chronic periodontitis,1,1,0,0,);(142,C0270850,petit mal epilepsy,1,0,0,0,);(142,C0270850,juvenile myoclonic epilepsy,1,0,0,0,);(142,C0270850,atonic seizures,1,0,0,0,);(142,C0270850,childhood absence epilepsy,0,1,0,0,);(143,C0276609|C0276610|C0348199|C0400913|C0400918|C0451704|C0451705|C0494092|C0494093,hepatitis b,1,1,1,0,);(144,C0338480|C1735856,aura,1,0,0,0,);(144,C0338480|C1735856,migraine without aura,1,1,0,0,);(145,C0338808|C0338970|C0476566|C0480203|C0494400,suicide,1,1,0,0,);(146,C0338960|C2267227,bulimia nervosa,1,1,0,0,);(147,C0338970|C1443045,borderline personality disorder,1,1,0,0,);(148,C0346724,basal cell carcinoma,1,1,0,0,);(149,C0348626,sinus rhythm,1,0,0,0,);(149,C0348626,long qt syndrome,1,0,0,0,);(149,C0348626,brugada syndrome,0,1,0,0,);(150,C0348645,berry aneurysm,1,0,0,0,);(150,C0348645,intracranial aneurysm,0,1,0,0,);(151,C0348694,silicosis,1,0,1,0,);(152,C0348703,idiopathic pulmonary fibrosis,1,1,1,0,);(152,C0348703,idiopathic pulmonary fibrosis (ipf),0,0,0,1,GSE79544, GSE73189, GSE138283, GSE52463);(153,C0348723|C0451753|C0477411|C0478155|C0494697|C1527336,sicca syndrome,0,0,1,0,);(153,C0348723|C0451753|C0477411|C0478155|C0494697|C1527336,syndrome sicca,1,0,0,0,);(154,C0349215,bipolar ii disorder,1,1,0,0,);(155,C0349258,sexual dysfunction (female),0,1,0,0,);(155,C0349258,erectile dysfunction,1,1,0,0,);(156,C0349349|C0579986,attention deficit disorder,1,0,0,0,);(156,C0349349|C0579986,attention deficit hyperactivity disorder,0,1,1,0,);(156,C0349349|C0579986,attention deficit/hyperactivity disorder,1,0,0,0,);(157,C0400914|C0524910,hepatitis c,1,0,1,0,);(158,C0404572,anovulation,1,0,1,0,);(159,C0433856|C0452047,traumatic brain injury,1,0,1,0,);(160,C0451707|C0728889,cholestasis,1,0,1,0,);(161,C0451708|C0451710|C0451711|C0451712|C0494794,chronic liver disease,1,0,1,0,);(162,C0476479,anger,1,1,0,0,);(163,C0476643|C0476684|C0481423|C0556008| C0694464,drug abuse,1,1,0,0,);(164,C0477362,akathisia,1,0,0,0,);(164,C0477362,restless legs syndrome,1,1,0,0,);(164,C0477362,stiff person syndrome,1,0,0,0,);(165,C0477491,chronic urticaria,0,0,1,0,);(165,C0477491,urticaria chronic,1,0,0,0,);(166,C0494026,clostridium difficile infection,1,0,1,0,);(167,C0494242,clotting,1,0,0,0,);(167,C0494242,factor vii,0,1,0,0,);(167,C0494242,fibrinogen,0,1,0,0,);(168,C0494372,sleep duration,0,1,0,0,);(168,C0494372,delirium tremens,1,0,0,0,);(169,C0494650|C0494660,allergic asthma,1,0,1,0,);(170,C0494679,interstitial lung disease,1,1,0,0,);(171,C0494697|C0494887|C0495047|C3495801,granulomatosis with polyangiitis,0,0,1,0,);(171,C0494697|C0494887|C0495047|C3495801,wegener granulomatosis,1,1,0,0,);(172,C0494783,hepatic encephalopathy,1,0,1,0,);(173,C0494831,atopic eczema,0,0,1,0,);(173,C0494831,eczema infantile, 1,0,0,0,);(173,C0494831,infantile eczema,0,0,1,0,);(174,C0495723,albuminuria,1,1,0,0,);(175,C0496594,alcoholism,1,0,1,0,);(176, C0496856,thyroid carcinoma,1,0,1,0,);(177,C0751362|C1305395,narcolepsy,1,1,0,0,);(178,C0995154,chronic back pain,0,1,0,0,);(178,C0995154,eye pain,1,0,0,0,);(179,C1265999,bowen disease,1,0,0,0,);(179,C1265999,cervical cancer,0,1,1,0,);(180,C1305395,daytime sleepiness,0,1,0,0,);(180,C1305395,excessive daytime sleepiness, 1,1,0,0,);(181,C1443045,obsessive-compulsive symptoms,0,1,0,0,);(181,C1443045,obsessive-compulsive disorder,1,1,0,0,);(182,C1735856,acute migraine,1,0,0,0,);(182,C1735856,basilar migraine,1,0,0,0,);(182,C1735856,classical migraine,1,0,0,0,);(182,C1735856,hemiplegic migraine,1,0,0,0,);(182,C1735856,migraine with aura,0,1,0,0,);(183,C2239176,hepatocellular carcinoma, 1,1,0,0,);(184,adenocarcinoma,adenocarcinoma,1,1,0,0,);(185,age-related macular degeneration,age-related macular degeneration,1,1,0,0,);(186,barrett oesophagus,barrett oesophagus,1,0,1,0,);(187,bipolar i disorder,bipolar i disorder,1,1,0,0,);(188,breast cancer,breast cancer,1,1,1,1,GSE124030);(188,breast cancer,breast cancer (dcis; ductal carcinoma in situ),0,0,0,1,GSE117970);(188,breast cancer,breast cancer (invasive breast cancer),0,0,0,1,GSE117970, GSE117970);(188,breast cancer,breast cancer (luminal b type),0,0,0,1,GSE110114);(189,candidemia,candidemia,1,1,0,0,);(190,colorectal cancer,colorectal cancer,1,1,1,0,);(190,colorectal cancer,colorectal tumor (moderately differentiated),0,0,0,1,GSE109203);(190,colorectal cancer,colorectal tumor (poorly differentiated),0,0,0,1,GSE109203);(190,colorectal cancer,colorectal tumor (well differentiated),0,0,0,1,GSE109203);(190,colorectal cancer,metastatic colorectal cancer to the liver (stage iv),0,0,0,1,GSE50760);(191,dandruff,dandruff,1,0,1,0,);(192,dementia with lewy bodies,dementia with lewy bodies,1,1,0,0,);(193,diabetic nephropathy,diabetic nephropathy,1,0,1,0,);(193,diabetic nephropathy,early diabetic nephropathy (dn),0,0,0,1,GSE142025);(194,endometrial cancer,endometrial cancer (upsc; uterine papillary serous carcinoma),0,0,0,1,GSE117970, GSE117970);(194,endometrial cancer,endometrial cancer (endometrioid),0,0,0,1,GSE117970, GSE117970);(194,endometrial cancer, endometrial tumor,0,0,0,1,GSE56087, GSE146889, GSE146889, GSE146889, GSE146889);(194,endometrial cancer,endometrial cancer (carcinoma),0,0,0,1,GSE117970);(194,endometrial cancer,endometrial cancer,1,1,1,0,);(194,endometrial cancer,endometrial cancer (carcinosarcoma),0,0,0,1,GSE117970);(195,ewing sarcoma,ewing sarcoma, 1,1,0,0,);(196,feeling guilty,feeling guilty, 1,1,0,0,);(197,glioblastoma,glioblastoma,1,1,0,0,);(198,glioma,glioma,1,1,0,0,);(199,hepar in-induced thrombocytopenia,heparin-induced thrombocytopenia,1,1,0,0,);(200,juvenile idiopathic arthritis,juvenile idiopathic arthritis,1,0,1,0,);(201,mean arterial pressure,mean arterial pressure,1,1,0,0,);(202,microalbuminuria,microalbuminuria,1,1,0,0,);(203,neuroblastoma,neuroblastoma,1,1,0,0,);(204,nicotine dependence,nicotine dependence,1,1,0,0,);(205,non- small cell lung cancer,non-small cell lung cancer, 1,1,0,0,);(206,osteosarcoma,osteosarcoma,1,1,0,0,);(207,ovarian cancer,ovarian cancer,1,1,0,0,);(208,post-traumatic stress disorder,post-traumatic stress disorder,1,1,0,0,);(209,prostate cancer,prostate cancer,1,1,1,0,);(210,reflux esophagitis,reflux esophagitis,1,0,1,0,);(211,renal cell carcinoma,renal cell carcinoma,1,1,0,0,);(212,sjogren syndrome,sjogren syndrome,1,0,1,0,);(213,stevens-johnson syndrome, stevens-johnson syndrome, 1,0,1,0,)。

<Listing 3: Novel marker>

Data are listed in the order (Disease ID for figure, UMLS ID, Tissue_gene, count). (1,C0001144|C0001145|C0022548|C0029485|C0152249|C0263437|C0263442|C0494863|C0702 166,Whole_blood_AKIRIN2,2911);(1,C0001144|C0001145|C0022548|C0029485|C0152249|C0 263437|C0263442|C0494863|C0702166,Monocyte_PER2,2255);(1,C0001144|C0001145|C0022 548|C0029485|C0152249|C0263437|C0263442|C0494863|C0702166,Colon_RAMP2,2035);(1,C 0001144|C0001145|C0022548|C0029485|C0152249|C0263437|C0263442|C0494863|C0702166, Unknown_CD86,1905);(1,C0001144|C0001145|C0022548|C0029485|C0152249|C0263437|C02 63442|C0494863|C0702166,Monocyte_RILP,1893);(1,C0001144|C0001145|C0022548|C002948 5|C0152249|C0263437|C0263442|C0494863|C0702166,Monocyte_SNX9,1875);(1,C0001144|C 0001145|C0022548|C0029485|C0152249|C0263437|C0263442|C0494863|C0702166,Endometri al tissue_TVP23CP2,1868);(1,C0001144|C0001145|C0022548|C0029485|C0152249|C0263437|C0 263442|C0494863|C0702166,Monocyte_EIF3G,1705);(1,C0001144|C0001145|C0022548|C0029485|C0152249|C0263437|C0263442|C0494863|C0702166,Endometrial tissue_SMC2,1699);(1,C0001144|C0001145|C0022548|C0029485|C0152249|C0263437|C0263442|C0494863|C0702166,Temporal_cortex_TRIM37,1687);(1,C0001144|C0001145|C0022548|C0029485|C0152249|C0263437|C0263442|C0494863|C0702166,Colorectum_VPS37B,1656);(1,C0001144|C0001145|C0022548|C0029485|C0152249|C0263437|C0263442|C0494863|C0702166, Brain white matter_STON1-GTF2A1L,1647);(1,C0001144|C0001145|C0022548|C0029485|C0152249|C0263437|C0263442|C0494863|C0702166,Endometrial tissue_TMEM159,1635);(1,C0001144|C0001145|C0022548|C0029485|C0152249|C0263437|C0263442|C0494863|C0702166,Endometrial tissue_GLB1,1550);(1,C0001144|C0001145|C0022548|C0029485|C0152249|C0263437|C0263442|C0494863|C0702166,Monocyte_PDHX,1494);(1,C0001144|C0001145|C0022548|C0029485|C0152249|C0263437|C0263442|C0494863|C0702166,Bladder_THEM6,1490);(1,C0001144|C0001145|C0022548|C0029485|C0152249|C0263437|C0263442|C0494863|C0702166,Temporal_cortex_MYADML2,1475);(1,C0001144|C0001145|C0022548|C0029485|C0152249|C0263437|C0263442|C0494863|C0702166,Brain white matter_CLEC12A,1473);(1,C0001144|C0001145|C0022548|C0029485|C0152249|C0263437|C0263442|C0494863|C0702166,Macrophage_MEPE,1424);(1,C0001144|C0001145|C0022548|C0029485|C0152249|C0263437|C0263442|C0494863|C0702166,Endometrial tissue_PODN,1416);(2,C0001339|C0153094|C0341465|C0341470|C0348760|C0348762,Whole_blood_AKIRIN2,2911);(2,C0001339|C0153094|C0341465|C0341470|C0348760|C0348762,Monocyte_PER2,2255);(2,C0001339|C0153094|C0341465|C0341470|C0348760|C0348762,Colon_RAMP2,2035);(2,C0001339|C0153094|C0341465|C0341470|C0348760|C0348762,Unknown_CD86,1905);(2,C0001339|C0153094|C0341465|C0341470|C0348760|C0348762,Monocyte_RILP,1893);(2,C0001339|C0153094|C0341465|C0341470|C0348760|C0348762,Monocyte_SNX9,1875 );(2,C0001339|C0153094|C0341465|C0341470|C0348760|C0348762,Endometrial tissue_TVP23CP2,1868);(2,C0001339|C0153094|C0341465|C0341470|C0348760|C0348762,Monocyte_EIF3G,1705);(2,C0001339|C0153094|C0341465|C0341470|C0348760|C0348762,Endometrial tissue_SMC2,1699);(2,C0001339|C0153094|C0341465|C0341470|C0348760|C0348762,Temporal_cortex_TRIM37,1687);(2,C0001339|C0153094|C0341465|C0341470|C0348760|C0348762,Colorectum_VPS37B,1656);(2,C0001339|C0153094|C0341465|C0341470|C0348760|C0348762,Brain white matter_STON1-GTF2A1L,1647);(2,C0001339|C0153094|C0341465|C0341470|C0348760|C0348762,Endometrial tissue_TMEM159,1635);(2,C0001339|C0153094|C0341465|C0341470|C0348760|C0348762,Endometrial tissue_GLB1,1550);(2,C0001339|C0153094|C0341465|C0341470|C0348760|C0348762,Monocyte_PDHX,1494);(2,C0001339|C0153094|C0341465|C0341470|C0348760|C0348762,Bladder_THEM6,1490);(2,C0001339|C0153094|C0341465|C0341470|C0348760|C0348762,Temporal_cortex_MYADML2,1475);(2,C0001339|C0153094|C0341465|C0341470|C0348760|C0348762,Brain white matter_CLEC12A,1473);(2,C0001339|C0153094|C0341465|C0341470|C0348760|C0348762,Macrophage_MEPE,1424);(2,C0001339|C0153094|C0341465|C0341470|C0348760|C0348762,Endometrial tissue_PODN,1416);(9,C0002171|C0477504,Whole_blood_AKIRIN2,2911);(9,C0002171|C0477504,Monocyte_PER2,2255);(9,C0002171|C0477504,Colon_RAMP2,2035);(9,C0002171|C0477504,Unknown_CD86,1905);(9,C0002171|C0477504,Monocyte_RILP,1893);(9,C0002171|C0477504,Monocyte_SNX9, 1875);(9,C0002171IC0477504,Endometrial tissue_TVP23CP2,1868);(9,C0002171|C0477504,Monocyte_EIF3G,1705);(9,C0002171|C0477504,Endometrial tissue_SMC2,1699);(9,C0002171|C0477504,Temporal_cortex_TRIM37,1687);(9,C0002171|C0477504,Colorectum_VPS37B,1656);(9,C0002171|C0477504,Brain white matter_STON1-GTF2A1L,1647);(9,C0002171|C0477504,Endometrial tissue_TMEM159,1635);(9,C0002171|C0477504,Endometrial tissue_GLB1,1550);(9,C0002171|C0477504,Monocyte_PDHX,1494);(9,C0002171|C0477504,Bladder_THEM6,1490);(9,C0002171|C0477504,Temporal_cortex_MYADML2,1475);(9,C000217 11C0477504,Brain white matter_CLEC12A,1473);(9,C0002171|C0477504,Macrophage_MEPE,1424);(9,C0002171|C047 7504,Endometrial tissue_PODN,1416);(10,C0003123|C0003125|C0338959|C0494440|C2267227,Whole_blood_AKIRIN2,2911);(10,C0003123|C0003125|C0338959|C0494440|C2267227,Monocyte_PER2,2255 );(10,C0003123|C0003125|C0338959|C0494440|C2267227,Colon_RAMP2,2035);(10,C0003123 |C0003125|C0338959|C0494440|C2267227,Unknown_CD86,1905);(10,C0003123|C0003125|C0338959|C0494440|C2267227,Monocyte_RILP,1893);(10,C0003123|C0003125|C0338959|C0494440|C2267227,Monocyte_SNX9,1875);(10,C0003123|C0003125|C0338959|C0494440|C2267227,Endometrial tissue_TVP23CP2,1868);(10,C0003123|C0003125|C0338959|C0494440|C2267227,Monocyte_EIF3G,1705);(10,C0003123|C0003125|C0338959|C0494440|C2267227,Endometrial tissue_SMC2,1699);(10,C0003123|C0003125|C0338959|C0494440|C2267227,Temporal_cortex _TRIM37,1687);(10,C0003123|C0003125|C0338959|C0494440|C2267227,Colorectum_VPS37 B,1656);(10,C0003123|C0003125|C0338959|C0494440|C2267227,Brain white matter_STON1-GTF2A1L,1647);(10,C0003123|C0003125|C0338959|C0494440|C2267227,Endometrial tissue_TMEM159,1635);(10,C0003123|C0003125|C0338959|C0494440|C2267227,Endometrial tissue_GLB1,1550);(10,C0003123|C0003125|C0338959|C0494440|C2267227,Monocyte_PDHX ,1494);(10,C0003123|C0003125|C0338959|C0494440|C2267227,Bladder_THEM6,1490);(10,C0003123|C0003125|C0338959|C0494440|C2267227,Temporal_cortex_MYADML2,1475);(10,C0003123|C0003125|C0338959|C0494440|C2267227,Brain white matter_CLEC12A,1473);(10,C0003123|C0003125|C0338959|C0494440|C2267227,Macrophage_MEPE,1424);(10,C0003123|C0003125|C0338959|C0494440|C2267227,Endometrial tissue_PODN,1416);(13,C0003615|C0031154|C0085693|C0152505|C0156095,Whole_blood_AKIRIN2,2911);(13,C0003615|C0031154|C0085693|C0152505|C0156095,Monocyte_PER2,2255 );(13,C0003615|C0031154|C0085693|C0152505|C0156095,Colon_RAMP2,2035);(13,C0003615|C0031154|C0085693|C0152505|C0156095,Unknown_CD86,1905);(13,C0003615|C0031154|C0085693|C0152505|C0156095,Monocyte_RILP,1893);(13,C0003615|C0031154|C0085693|C0152505|C0156095,Monocyte_SNX9,1875);(13,C0003615|C0031154|C0085693|C0152505|C0156095,Endometrial tissue_TVP23CP2,1868);(13,C0003615|C0031154|C0085693|C0152505|C0156095,Monocyte_EIF3G,1705);(13,C0003615|C0031154|C0085693|C0152505|C0156095,Endometrial tissue_SMC2,1699);(13,C0003615|C0031154|C0085693|C0152505|C0156095,Temporal_cortex _TRIM37,1687);(13,C0003615|C0031154|C0085693|C0152505|C0156095,Colorectum_VPS37 B,1656);(13,C0003615|C0031154|C0085693|C0152505|C0156095,Brain white matter_STON1-GTF2A1L,1647);(13,C0003615|C0031154|C0085693|C0152505|C0156095,Endometrial tissue_TMEM159,1635);(13,C0003615|C0031154|C0085693|C0152505|C0156095,Endometrial tissue_GLB1,1550);(13,C0003615|C0031154|C0085693|C0152505|C0156095,Monocyte_PDHX ,1494);(13,C0003615|C0031154|C0085693|C0152505|C0156095,Bladder_THEM6,1490);(13,C0003615|C0031154|C0085693|C0152505|C0156095,Temporal_cortex_MYADML2,1475);(13,C0003615|C0031154|C0085693|C0152505|C0156095,Brain white matter_CLEC12A,1473);(13,C0003615|C0031154|C0085693|C0152505|C0156095,Macrophage_MEPE,1424);(13,C0003615|C0031154|C0085693|C0152505|C0156095,Endometrial tissue_PODN,1416);(16,C0003873|C0015773|C0026848|C0029660|C0038013|C0409651|C0409652|C0477411|C0477541|C0477542|C0494896|C0494897|C0694497|C3714757,Whole_blood_AKIRIN2,2911);(16,C0003873|C0015773|C0026848|C0029660|C0038013|C0409651|C0409652|C0477411|C0477541|C0477542|C0494896|C0494897|C0694497|C3714757,Monocyte_PER2,2255);(16,C0003873|C0015773|C0026848|C0029660|C0038013|C0409651|C0409652|C0477411|C0477541|C0477542|C0494896|C0494897|C0694497|C3714757,Colon_RAMP2,2035);(16,C0003873|C0015773|C0026848|C0029660|C0038013|C0409651|C0409652|C0477411|C0477541|C0477542|C0494896|C0494897|C0694497|C3714757,Unknown_CD86,1905);(16,C0003873|C0015773|C0026848|C0029660|C0038013|C0409651|C0409652|C0477411|C0477541|C0477542|C0494896|C0494897|C0694497|C3714757,Monocyte_RILP,1893);(16,C0003873|C0015773|C0026848|C0029660|C0038013|C0409651|C0409652|C0477411|C0477541|C0477542|C0494896|C0494897|C0694497|C3714757,Monocyte_SNX9,1875);(16,C0003873|C0015773|C0026848|C0029660|C0038013|C0409651|C0409652|C04774111C0477541|C0477542|C0494896|C0494897|C0694497|C3714757,En-dometrial tissue_TVP23CP2,1868);(16,C0003873|C0015773|C0026848|C0029660|C0038013|C0409651|C0409652|C0477411|C0477541|C0477542|C0494896|C0494897|C0694497|C3714757,Monocyte,EIF3G,1705);(16,C0003873|C0015773|C0026848|C0029660|C0038013|C0409651|C0409652|C0477411|C0477541|C0477542|C0494896|C0494897|C0694497IC3714757,Endometrial tissue_SMC2,1699);(16,C0003873|C0015773|C0026848|C0029660|C0038013|C0409651|C0409652|C0477411|C0477541|C0477542|C0494896|C0494897|C0694497|C3714757,Temporal_cortex_TRIM37,1687);(16,C0003873|C0015773|C0026848|C0029660|C0038013|C0409651|C0409652|C0477411|C0477541|C0477542|C0494896|C0494897|C0694497|C3714757,Colorectum_VPS37B,1656);(16,C0003873|C0015773|C0026848|C0029660|C0038013|C0409651|C0409652|C0477411|C0477541|C0477542|C0494896|C0494897|C0694497|C3714757,Brain white matter_STON1-GTF2A1L,1647);(16,C0003873|C0015773|C0026848|C0029660|C0038013|C0409651|C0409652|C0477411|C0477541|C0477542|C0494896|C0494897|C0694497|C3714757,Endometrial tissue_TMEM159,1635);(16,C0003873|C0015773|C0026848|C0029660|C0038013|C0409651|C0409652|C0477411|C0477541|C0477542|C0494896|C0494897|C0694497|C3714757,Endometrial tissue_GLB1,1550);(16,C0003873|C0015773|C0026848|C0029660|C0038013|C0409651|C0409 652|C0477411|C0477541|C0477542|C0494896|C0494897|C0694497|C3714757,Monocyte_PDHX,1494);(16,C0003873|C0015773|C0026848|C0029660|C0038013|C0409651|C0409652|C0477411|C0477541|C0477542|C0494896|C0494897|C0694497|C3714757,Bladder_THEM6,1490);(16,C0003873|C0015773|C0026848|C0029660|C0038013|C0409651|C0409652|C0477411|C0477541|C0477542|C0494896|C0494897|C0694497|C3714757,Temporal_cortex_MYADML2,1475);(16,C0003873|C0015773|C0026848|C0029660|C0038013|C0409651|C0409652|C0477411|C0477541|C0477542|C0494896|C0494897|C0694497|C3714757,Brain white matter_CLEC12A,1473);(16,C0003873|C0015773|C0026848|C0029660|C0038013|C0409651|C0409652|C0477411|C0477541|C0477542|C0494896|C0494897|C0694497|C3714757,Macrophage_MEPE,1424);(16,C0003873|C0015773|C0026848|C0029660|C0038013|C0409651|C0409652|C0477411|C0477541|C0477542|C0494896|C0494897|C0694497|C3714757,Endometrial tissue_PODN,1416);(17,C0003962|C0036528|C0152717|C0156180|C0477842|C0495216|C0495676|C0495700,Whole_blood_AKIRIN2,2911);(17,C0003962|C0036528|C0152717|C0156180|C0477842|C0495216|C0495676|C0495700,Monocyte_PER2,2255);(17,C0003962|C0036528|C0152717|C0156180|C0477842|C0495216|C0495676|C0495700,Colon_RAMP2,2035);(17,C0003962|C0036528|C0152717|C0156180|C0477842|C0495216|C0495676|C0495700,Unknown_CD86,1905);(17,C0003962|C0036528|C0152717|C0156180|C0477842|C0495216|C0495676|C0495700,Monocyte_RILP,1893);(17,C0003962|C0036528|C0152717|C0156180|C0477842|C0495216|C0495676|C0495700,Monocyte_SNX9,1875);(17,C0003962|C0036528|C0152717|C0156180|C0477842|C0495216|C0495676|C0495700,Endometrial tissue_TVP23CP2,1868);(17,C0003962|C0036528|C0152717|C0156180|C0477842|C0495216|C0495676|C0495700,Monocyte_EIF3G,1705);(17,C0003962|C0036528|C0152717|C0156180|C0477842|C0495216|C0495676|C0495700,Endometrial tissue_SMC2,1699);(17,C0003962|C0036528|C0152717|C0156180|C0477842|C0495216|C0495676|C0495700,Temporal_cortex_TRIM37,1687);(17,C0003962|C0036528|C0152717|C0156180|C0477842|C0495216|C0495676|C0495700,Colorectum_VPS37B,1656);(17,C0003962|C0036528|C0152717|C0156180|C0477842|C0495216|C0495676|C0495700,Brain white matter_STON1-GTF2A1L,1647);(17,C0003962|C0036528|C0152717|C0156180|C0477842|C0495216|C0495676|C0495700,Endometrial tissue_TMEM159,1635);(17,C0003962|C0036528|C0152717|C0156180|C0477842|C0495216|C0495676|C0495700,Endometrial tissue_GLB1,1550);(17,C0003962|C0036528|C0152717|C0156180|C0477842|C0495216|C0495676|C0495700,Monocyte_PDHX,1494);(17,C0003962|C0036528|C0152717|C0156180|C0477842|C0495216|C0495676|C0495700,Bladder_THEM6,1490);(17,C0003962|C0036528|C0152717|C0156180|C0477842|C0495216|C0495676|C0495700,Temporal_cortex_MYADML2,1475);(17,C0003962|C0036528|C0152717|C0156180|C0477842|C0495216|C0495676|C0495700,Brain white matter_CLEC12A,1473);(17,C0003962|C0036528|C0152717|C0156180|C0477842|C0495216|C0495676|C0495700,Macrophage_MEPE,1424);(17,C0003962|C0036528|C0152717|C0156180|C0477842|C0495216|C0495676|C0495700,Endometrial tissue_PODN,1416);(19,C0004153|C0007775|C0010054|C0017327|C0155733|C0155734|C0348648|C0494764|C0495064|C0495663|C3495604,Whole_blood_AKIRIN2,2911);(19,C0004153|C0007775|C0010054|C0017327|C0155733|C0155734|C0348648|C0494764|C0495064|C0495663|C3495604,Monocyte_PER2,2255);(19,C0004153|C0007775|C0010054|C0017327|C0155733|C0155734|C0348648|C0494764|C0495064|C0495663|C3495604,Colon_RAMP2,2035);(19,C0004153|C0007775|C0010054|C0017327|C0155733|C0155734|C0348648|C0494764|C0495064|C0495663|C3495604,Unknown_CD86,1905);(19,C0004153|C0007775|C0010054|C0017327|C0155733|C0155734|C0348648|C0494764|C0495064|C0495663|C3495604,Monocyte_RILP,1893);(19,C0004153|C0007775|C0010054|C0017327|C0155733|C0155734|C0348648|C0494764|C0495064|C0495663|C3495604,Monocyte_SNX9,1875);(19,C0004153|C0007775|C0010054|C0017327|C0155733|C0155734|C0348648|C0494764|C0495064|C0495663|C3495604,Endometrial tissue_TVP23CP2,1868);(19,C0004153|C0007775|C0010054|C0017327|C0155733|C0155734|C0348648|C0494764|C0495064|C0495663|C3495604,Monocyte_EIF3G,1705);(19,C0004153|C0007775|C0010054|C0017327|C0155733|C0155734|C0348648|C0494764|C0495064|C0495663|C3495604,Endometrial tissue_SMC2,1699);(19,C0004153|C0007775|C0010054|C0017327|C0155733|C0155734|C0348648|C0494764|C0495064|C0495663|C3495604,Temporal_cortex_TRIM37,1687);(19,C0004153|C0007775|C0010054|C0017327|C0155733|C0155734|C0348648|C0494764|C0495064|C0495663|C3495604,Colorectum_VPS37B,1656);(19,C0004153|C0007775|C0010054|C0017327|C0155733|C0155734|C0348648|C0494764|C0495064|C0495663|C3495604,Brain white matter_STON1-GTF2A1L,1647);(19,C0004153|C0007775|C0010054|C0017327|C0155733|C0155734|C034864 8|C0494764|C0495064|C0495663|C3495604,Endometrial tissue_TMEM159,1635);(19,C0004153|C0007775|C0010054|C0017327|C0155733|C0155734|C0348648|C0494764|C0495064|C0495663|C3495604,Endometrial tissue_GLB1,1550);(19,C0004153|C0007775|C0010054|C0017327|C0155733|C0155734|C0348648|C0494764|C0495064|C0495663|C3495604,Monocyte_PDHX,1494);(19,C0004153|C0007775|C0010054|C0017327|C0155733|C0155734|C0348648|C0494764|C0495064|C0495663|C3495604,Bladder_THEM6,1490);(19,C0004153|C0007775|C0010054|C0017327|C0155733|C0155734|C0348648|C0494764|C0495064|C0495663|C3495604,Temporal_cortex_MYADML2,1475);(19,C0004153|C0007775|C0010054|C0017327|C0155733|C0155734|C0348648|C0494764|C0495064|C0495663|C3495604,Brain white matter_CLEC12A,1473);(19,C0004153|C0007775|C0010054|C0017327|C0155733|C0155734|C0348648|C0494764|C0495064|C0495663|C3495604,Macrophage_MEPE,1424);(19,C0004153|C0007775|C0010054|C0017327|C0155733|C0155734|C0348648|C0494764|C0495064|C0495663|C3495604,Endometrial tissue_PODN,1416);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Whole_blood_AKIRIN2,2911);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Monocyte_PER2,2255);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Colon_RAMP2,2035);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Unknown_CD86,1905);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Monocyte_RILP,1893);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C034 3323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Monocyte_SNX9,1875);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0 869210,Endometrial tissue_TVP23CP2,1868);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Monocyte_EIF3G,1705);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Endometrial tissue_SMC2,1699);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Temporal_cortex_TRIM37,1687);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Colorectum_VPS37B,1656);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Brain white matter_STON1-GTF2A1L,1647);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339 961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Endometrial tissue_TMEM159,1635);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Endometrial tissue_GLB1,1550);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Monocyte_PDHX,1494);(21,CO004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Bladder_THEM6,1490);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Temporal_cortex_MYADML2,1475);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Brain white matter_CLEC12A,1473);(21,C0004626|C0032285|C0032300|C0032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Macrophage_MEPE,1424);(21,C0004626|C0032285|C0032300|C0 032302|C0032308|C0032310|C0034063|C0152413|C0152487|C0152502|C0155858|C0155860|C0155862|C0158935|C0158944|C0206062|C0260334|C0264522|C0276026|C0276089|C0276156|C0276333|C0339951|C0339959|C0339961|C0339969|C0339971|C0343320|C0343323|C0343324|C0343666|C0348152|C0348161|C0348193|C0348677|C0348678|C0348679|C0348680|C0348681|C0348684|C0348685|C0348703|C0348801|C0348804|C0348806|C0348809|C0348814|C0348968|C0348970|C0349359|C0349468|C0375326|C0477908|C0477909|C0494034|C0494059|C0494086|C0494137|C0494638|C0494639|C0494640|C0494643|C0494644|C0494675|C0494678|C0494683|C0495389|C0495390|C0519030|C0694504|C0869210,Endometrial tissue_PODN,1416);(25,C0006267|C0152239|C0348809|C0348968|C0431530|C0494034|C0494659,Whole_blood_AKIRIN2,2911);(25,C0006267|C0152239|C0348809|C0348968|C0431530|C0494034|C0494659,Monocyte_PER2,2255);(25,C0006267|C0152239|C0348809|C0348968|C0431530|C0494034|C0494659,Colon_RAMP2,2035);(25,C0006267|C0152239|C0348809|C0348968|C0431530|C0494034|C0494659,Unknown_CD86,1905);(25,C0006267|C0152239|C0348809|C0348968|C0431530|C0494034|C0494659,Monocyte_RILP,1893);(25,C0006267|C0152239|C0348809|C0348968|C0431530|C0494034|C0494659,Monocyte_SNX9,1875);(25,C0006267|C0152239|C0348809|C0348968|C0431530|C0494034|C0494659,Endometrial tissue_TVP23CP2,1868);(25,C0006267|C0152239|C0348809|C0348968|C0431530|C0494034|C0494659,Monocyte_EIF3G,1705);(25,C0006267|C0152239|C0348809|C0348968|C0431530|C0494034|C0494659, Endometrial tissue_SMC2,1699);(25,C0006267|C0152239|C0348809|C0348968|C0431530|C0494034|C0494659,Temporal_cortex_TRIM37,1687);(25,C0006267|C0152239|C0348809|C0348968|C0431530|C0494034|C0494659,Colorectum_VPS37B,1656);(25,C0006267|C0152239|C0348809|C0348968|C0431530|C0494034|C0494659,Brain white matter_STON1-GTF2A1L,1647);(25,C0006267|C0152239|C0348809|C0348968|C0431530|C0494034|C0494659,Endometrial tissue_TMEM159,1635);(25,C0006267|C0152239|C0348809|C0348968|C0431530|C0494034|C0494659,Endometrial tissue_GLB1,1550);(25,C0006267|C0152239|C0348809|C0348968|C0431530|C0494034|C0494659,Monocyte_PDHX,1494);(25,C0006267|C0152239|C0348809|C0348968|C0431530|C0494034|C0494659,Bladder_THEM6,1490);(25,C0006267|C0152239|C0348809|C0348968|C0431530|C0494034|C0494659,Temporal_cortex_MYADML2,1475);(25,C0006267|C0152239|C0348809|C0348968|C0431530|C0494034|C0494659,Brain white matter_CLEC12A,1473);(25,C0006267|C0152239|C0348809|C0348968|C0431530|C0494034|C0494659,Macrophage_MEPE,1424);(25,C0006267|C0152239|C0348809|C0348968|C0431530|C0494034|C0494659,Endometrial tissue_PODN,1416);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Whole_blood_AKIRIN2,2911);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Monocyte_PER2,2255);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Colon_RAMP2,2035);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Unknown_CD86,1905);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Monocyte_RILP,1893);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Monocyte_SNX9,1875);(29,C0007820|C0013537|C0017614|C0018801|C0020538|C0020541|C0020545|C0022661|C003 1190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Endometrial tissue_TVP23CP2,1868);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Monocyte_EIF3G,1705);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968, Endometrial tissue_SMC2,1699);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Temporal_cortex_TRIM37,1687);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Colorectum_VPS37B,1656);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Brain white matter_STON1-GTF2A1L,1647);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Endometrial tissue_TMEM159,1635);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,En-dometrial tissue_GLB1,1550);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Monocyte_PDHX,1494);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Bladder_THEM6,1490);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Temporal_cortex_MYADML2,1475);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Brain white matter_CLEC12A,1473);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C0848548|C3495403|C3648968,Macrophage_MEPE,1424);(29,C0007820|C0013537|C0017614|C0018801|C0020538|C0020541|C0020545|C0022661|C0031190|C0033845|C0035078|C0085580|C0151744|C0152105|C0152171|C0155616|C0155711|C0348586|C0348587|C0348752|C0348859|C0349368|C0392682|C0393561|C0452204|C0495174|C0495176|C0495177|C0495178|C0495180|C0495183|C08 48548|C3495403|C3648968, Endometrial tissue_PODN,1416);(30,C0007860|C0014719|C0348904|C0477866|C0494054,Whole_blood_AKIRIN2,2911);(30,C0007860|C0014719|C0348904|C0477866|C0494054,Monocyte_PER2,2255 );(30,C0007860|C0014719|C0348904|C0477866|C0494054,Colon_RAMP2,2035);(30,C0007860|C0014719|C0348904|C0477866|C0494054,Unknown_CD86,1905);(30,C0007860|C0014719|C0348904|C0477866|C0494054,Monocyte_RILP,1893);(30,C0007860|C0014719|C0348904|C0477866|C0494054,Monocyte_SNX9,1875);(30,C0007860|C0014719|C0348904|C0477866|C0494054,Endometrial tissue_TVP23CP2,1868);(30,C0007860|C0014719|C0348904|C0477866|C0494054,Monocyte_EIF3G,1705);(30,C0007860|C0014719|C0348904|C0477866|C0494054, Endometrial tissue_SMC2,1699);(30,C0007860|C0014719|C0348904|C0477866|C0494054,Temporal_cortex_TRIM37,1687);(30,C0007860|C0014719|C0348904|C0477866|C0494054,Colorectum_VPS37B,1656);(30,C0007860|C0014719|C0348904|C0477866|C0494054,Brain white matter_STON1-GTF2A1L,1647);(30,C0007860|C0014719|C0348904|C0477866|C0494054,Endometrial tissue_TMEM159,1635);(30,C0007860|C0014719|C0348904|C0477866|C0494054,Endometrial tissue_GLBI,1550);(30,C0007860|C0014719|C0348904|C0477866|C0494054,Monocyte_PDHX ,1494);(30,C0007860|C0014719|C0348904|C0477866|C0494054,Bladder_THEM6,1490);(30,C0007860|C0014719|C0348904|C0477866|C0494054,Temporal_cortex_MYADML2,1475);(30,C0007860|C0014719|C0348904|C0477866|C0494054,Brain white matter_CLEC12A,1473);(30,C0007860|C0014719|C0348904|C0477866|C0494054,Macrophage_MEPE,1424);(30,C0007860|C0014719|C0348904|C0477866|C0494054, Endometrial tissue_PODN,1416);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Whole_blood_AKIRIN2,2911);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476 550|C0476570|C0476572|C0476635|C0477322|C0477391|C0477762|C0477808|C0477809|C047
7810|C0477811|C0477812|C0477813|C0477814|C0477815|C0477816|C0477817|C0477819|C04
77820|C0477865|C0477876|C0477878|C0477881|C0477885|C0478521|C0478618|C0494048|C0
494450|C0494514|C0494598|C0494783|C0495168|C0495169|C0495170|C0495171|C0495172|C
0495173|C0495174|C0495175|C0495176|C0495177|C0495180|C0495184|C0495187|C0495188|
C0495189|C0495190|C0495192|C0495196|C0495197|C0495198|C0495199|C0495224|C049522
5|C0495227|C0495251|C0495292|C0495301|C0495302|C0495304|C0495308|C0495309|C04953     10|C0495311
|C0495312|C0495313                                    |C0495314|C0495315|C0495316|C0495317|C0495318|C0495
351|C0495699|C0495706|C0496132|C0496136|C0496158|C0496641|C0496642|C0496643|C049
6644|C0496645|C0496646|C0496693|C0496727|C0694449|C0694451|C0694452|C0694454|C06
94456|C0700573|C0728889|C0728936|C0851207|C0851208|C0851209|C0851217|C0869210|C0
869272|C0878544|C1314803|C1367972|C1533618|C1533626|C1704300|C3146283|C3648968,
Monocyte_PER2,2255);(31,C0007871|C0011849|C0011854|C0011860|C0012242|C0012739|C0
013537|C0013927|C0018790|C0018801|C0019693|C0020450|C0020538|C0021361|C0024528|C
0027498|C0027765|C0028962|C0029496|C0029604|C0031154|C0032969|C0032984|C0032987|
C0032989|C0032991|C0032993|C0032994|C0033010|C0034065|C0035078|C0035204|C003698
6|C0038843|C0039070|C0039236|C0080276|C0085207|C0085740|C0152150|C0152151|C01521
52|C0152964|C0155749|C0155774|C0155778|C0155796|C0156373|C0156543|C0156604|C0156
677|C0156699|C0156703|C0156756|C0157535|C0158806|C0158816|C0161754|C0178269|C017
8288|C0178298|C0233214|C0242172|C0260550|C0260551|C0263660|C0269294|C0269661|C02
69672|C0271641|C0275667|C0275820|C0302487|C0332544|C0339951|C0340747|C0342044|C0
342054|C0342068|C0348447|C0348786|C0348859|C0348877|C0348881|C0348950|C0349253|C
0362068|C0391989|C0392070|C0405328|C0411164|C0411169|C0451717|C0451783|C0451790|     C0451791
|C0451792|C0451799|C0451800|C0451801                           |C0451802|C0451803|C0451810|C045181
4|C0451816|C0451822|C0452164|C0452165|C0475523|C0475578|C0476442|C0476491|C04765
50|C0476570|C0476572|C0476635|C0477322|C0477391|C0477762|C0477808|C0477809|C0477
810|C0477811|C0477812|C0477813|C0477814|C0477815|C0477816|C0477817|C0477819|C047
7820|C0477865|C0477876|C0477878|C0477881|C0477885|C0478521|C0478618|C0494048|C04
94450|C0494514|C0494598|C0494783|C0495168|C0495169|C0495170|C0495171|C0495172|C0
495173|C0495174|C0495175|C0495176|C0495177|C0495180|C0495184|C0495187|C0495188|C
0495189|C0495190|C0495192|C0495196|C0495197|C0495198|C0495199|C0495224|C0495225|
C0495227|C0495251|C0495292|C0495301|C0495302|C0495304|C0495308|C0495309|C049531
0|C0495311|C0495312|C0495313|C0495314|C0495315|C0495316|C0495317|C0495318|C04953
51|C0495699|C0495706|C0496132|C0496136|C0496158|C0496641|C0496642|C0496643|C0496
644|C0496645|C0496646|C0496693|C0496727|C0694449|C0694451|C0694452|C0694454|C069
4456|C0700573|C0728889|C0728936|C0851207|C0851208|C0851209|C0851217|C0869210|C08
69272|C0878544|C1314803|C1367972|C1533618|C1533626|C1704300|C3146283|C3648968,Co
lon_RAMP2,2035);(31,C0007871|C0011849|C0011854|C0011860|C0012242|C0012739|C00135
37|C0013927|C0018790|C0018801|C0019693|C0020450|C0020538|C0021361|C0024528|C0027
498|C0027765|C0028962|C0029496|C0029604|C0031154|C0032969|C0032984|C0032987|C003
2989|C0032991|C0032993|C0032994|C0033010|C0034065|C0035078|C0035204|C0036986|C00
38843|C0039070|C0039236|C0080276|C0085207|C0085740|C0152150|C0152151|C0152152|C0
152964|C0155749|C0155774|C0155778|C0155796|C0156373|C0156543|C0156604|C0156677|C
0156699|C0156703|C0156756|C0157535|C0158806|C0158816|C0161754|C0178269|C0178288|
C0178298|C0233214|C0242172|C0260550|C0260551|C0263660|C0269294|C0269661|C026967
2|C0271641|C0275667|C0275820|C0302487|C0332544|C0339951|C0340747|C0342044|C03420
54|C0342068|C0348447|C0348786|C0348859|C0348877|C0348881|C0348950|C0349253|C0362
068|C0391989|C0392070|C0405328|C0411164|C0411169|C0451717|C0451783|C0451790|C045
1791|C0451792|C0451799|C0451800|C0451801|C0451802|C0451803|C0451810|C0451814|C04
51816|C0451822|C0452164|C0452165|C0475523|C0475578|C0476442|C0476491|C0476550|C0
476570|C0476572|C0476635|C0477322|C0477391|C0477762|C0477808|C0477809|C0477810|C
0477811|C0477812|C0477813|C0477814|C0477815|C0477816|C0477817|C0477819|C0477820|
C0477865|C0477876|C0477878|C0477881|C0477885|C0478521|C0478618|C0494048|C049445     73
0|C0494514|C0494598|C0494783|C0495168|C0495169|C0495170|C0495171|C0495172|C04951
|C0495174|C0495175|C0495176|C0495177|C0495180|C0495184|C0495187|C0495188|C0495
189|C0495190|C0495192|C0495196|C0495197|C0495198|C0495199|C0495224|C0495225|C049
5227|C0495251|C0495292|C0495301|C0495302|C0495304|C0495308|C0495309|C0495310|C04
95311|C0495312|C0495313|C0495314|C0495315|C0495316|C0495317|C0495318|C0495351|C0 495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Unknown_CD86,1905);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Monocyte_RILP,1893);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Monocyte_SNX9,1875);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0 039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Endometrial tissue_TVP23CP2,1868);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968, Monocyte_EIF3G,1705);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C04518 14|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Endometrial tissue_SMC2,1699);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Temporal_cortex_TRIM37,1687);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C04518101C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C049 5310|C0495311|C0495312|C0495313 |C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Colorectum_VPS37B,1656);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Brain white matter_STON1-GTF2A1L,1647);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Endometrial tissue_TMEM159,1635);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C003298 7|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Endometrial tissue_GLB1,1550);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Monocyte_PDHX,1494);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C045 1791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Bladder_THEM6,1490);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643 |C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968,Temporal_cortex_MYADML2,1475);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C04952 25|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968, Brain white matter_CLEC12A,1473);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968, Macrophage_MEPE,1424);(31,C0007871|C0011849|C0011854|C0011860|C0012242|C0012739|C0013537|C0013927|C0018790|C0018801|C0019693|C0020450|C0020538|C0021361|C0024528|C0027498|C0027765|C0028962|C0029496|C0029604|C0031154|C0032969|C0032984|C0032987|C0032989|C0032991|C0032993|C0032994|C0033010|C0034065|C0035078|C0035204|C0036986|C0038843|C0039070|C0039236|C0080276|C0085207|C0085740|C0152150|C0152151|C0152152|C0152964|C0155749|C0155774|C0155778|C0155796|C0156373|C0156543|C0156604|C0156677|C0156699|C0156703|C0156756|C0157535|C0158806|C0158816|C0161754|C0178269|C0178288|C0178298|C0233214|C0242172|C0260550|C0260551|C0263660|C0269294|C0269661|C0269672|C0271641|C0275667|C0275820|C0302487|C0332544|C0339951|C0340747|C0342044|C0342054|C0342068|C0348447|C0348786|C0348859|C0348877|C0348881|C0348950|C0349253|C0362068|C0391989|C0392070|C0405328|C0411164|C0411169|C0451717|C0451783|C0451790|C0451791|C0451792|C0451799|C0451800|C0451801|C0451802|C0451803|C0451810|C0451814|C0451816|C0451822|C0452164|C0452165|C0475523|C0475578|C0476442|C0476491|C0476550|C0476570|C0476572|C0476635|C0477322|C0477391|C0477762|C0477808|C0477809|C0477810|C0477811|C0477812|C0477813|C0477814|C0477815|C0477816|C0477817|C0477819|C0477820|C0477865|C0477876|C0477878|C0477881|C0477885|C0478521|C0478618|C0494048|C0494450|C0494514|C0494598|C0494783|C0495168|C0495169|C0495170|C0495171|C0495172|C0495173|C0495174|C0495175|C0495176|C0495177|C0495180|C0495184|C0495187|C0495188|C0495189|C0495190|C0495192|C0495196|C0495197|C0495198|C0495199|C0495224|C0495225|C0495227|C0495251|C0495292|C0495301|C0495302|C0495304|C0495308|C0495309|C0495310|C0495311|C0495312|C0495313|C0495314|C0495315|C0495316|C0495317|C0495318|C0495351|C0495699|C0495706|C0496132|C0496136|C0496158|C0496641|C0496642|C0496643|C0496644|C0496645|C0496646|C0496693|C0496727|C0694449|C0694451|C0694452|C0694454|C0694456|C0700573|C0728889|C0728936|C0851207|C0851208|C0851209|C0851217|C0869210|C0869272|C0878544|C1314803|C1367972|C1533618|C1533626|C1704300|C3146283|C3648968, Endometrial tissue_PODN,1416);(32,C0008312,Whole_blood_AKIRIN2,2911);(32,C0008312,Whole_blood_AKIRIN2,2911);(32,C0008312,Whole_blood_AKIRIN2,2911);(32,C0008312,Monocyte_PER 2,2255);(32,C0008312,Monocyte_PER2,2255);(32,C0008312,Monocyte_PER2,2255);(32,C0008312,Colon_RAMP2,2035);(32,C0008312,Colon_RAMP2,2035);(32,C0008312,Colon_RAMP2 ,2035);(32,C0008312,Unknown_CD86,1905);(32,C0008312,Unknown_CD86,1905);(32,C0008312,Unknown_CD86,1905);(32,C0008312,Monocyte_RILP,1893);(32,C0008312,Monocyte_RILP,1893);(32,C0008312,Monocyte_RILP,1893);(32,C0008312,Monocyte_SNX9,1875);(32,C0008312,Monocyte_SNX9,1875);(32,C0008312,Monocyte_SNX9,1875);(32,C0008312,Endometrial tissue_TVP23CP2,1868);(32,C0008312,Endometrial tissue_TVP23CP2,1868);(33,C0008677|C0024117|C0264222|C0348686|C0348693|C0348817|C0348818|C0494659,Whole_blood_AKIRIN2,2911);(33,C0008677|C0024117|C0264222|C0348686|C0348693|C0348817|C0348818|C0494659,Monocyte_PER2,2255);(33,C0008677|C0024117|C0264222|C0348686|C0348693|C0348817|C0348818|C0494659,Colon_RAMP2,2035);(33,C0008677|C0024117|C0264222|C0348686|C0348693|C0348817|C0348818|C0494659,Unknown_CD86,1905);(33,C0008677|C0024117|C0264222|C0348686|C0348693|C0348817|C0348818|C0494659,Monocyte_RILP,1893);(33,C0008677|C0024117|C0264222|C0348686|C0348693|C0348817|C0348818|C0494659,Monocyte_SNX9,1875);(33,C0008677|C0024117|C0264222|C0348686|C0348693|C0348817|C0348818|C0494659,Endometrial tissue_TVP23CP2,1868);(33,C0008677|C0024117|C0264222|C0348686|C0348693|C0348817|C0348818|C0494659,Monocyte_EIF3G,1705);(33,C0008677|C0024117|C0264222|C0348686|C0348693|C0348817|C0348818|C0494659,Endometrial tissue_SMC2,1699);(33,C0008677|C0024117|C0264222|C0348686|C0348693|C0348817|C0348818|C0494659,Temporal_cortex_TRIM37,1687);(33,C0008677|C0024117|C0264222|C0348686|C0348693|C0348817|C0348818|C0494659,Colorectum_VPS37B,1656);(33,C0008677|C0024117|C0264222|C0348686|C0348693|C0348817|C0348818|C0494659,Brain white matter_STON1-GTF2A1L,1647);(33,C0008677|C0024117|C0264222|C0348686|C0348693|C0348817|C0348818|C0494659,Endometrial tissue_TMEM159,1635);(33,C0008677|C0024117|C0264222|C0348686|C0348693|C0348817|C0348818|C0494659,Endometrial tissue_GLB1,1550);(33,C0008677|C0024117|C0264222|C0348686|C0348693|C0348817|C0348818|C0494659,Monocyte_PDHX,1494);(33,C0008677|C0024117|C0264222|C0348686|C0348693|C0348817|C0348818|C0494659,Bladder_THEM6,1490);(33,C0008677|C0024117|C0264222|C0348686|C0348693|C0348817|C0348818|C0494659,Temporal_cortex_MYADML2,1475);(33,C0008677|C0024117|C0264222|C0348686|C0348693|C0348817|C0348818|C0494659,Brain white matter_CLEC12A,1473);(33,C0008677|C0024117|C0264222|C0348686|C0348693|C0348817|C0348818|C0494659,Macrophage_MEPE,1424);(33,C0008677|C0024117|C0264222|C0348686|C0348693|C0348817|C0348818|C0494659,Endometrial tissue_PODN,1416);(34,C0008684|C0155937|C0348160|C0494704|C0494706,Whole_blood_AKIRIN2,2911);(34,C0008684|C0155937|C0348160|C0494704|C0494706,Monocyte_PER2,2255 );(34,C0008684|C0155937|C0348160|C0494704|C0494706,Colon_RAMP2,2035);(34,C0008684|C0155937|C0348160|C0494704|C0494706,Unknown_CD86,1905);(34,C0008684|C0155937|C0348160|C0494704|C0494706,Monocyte_RILP,1893);(34,C0008684|C0155937|C0348160|C0494704|C0494706,Monocyte_SNX9,1875);(34,C0008684|C0155937|C0348160|C0494704|C0494706,Endometrial tissue_TVP23CP2,1868);(34,C0008684|C0155937|C0348160|C0494704|C0494706,Monocyte_EIF3G,1705);(34,C0008684|C0155937|C0348160|C0494704|C0494706,Endometrial tissue_SMC2,1699);(34,C0008684|C0155937|C0348160|C0494704|C0494706,Temporal_cortex_TRIM37,1687);(34,C0008684|C0155937|C0348160|C0494704|C0494706,Colorectum_VPS37B,1656);(34,C0008684|C0155937|C0348160|C0494704|C0494706,Brain white matter_STON1-GTF2A1L,1647);(34,C0008684|C0155937|C0348160|C0494704|C0494706,Endometrial tissue_TMEM159,1635);(34,C0008684|C0155937|C0348160|C0494704|C0494706,Endometrial tissue_GLB1,1550);(34,C0008684|C0155937|C0348160|C0494704|C0494706,Monocyte_PDHX ,1494);(34,C0008684|C0155937|C0348160|C0494704|C0494706,Bladder_THEM6,1490);(34,C0008684|C0155937|C0348160|C0494704|C0494706,Temporal_cortex_MYADML2,1475);(34,C0008684|C0155937|C0348160|C0494704|C0494706,Brain white matter_CLEC12A,1473);(34,C0008684|C0155937|C0348160|C0494704|C0494706,Macrophage_MEPE,1424);(34,C0008684|C0155937|C0348160|C0494704|C0494706,Endometrial tissue_PODN,1416);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Whole_blood_AKIRIN2,2911);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Monocyte_PER2,2255);(36,C 0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Colon_RAMP2,2035);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Unknown_CD86,1905);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Monocyte_RILP,1893);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Monocyte_SNX9,1875);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Endometrial tissue_TVP23CP2,1868);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Monocyte_EIF3G,1705);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Endometrial tissue_SMC2,1699);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Temporal_cortex_TRIM37,1687);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Colorectum_VPS37B,1656) ;(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Brain white matter_STON1-GTF2AIL,1647);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Endometrial tissue_TMEM159,1635);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Endometrial tissue_GLB1,1550);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Monocyte_PDHX,1494);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Bladder_THEM6,1490);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Temporal_cortex_MYADML2,1475);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Brain white matter_CLEC12A,1473);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Macrophage_MEPE, 1424);(36,C0008924|C0008925|C0158646|C0432090|C0432098|C0451896|C0451897|C0477977|C0478022|C0495504|C0495539|C0495540|C0495541|C0495542|C0495543|C0495545|C0869081|C2981150,Endometrial tissue_PODN,1416);(40,C0009806|C0014089|C0278008,Whole_blood_AKIRIN2,2911);(40,C0009806|C0014089|C0278008,Monocyte_PER2,2255);(40,C0009806|C0014089|C0278008,Colon_RAMP2,2035);(40,C0009806|C0014089|C0278008,Unknown_CD86,1905);(40,C0009806|C0014089|C0278008,Monocyte_RILP,1893);(40,C0009806|C0014089|C0278008,Monocyte_SNX9,1875);(40,C0009806|C0014089|C0278008,Endometrial tissue_TVP23CP2,1868);(40,C0009806|C0014089|C0278008,Monocyte_EIF3G,1705);(40,C0009806|C0014089|C0278008,Endometrial tissue_SMC2,1699);(40,C0009806|C0014089|C0278008,Temporal_cortex_TRIM37,1687);(40,C0009806|C0014089|C0278008,Colorectum_VPS37B,1656);(40,C0009806|C0014089|C0278008,Brain white matter_STON1-GTF2AIL,1647);(40,C0009806|C0014089|C0278008,Endometrial tissue_TMEM159,1635);(40,C0009806|C0014089|C0278008,Endometrial tissue_GLB1,1550);(40,C0009806|C0014089|C0278008,Monocyte_PDHX,1494);(40,C0009806|C0014089|C0278008,Bladder_THEM6,1490);(40,C0009806|C0014089|C0278008,Temporal_co rtex_MYADML2,1475);(40,C0009806|C0014089|C0278008,Brain white matter_CLEC12A,1473);(40,C0009806|C0014089|C0278008,Macrophage_MEPE,1424);(40,C0009806|C0014089|C0278008,Endometrial tissue_PODN,1416);(41,C0010054,Whole_blood_AKIRIN2,2911);(41,C0010054,Monocyte_PER2,2255);(41,C0010054,Colon_RAMP2,2035);(41,C0010054,Unknown_CD86,1905);(41,C0010054,Monocyte_RILP,1893);(41,C0010054,Monocyte_SNX9,1875);(41,C0010054,Endometrial tissue_TVP23CP2,1868);(41,C0010054,Monocyte_EIF3G,1705);(41,C0010054,Endometrial tissue_SMC2,1699);(41,C0010054,Temporal_cortex_TRIM37,1687);(41,C0010054,Colorectum_VPS37B,1656);(41,C0010054,Brain white matter_STON1-GTF2A1L,1647);(41,C0010054,Endometrial tissue_TMEM159,1635);(41,C0010054, Endometrial tissue_GLBl,1550);(41,C0010054,Monocyte_PDHX,1494);(41,C0010054,Bladder_THEM6,1490);(41,C0010054,Temporal_cortex_MYADML2,1475);(41,C0010054,Brain white matter_CLEC12A,1473);(41,C0010054,Macrophage_MEPE,1424);(41,C0010054,Endometrial tissue_PODN,1416);(42,C0010054|C0348590,Whole_blood_AKIRIN2,2911);(42,C0010054|C0348590,Monocyte_PER2,2255);(42,C00100541C0348590,Colon_RAMP2,2035);(42,C00100541C0348590,Unknown_CD86,1905);(42,C0010054|C0348590,Monocyte_RILP,1893);(42,C0010054|C0348590,Monocyte_SNX9,1875);(42,C0010054|C0348590,Endometrial tissue_TVP23CP2,1868);(42,C0010054|C0348590,Monocyte_EIF3G,1705);(42,C0010054|C0348590,Endometrial tissue_SMC2,1699);(42,C0010054|C0348590,Temporal_cortex_TRIM37,1687);(42,C0010054|C0348590,Colorectum_VPS37B,1656);(42,C0010054|C0348590,Brain white matter_STON1-GTF2A1L,1647);(42,C0010054|C0348590,Endometrial tissue_TMEM159,1635);(42,C0010054|C0348590,Endometrial tissue_GLB1,1550);(42,C00100541C0348590,Monocyte_PDHX,1494);(42,C00100541C0348590 ,Bladder_THEM6,1490);(42,C0010054|C0348590,Temporal_cortex_MYADML2,1475);(42,C0010054|C0348590,Brain white matter_CLEC12A,1473);(42,C0010054|C0348590,Macrophage_MEPE,1424);(42,C0010054|C0348590,Endometrial tissue_PODN,1416);(43,C0010346|C0156146|C0156147|C0348736|C0409695|C0451836,Whole_blood_AKIRIN2,2911);(43,C0010346|C0156146|C0156147|C0348736|C0409695|C0451836,Monocyte_PER2,2255);(43,C0010346|C0156146|C0156147|C0348736|C0409695|C0451836,Colon_RAMP2,2035);(43,C0010346|C0156146|C0156147|C0348736|C0409695|C0451836,Unknown_CD86,1905);(43,C0010346|C0156146|C0156147|C0348736|C0409695|C0451836,Monocyte_RILP,1893);(43,C0010346|C0156146|C0156147|C0348736|C0409695|C0451836,Monocyte_SNX9,1875);(43,C0010346|C0156146|C0156147|C0348736|C0409695|C0451836,Endometrial tissue_TVP23CP2,1868);(43,C0010346|C0156146|C0156147|C0348736|C0409695|C0451836,Monocyte_EIF3G,1705);(43,C0010346|C0156146|C0156147|C0348736|C0409695|C0451836,Endometrial tissue_SMC2,1699);(43,C0010346|C0156146|C0156147|C0348736|C0409695|C0451836,Temporal_cortex_TRIM37,1687);(43,C0010346|C0156146|C0156147|C0348736|C0409695|C0451836,Colorectum_VPS37B,1656);(43,C0010346|C0156146|C0156147|C0348736|C0409695|C045183 6,Brain white matter_STONI-GTF2A1L,1647);(43,C0010346|C0156146|C0156147|C0348736|C0409695|C0451836,Endometr ial tissue_TMEM159,1635);(43,C0010346|C0156146|C0156147|C0348736|C0409695|C0451836,E ndometrial tissue_GLB1,1550);(43,C0010346|C0156146|C0156147|C0348736|C0409695|C0451836,Monocyte_PDHX,1494);(43,C0010346|C0156146|C0156147|C0348736|C0409695|C0451836,Bladder. THEM6,1490);(43,C0010346|C0156146|C0156147|C0348736|C0409695|C0451836,Temporal_cortex_MYADML2,1475);(43,C0010346|C0156146|C0156147|C0348736|C0409695|C0451836,Brain white matter_CLEC12A,1473);(43,C00103461C01561461C01561471C03487361C04096951C0451836,Macrophage_MEPE,1424);(43,C0010346|C0156146|C0156147|C0348736|C0409695|C0451836,E ndometrial tissue_PODN,1416);(51,C0011849|C0011854|C0011860|C0271641|C0348447|C0851207,Whole_blood_AKIRIN2,2911);(51,C0011849|C0011854|C0011860|C0271641|C0348447|C0851207,Whole_blood_AKIRIN2,2911);(51,C0011849|C0011854|C0011860|C0271641|C0348447|C0851207,Monocyte_PER2,2255);(51,C0011849|C0011854|C0011860|C0271641|C0348447|C0851207,Monocyte_PER2,2255);(51,C0011849|C0011854|C0011860|C0271641|C0348447|C0851207,Colon_RAMP2,2035);(51,C0011849|C0011854|C0011860|C0271641|C0348447|C0851207,Colon_RAMP2,2035);(51,C0011849|C0011854|C0011860|C0271641|C0348447|C0851207,Unknown.CD86,1905);(51,C0011849|C0011854|C0011860|C0271641|C0348447|C0851207,Unknown_CD86,1905);(51,C0011849|C0011854|C0011860|C0271641|C0348447|C0851207,Monocyte_RILP,1893);(51,C0011849|C0011854|C0011860|C0271641|C0348447|C0851207,Monocyte_RILP,1893);(51,C0011849|C0011854|C0011860|C0271641|C0348447|C0851207,Monocyte_SNX9,1875);(51,C0011849|C0011854|C0011860|C0271641|C0348447|C0851207,Monocyte_SNX9,1875);(51,C0011849|C0011854|C0011860|C0271641|C0348447|C0851207,Endometrial tissue_TVP23CP2,1868);(51,C0011849|C0011854|C0011860|C0271641|C0348447|C0851207,E ndometrial tissue_TVP23CP2,1868);(51,C0011849|C0011854|C0011860|C0271641|C0348447|C0851207,M onocyte_EIF3G,1705);(51,C0011849|C0011854|C0011860|C0271641|C0348447|C0851207,Mon ocyte_EIF3G,1705);(51,C0011849|C0011854|C0011860|C0271641|C0348447|C0851207,Endom etrial tissue_SMC2,1699);(51,C0011849|C0011854|C0011860|C0271641|C0348447|C0851207,Endom etrial tissue_SMC2,1699);(51,C0011849|C0011854|C0011860|C0271641|C0348447|C0851207,Tempo ral_cortex_TRIM37,1687);(51,C0011849|C0011854|C0011860|C0271641|C0348447|C0851207, Temporal_cortex_TRIM37,1687);(56,C0013395|C0018834|C0232492,Whole_blood_AKIRIN2, 2911);(56,C0013395|C0018834|C0232492,Monocyte_PER2,2255);(56,C0013395|C0018834|C0 232492,Colon_RAMP2,2035);(56,C0013395|C0018834|C0232492,Unknown_CD86,1905);(56, C0013395|C0018834|C0232492,Monocyte_RILP,1893);(56,C0013395|C0018834|C0232492,Mo nocyte_SNX9,1875);(56,C0013395|C0018834|C0232492,Endometrial tissue_TVP23CP2,1868);(56,C0013395|C0018834|C0232492,Monocyte_EIF3G,1705);(56,C001 3395|C0018834|C0232492,Endometrial tissue_SMC2,1699);(56,C0013395|C0018834|C0232492,Temporal_cortex_TRIM37,1687);(56, C0013395|C0018834|C0232492,Colorectum_VPS37B,1656);(56,C0013395|C0018834|C023249 2,Brain white matter_STON1-GTF2A1L,1647);(56,C00133951C00188341C0232492,Endometrial tissue_TMEM159,1635);(56,C0013395|C0018834|C0232492,Endometrial tissue_GLB1,1550);(56,C0013395|C0018834|C0232492,Monocyte_PDHX,1494);(56,C0013395| C0018834|C0232492,Bladder_THEM6,1490);(56,C0013395|C0018834|C0232492,Temporal_co rtex_MYADML2,1475);(56,C0013395|C0018834|C0232492,Brain white matter_CLEC12A,1473);(56,C0013395|C0018834|C0232492,Macrophage_MEPE,1424);(56,C0 013395|C0018834|C0232492,Endometrial tissue_PODN,1416);(62,C0015674|C0024528|C0027804|C0152145|C0236795|C0236816|C0451 880|C0477817|C0546983|C1270972,Whole_blood_AKIRIN2,2911);(62,C0015674|C0024528|C 0027804|C0152145|C0236795|C0236816|C0451880|C0477817|C0546983|C1270972,Monocyte_ PER2,2255);(62,C0015674|C0024528|C0027804|C0152145|C0236795|C0236816|C0451880|C0 477817|C0546983|C1270972,Colon_RAMP2,2035);(62,C0015674|C0024528|C0027804|C01521 45|C0236795|C0236816|C0451880|C0477817|C0546983|C1270972,Unknown_CD86,1905);(62, C0015674|C0024528|C0027804|C0152145|C0236795|C0236816|C0451880|C0477817|C054698 3|C1270972,Monocyte_RILP,1893);(62,C0015674|C0024528|C0027804|C0152145|C0236795|C 0236816|C0451880|C0477817|C0546983|C1270972,Monocyte_SNX9,1875);(62,C0015674|C00 24528|C0027804|C015215|C0236795|C0236816|C0451880|C0477817|C0546983|C1270972,En dometrial tissue_TVP23CP2,1868);(62,C0015674|C0024528|C0027804|C0152145|C0236795|C0236816|C 0451880|C0477817|C0546983|C1270972,Monocyte_EIF3G,1705);(62,C0015674|C0024528|C00 27804|C0152145|C0236795|C0236816|C0451880|C0477817|C0546983|C1270972,Endometrial tissue_SMC2,1699);(62,C0015674|C0024528|C0027804|C0152145|C0236795|C0236816|C0451 880|C0477817|C0546983|C1270972,Temporal_cortex_TRIM37,1687);(62,C0015674|C0024528| C0027804|C0152145|C0236795|C0236816|C0451880|C0477817|C0546983|C1270972,Colorectu m_VPS37B,1656);(62,C0015674|C0024528|C0027804|C0152145|C0236795|C0236816|C04518 80|C0477817|C0546983|C1270972,Brain white matter_STON1-GTF2AIL,1647);(62,C0015674|C0024528|C0027804|C0152145|C0236795|C0236816|C045188 0|C0477817|C0546983|C1270972,Endometrial tissue_TMEM159,1635);(62,C0015674|C0024528|C0027804|C0152145|C0236795|C0236816|C 0451880|C0477817|C0546983|C1270972,Endometrial tissue_GLB1,1550);(62,C0015674|C0024528|C0027804|C0152145|C0236795|C0236816|C0451 880|C0477817|C0546983|C1270972,Monocyte_PDHX,1494);(62,C0015674|C0024528|C002780 4|C0152145|C0236795|C0236816|C0451880|C0477817|C0546983|C1270972,Bladder_THEM6, 1490);(62,C0015674|C0024528|C0027804|C0152145|C0236795|C0236816|C0451880|C0477817 |C0546983|C1270972,Temporal_cortex_MYADML2,1475);(62,C0015674|C0024528|C0027804| C0152145|C0236795|C0236816|C0451880|C0477817|C0546983|C1270972,Brain white matter_CLEC12A,1473);(62,C0015674|C0024528|C0027804|C0152145|C0236795|C0236816|C 0451880|C0477817|C0546983|C1270972,Macrophage_MEPE,1424);(62,C0015674|C0024528|C 0027804|C0152145|C0236795|C0236816|C0451880|C0477817|C0546983|C1270972,Endometri al tissue_PODN,1416);(64,C0017154,Whole_blood_AKIRIN2,2911);(64,C0017154,Whole_blood _AKIRIN2,2911);(64,C0017154,Whole_blood_AKIRIN2,2911);(64,C0017154,Monocyte_PER 2,2255);(64,C0017154,Monocyte_PER2,2255);(64,C0017154,Monocyte_PER2,2255);(64,C001 7154,Colon_RAMP2,2035);(64,C0017154,Colon_RAMP2,2035);(64,C0017154,Colon_RAMP2 ,2035);(64,C0017154,Unknown_CD86,1905);(64,C0017154,Unknown_CD86,1905);(64,C0017154,Unknown_CD86,1905);(64,C0017154,Monocyte_RILP,1893);(64,C0017154,Monocyte_RILP,1893);(64,C0017154,Monocyte_RILP,1893);(64,C0017154,Monocyte_SNX9,1875);(64,C0017154,Monocyte_SNX9,1875);(64,C0017154,Monocyte_SNX9,1875);(64,C0017154,Endometrial tissue_TVP23CP2,1868);(64,C0017154,Endometrial tissue_TVP23CP2,1868);(68,C0018681|C0149931|C0338480|C0338483|C0477373|C1735856,Whole_blood_AKIRIN2,2911);(68,C0018681|C0149931|C0338480|C0338483|C0477373|C1735856,Monocyte_PER2,2255);(68,C0018681|C0149931|C0338480|C0338483|C0477373|C1735856,Colon_RAMP2,2035);(68,C0018681|C0149931|C0338480|C0338483|C0477373|C1735856,Unknown_CD86,1905);(68,C0018681|C0149931|C0338480|C0338483|C0477373|C1735856,Monocyte_RILP,1893);(68,C0018681|C0149931|C0338480|C0338483|C0477373|C1735856,Monocyte_SNX9,1875);(68,C0018681|C0149931|C0338480|C0338483|C0477373|C1735856,Endometrial tissue_TVP23CP2,1868);(68,C0018681|C0149931|C0338480|C0338483|C0477373|C1735856,Monocyte_EIF3G,1705);(68,C0018681|C0149931|C0338480|C0338483|C0477373|C1735856,Endometrial tissue_SMC2,1699);(68,C0018681|C0149931|C0338480|C0338483|C0477373|C1735856,Temporal_cortex_TRIM37,1687);(68,C0018681|C0149931|C0338480|C0338483|C0477373|C1735856,Colorectum_VPS37B,1656);(68,C0018681|C0149931|C0338480|C0338483|C0477373|C1735856,Brain white matter_STONI-GTF2A1L,1647);(68,C0018681|C0149931|C0338480|C0338483|C0477373|C1735856,Endometrial tissue_TMEM159,1635);(68,C0018681|C0149931|C0338480|C0338483|C0477373|C1735856,Endometrial tissue_GLB1,1550);(68,C0018681|C0149931|C0338480|C0338483|C0477373|C1735856,Monocyte_PDHX,1494);(68,C0018681|C0149931|C0338480|C0338483|C0477373|C1735856,Bladder,THEM6,1490);(68,C0018681|C0149931|C0338480|C0338483|C0477373|C1735856,Temporal_cortex_MYADML2,1475);(68,C0018681|C0149931|C0338480|C0338483|C0477373|C1735856,Brain white matter_CLEC12A,1473);(68,C0018681|C0149931|C0338480|C0338483|C0477373|C1735856,Macrophage_MEPE,1424);(68,C0018681|C0149931|C0338480|C0338483|C0477373|C1735856,Endometrial tissue_PODN,1416);(73,C0019364|C0022568|C0022573|C0041322|C0155087|C0339239|C0348191|C0348526|C0348532|C0348533|C0348985|C0494081|C0494524,Whole_blood_AKIRIN2,2911);(73,C0019364|C0022568|C0022573|C0041322|C0155087|C0339239|C0348191|C0348526|C0348532|C0348533|C0348985|C0494081|C0494524,Monocyte_PER2,2255);(73,C0019364|C0022568|C0022573|C0041322|C0155087|C0339239|C0348191|C0348526|C0348532|C0348533|C0348985|C0494081|C0494524,Colon_RAMP2,2035);(73,C0019364|C0022568|C0022573|C0041322|C0155087|C0339239|C0348191|C0348526|C0348532|C0348533|C0348985|C0494081|C0494524,Unknown_CD86,1905);(73,C0019364|C0022568|C0022573|C0041322|C0155087|C0339239|C0348191|C0348526|C0348532|C0348533|C0348985|C0494081|C0494524,Monocyte_RILP,1893);(73,C0019364|C0022568|C0022573|C0041322|C0155087|C0339239|C0348191|C0348526|C0348532|C0348533|C0348985|C0494081|C0494524,Monocyte_SNX9,1875);(73,C0019364|C0022568|C0022573|C0041322|C0155087|C0339239|C0348191|C0348526|C0348532|C0348533|C0348985|C0494081|C0494524,Endometrial tissue_TVP23CP2,1868);(73,C0019364|C0022568|C0022573|C0041322|C0155087|C0339239|C0348191|C0348526|C0348532|C0348533|C0348985|C0494081|C0494524,Monocyte_EIF3G,1705);(73,C0019364|C0022568|C0022573|C0041322|C0155087|C0339239|C0348191|C0348526|C0348532|C0348533|C0348985|C0494081|C0494524,Endometrial tissue_SMC2,1699);(73,C0019364|C0022568|C0022573|C0041322|C0155087|C0339239|C0348191|C0348526|C0348532|C0348533|C0348985|C0494081|C0494524,Temporal_cortex_TRIM37,1687);(73,C0019364|C0022568|C0022573|C0041322|C0155087|C0339239|C0348191|C0348526|C0348532|C0348533|C0348985|C0494081|C0494524,Colorectum_VPS37B,1656);(73,C0019364|C0022568|C0022573|C0041322|C0155087|C0339239|C0348191|C0348526|C0348532|C0348533|C0348985|C0494081|C0494524,Brain white matter_STON1-GTF2A1L,1647);(73,C0019364|C0022568|C0022573|C0041322|C0155087|C0339239|C0348191|C0348526|C0348532|C0348533|C0348985|C0494081|C0494524,Endometrial tissue_TMEM159,1635);(73,C0019364|C0022568|C0022573|C0041322|C0155087|C0339239|C0348191|C0348526|C0348532|C0348533|C0348985|C0494081|C0494524,Endometrial tissue_GLB1,1550);(73,C0019364|C0022568|C0022573|C0041322|C0155087|C0339239|C0348191|C0348526|C0348532|C0348533|C0348985|C0494081|C0494524,Monocyte_PDHX,1494);(73,C0019364|C0022568|C0022573|C0041322|C0155087|C0339239|C0348191|C0348526|C0348532|C0348533|C0348985|C0494081|C0494524,Bladder_THEM6,1490);(73,C0019364|C0022568|C0022573|C0041322|C0155087|C0339239|C0348191|C0348526|C0348532|C0348533|C0348985|C0494081|C0494524,Temporal_cortex_MYADML2,1475);(73,C0019364|C0022568|C002257 3|C0041322|C0155087|C0339239|C0348191|C0348526|C0348532|C0348533|C0348985|C0494081|C0494524,Brain white matter_CLEC12A,1473);(73,C0019364|C0022568|C0022573|C0041322|C0155087|C0339239|C0348191|C0348526|C0348532|C0348533|C0348985|C0494081|C0494524,Macrophage_MEPE,1424);(73,C0019364|C0022568|C0022573|C0041322|C0155087|C0339239|C0348191|C0348526|C0348532|C0348533|C0348985|C0494081|C0494524,Endometrial tissue_PODN,1416);(74,C0019693,Whole_blood_AKIRIN2,2911);(74,C0019693,Whole_blood_AKIRIN2,2911);(74,C0019693,Monocyte_PER2,2255);(74,C0019693,Monocyte_PER2,2255);(74,C0019693,Colon_RAMP2,2035);(74,C0019693,Colon_RAMP2,2035);(74,C0019693,Unknown_CD86,1905);(74,C0019693,Unknown_CD86,1905);(74,C0019693,Monocyte_RILP,1893);(74,C0019693,Monocyte_RILP,1893);(74,C0019693,Monocyte_SNX9,1875);(74,C0019693,Monocyte_SNX9,1875);(74,C0019693,Endometrial tissue_TVP23CP2,1868);(74,C0019693,Endometrial tissue_TVP23CP2,1868);(74,C00119693,Monocyte_EIF3G,1705);(74,C0019693,Monocyte_EIF3G,1705);(74,C0019693,Endometrial tissue_SMC2,1699);(74,C0019693,Endometrial tissue_SMC2,1699);(74,C0019693,Temporal_cortex_TRIM37,1687);(74,C0019693,Temporal_cortex_TRIM37,1687);(75,C0020179|C0349080,Whole_blood_AKIRIN2,2911);(75,C0020179|C0349080,Monocyte_PER2,2255);(75,C0020179|C0349080,Colon_RAMP2,2035);(75,C0020179|C0349080,Unknown_CD86,1905);(75,C0020179|C0349080,Monocyte_RILP,1893);(75,C0020179|C0349080,Monocyte_SNX9,1875);(75,C0020179|C0349080,Endometrial tissue_TVP23CP2,1868);(75,C0020179|C0349080,Monocyte_EIF3G,1705);(75,C0020179|C0349080,Endometrial tissue_SMC2,1699);(75,C0020179|C0349080,Temporal_cortex_TRIM37,1687);(75,C0020179|C0349080,Colorectum_VPS37B,1656);(75,C0020179|C0349080,Brain white matter_STON1-GTF2A1L,1647);(75,C0020179|C0349080,Endometrial tissue_TMEM159,1635);(75,C0020179|C0349080,Endometrial tissue_GLB1,1550);(75,C0020179|C0349080,Monocyte_PDHX,1494);(75,C0020179|C0349080 ,Bladder_THEM6,1490);(75,C0020179|C0349080,Temporal_cortex_MYADML2,1475);(75,C0020179|C0349080,Brain white matter_CLEC12A,1473);(75,C0020179|C0349080,Macrophage_MEPE,1424);(75,C0020179|C0349080,Endometrial tissue_PODN,1416);(77,C0020517|C0034525|C0156153|C0156154|C0267166|C0348099|C0348101|C0348674|C0348676|C0348738|C0413022|C0494761|C0494762|C0494807|C0496106|C0546822,Whole_blood_AKIRIN2,2911);(77,C0020517|C0034525|C0156153|C0156154|C0267166|C0348099|C0348101|C0348674|C0348676|C0348738|C0413022|C0494761|C0494762|C0494807|C0496106|C0546822,Monocyte_PER2,2255);(77,C0020517|C0034525|C0156153|C0156154|C0267166|C0348099|C0348101|C0348674|C0348676|C0348738|C0413022|C0494761|C0494762|C0494807|C0496106|C0546822,Colon_RAMP2,2035);(77,C0020517|C0034525|C0156153|C0156154|C0267166|C0348099|C0348101|C0348674|C0348676|C0348738|C0413022|C0494761|C0494762|C0494807|C0496106|C0546822,Unknown_CD86,1905);(77,C0020517|C0034525|C0156153|C0156154|C0267166|C0348099|C0348101|C0348674|C0348676|C0348738|C0413022|C0494761|C0494762|C0494807|C0496106|C0546822,Monocyte_RILP,1893);(77,C0020517|C0034525|C0156153|C0156154|C0267166|C0348099|C0348101|C0348674|C0348676|C0348738|C0413022|C0494761|C0494762|C0494807|C0496106|C0546822,Monocyte_SNX9,1875);(77,C0020517|C0034525|C0156153|C0156154|C0267166|C0348099|C0348101|C0348674|C0348676|C0348738|C0413022|C0494761|C0494762|C0494807|C0496106|C0546822,Endometrial tissue_TVP23CP2,1868);(77,C0020517|C0034525|C0156153|C0156154|C0267166|C0348099|C0348101|C0348674|C0348676|C0348738|C0413022|C0494761|C0494762|C0494807|C0496106|C0546822,Monocyte_EIF3G,1705);(77,C0020517|C0034525|C0156153|C0156154|C0267166|C0348099|C0348101|C0348674|C0348676|C0348738|C0413022|C0494761|C0494762|C0494807|C04961061C0546822,Endometrial tissue_SMC2,1699);(77,C0020517|C0034525|C0156153|C0156154|C0267166|C0348099|C0348101|C0348674|C0348676|C0348738|C0413022|C0494761|C0494762|C0494807|C0496106|C0546822,Temporal_cortex_TRIM37,1687);(77,C0020517|C0034525|C0156153|C0156154|C0267166|C0348099|C0348101|C0348674|C0348676|C0348738|C0413022|C0494761|C0494762|C0494807|C0496106|C0546822,Colorectum_VPS37B,1656);(77,C0020517|C0034525|C0156153|C0156154|C0267166|C0348099|C0348101|C0348674|C0348676|C0348738|C0413022|C0494761|C0494762|C0494807|C0496106|C0546822,Brain white matter_STON1-GTF2A1L,1647);(77,C0020517|C0034525|C0156153|C0156154|C0267166|C0348099|C0348101|C0348674|C0348676|C0348738|C0413022|C0494761|C0494762|C0494807|C0496106|C0546822,Endometrial tissue_TMEM159,1635);(77,C0020517|C0034525|C0156153|C0156154|C0267166|C0348099|C0348101|C0348674|C0348676|C0348738|C0413022|C0494761|C0494762|C0494807|C0496106|C0546822,Endometrial tissue_GLB1,1550);(77,C0020517|C0034525|C0156153|C0156154|C0267166|C0348099|C0348101|C0348674|C0348676|C0348738|C0413022|C0494761|C0494762|C0494807|C0496106|C0546822,Monocyte_PDHX,1494);(77,C0020517|C0034525|C0156153|C0156154|C0267166|C0348099|C0348101|C0348674|C0348676|C0348738|C0413022|C0494761|C0494762|C0494807|C0496106|C0546822,Bladder_THEM6,1490);(77,C0020517|C0034525|C0156153|C0156154|C0267166|C0348099|C0348101|C0348674|C0348676|C0348738|C0413022|C0494761|C0494762|C0494807|C0496106|C0546822,Temporal_cortex_MYADML2,1475);(77,C0020517|C0034525|C0156153|C0156154|C0267166|C0348099|C0348101|C0348674|C0348676|C0348738|C0413022|C0494761|C0494762|C0494807|C0496106|C0546822,Brain white matter_CLEC12A,1473);(77,C0020517|C0034525|C0156153|C0156154|C0267166|C0348099|C0348101|C0348674|C0348676|C0348738|C0413022|C0494761|C0494762|C0494807|C0496106|C0546822,Macrophage_MEPE,1424);(77,C0020517|C0034525|C0156153|C0156154|C0267166|C0348099|C0348101|C0348674|C0348676|C0348738|C0413022|C0494761|C0494762|C0494807|C0496106|C0546822,Endometrial tissue_PODN,1416);(83,C0021364|C0405581|C0476525,Whole_blood_AKIRIN2,2911);(83,C0021364|C0405581|C0476525,Monocyte_PER2,2255);(83,C0021364|C0405581|C0476525,Colon_RAMP2,2035);(83,C0021364|C0405581|C0476525,Unknown_CD86,1905);(83,C0021364|C0405581|C0476525,Monocyte_RILP,1893);(83,C0021364|C0405581|C0476525,Monocyte_SNX9,1875);(83,C0021364|C0405581|C0476525,Endometrial tissue_TVP23CP2,1868);(83,C0021364|C0405581|C0476525,Monocyte_EIF3G,1705);(83,C0021364|C0405581|C0476525,Endometrial tissue_SMC2,1699);(83,C0021364|C0405581|C0476525,Temporal_cortex_TRIM37,1687);(83,C0021364|C0405581|C0476525,Colorectum_VPS37B,1656);(83,C0021364|C0405581|C0476525,Brain white matter_STON1-GTF2A1L,1647);(83,C0021364|C0405581|C0476525,Endometrial tissue_TMEM159,1635);(83,C0021364|C0405581|C0476525,Endometrial tissue_GLB1,1550);(83,C0021364|C0405581|C0476525,Monocyte_PDHX,1494);(83,C0021364|C0405581|C0476525,Bladder_THEM6,1490);(83,C0021364|C0405581|C0476525,Temporal_cortex_MYADML2,1475);(83,C0021364|C0405581|C0476525,Brain white matter_CLEC12A,1473);(83,C0021364|C0405581|C0476525,Macrophage_MEPE,1424);(83,C0021364|C0405581|C0476525,Endometrial tissue_PODN,1416);(86,C0022650,Whole_blood_AKIRIN2,2911);(86,C0022650,Whole_blood_AKIRIN2,2911);(86,C0022650,Monocyte_PER2,2255);(86,C0022650,Monocyte_PER2,2255);(86,C0022650,Colon_RAMP2,2035);(86,C0022650,Colon_RAMP2,2035);(86,C0022650,Unknown_CD86,1905);(86,C0022650,Unknown_CD86,1905);(86,C0022650,Monocyte_RILP,1893);(86,C0022650,Monocyte_RILP,1893);(86,C0022650,Monocyte_SNX9,1875);(86,C0022650,Monocyte_SNX9,1875);(86,C0022650,Endometrial tissue_TVP23CP2,1868);(86,C0022650,Endometrial tissue_TVP23CP2,1868);(86,C0022650,Monocyte_EIF3G,1705);(86,C0022650,Monocyte_EIF3G,1705);(86,C0022650,Endometrial tissue_SMC2,1699);(86,C0022650,Endometrial tissue_SMC2,1699);(86,C0022650,Temporal_cortex_TRIM37,1687);(86,C0022650,Temporal_cortex_TRIM37,1687);(87,C0022661|C0041349|C0268731|C0348642|C0477312|C0495063|C0848548,Whole_blood_AKIRIN2,2911);(87,C0022661|C0041349|C0268731|C0348642|C0477312|C0495063|C0848548,Monocyte_PER2,2255);(87,C0022661|C0041349|C0268731|C0348642|C0477312|C0495063|C0848548,Colon_RAMP2,2035);(87,C0022661|C0041349|C0268731|C0348642|C0477312|C0495063|C0848548,Unknown_CD86,1905);(87,C0022661|C0041349|C0268731|C0348642|C0477312|C0495063|C0848548,Monocyte_RILP,1893);(87,C0022661|C0041349|C0268731|C0348642|C0477312|C0495063|C0848548,Monocyte_SNX9,1875);(87,C0022661|C0041349|C0268731|C0348642|C0477312|C0495063|C0848548,Endometrial tissue_TVP23CP2,1868);(87,C0022661|C0041349|C0268731|C0348642|C0477312|C0495063|C0848548,Monocyte_EIF3G,1705);(87,C0022661|C0041349|C0268731|C0348642|C0477312|C0495063|C0848548,Endometrial tissue_SMC2,1699);(87,C0022661|C0041349|C0268731|C0348642|C0477312|C0495063|C0848548,Temporal_cortex_TRIM37,1687);(87,C0022661|C0041349|C0268731|C0348642|C0477312|C0495063|C0848548,Colorectum_VPS37B,1656);(87,C0022661|C0041349|C0268731|C0348642|C0477312|C0495063|C0848548,Brain white matter_STONI-GTF2A1L,1647);(87,C0022661|C0041349|C0268731|C0348642|C0477312|C0495063|C0848548,Endometrial tissue_TMEM159,1635);(87,C0022661|C0041349|C0268731|C0348642|C0477312|C0495063|C0848548,Endometrial tissue_GLBI,1550);(87,C0022661|C0041349|C0268731|C0348642|C0477312|C0495063|C0848548,Monocyte_PDHX,1494);(87,C0022661|C0041349|C0268731|C0348642|C0477312|C049506 3|C0848548,Bladder_THEM6,1490);(87,C0022661|C0041349|C0268731|C0348642|C0477312|C0495063|C0848548,Temporal_cortex_MYADML2,1475);(87,C0022661|C0041349|C0268731|C0348642|C0477312|C0495063|C0848548,Brain white matter_CLEC12A,1473);(87,C0022661|C0041349|C0268731|C0348642|C0477312|C0495063|C0848548,Macrophage_MEPE,1424);(87,C0022661|C0041349|C0268731|C0348642|C0477312|C0495063|C0848548,Endometrial tissue_PODN,1416);(92,C0025517|C0263660|C0348791,Whole_blood_AKIRIN2,2911);(92,C0025517|C0263660|C0348791,Monocyte_PER2,2255);(92,C0025517|C0263660|C0348791,Colon_RAMP2,2035);(92,C0025517|C0263660|C0348791,Unknown_CD86,1905);(92,C0025517|C0263660|C0348791,Monocyte_RILP,1893);(92,C0025517|C0263660|C0348791,Monocyte_SNX9,1875);(92,C0025517|C0263660|C0348791,Endometrial tissue_TVP23CP2,1868);(92,C0025517|C0263660|C0348791,Monocyte_EIF3G,1705);(92,C0025517|C0263660|C0348791,Endometrial tissue_SMC2,1699);(92,C0025517|C0263660|C0348791,Temporal_cortex_TRIM37,1687);(92,C0025517|C0263660|C0348791,Colorectum_VPS37B,1656);(92,C0025517|C0263660|C0348791,Brain white matter_STON1-GTF2A1L,1647);(92,C0025517|C0263660|C0348791,Endometrial tissue_TMEM159,1635);(92,C0025517|C0263660|C0348791,Endometrial tissue_GLB1,1550);(92,C0025517|C0263660|C0348791,Monocyte_PDHX,1494);(92,C0025517|C0263660|C0348791,Bladder_THEM6,1490);(92,C0025517|C0263660|C0348791,Temporal_cortex_MYADML2,1475);(92,C0025517|C0263660|C0348791,Brain white matter_CLEC12A,1473);(92,C0025517|C0263660|C0348791,Macrophage_MEPE,1424);(92,C0025517|C0263660|C0348791,Endometrial tissue_PODN,1416);(95,C0026896|C0154199|C0494501|C0495465,Whole_blood_AKIRIN2,2911);(95,C0026896|C0154199|C0494501|C0495465,Monocyte_PER2,2255);(95,C0026896|C0154199|C0494501|C0495465,Colon_RAMP2,2035);(95,C0026896|C0154199|C0494501|C0495465,Unknown_CD86,1905);(95,C0026896|C0154199|C0494501|C0495465,Monocyte_RILP,1893);(95,C0026896|C0154199|C0494501|C0495465,Monocyte_SNX9,1875);(95,C0026896|C0154199|C0494501|C0495465,Endometrial tissue_TVP23CP2,1868);(95,C0026896|C0154199|C0494501|C0495465,Monocyte_EIF3G,1705);(95,C0026896|C0154199|C0494501|C0495465,Endometrial tissue_SMC2,1699);(95,C0026896|C0154199|C0494501|C0495465,Temporal_cortex_TRIM37,1687);(95,C0026896|C0154199|C0494501|C0495465,Colorectum_VPS37B,1656);(95,C0026896|C0154199|C0494501|C0495465,Brain white matter_STON1-GTF2A1L,1647);(95,C0026896|C0154199|C0494501|C0495465,Endometrial tissue_TMEM159,1635);(95,C0026896|C0154199|C0494501|C0495465,Endometrial tissue_GLB1,1550);(95,C0026896|C0154199|C0494501|C0495465,Monocyte_PDHX,1494);(95,C0026896|C0154199|C0494501|C0495465,Bladder_THEM6,1490);(95,C0026896|C0154199|C0494501|C0495465,Temporal_cortex_MYADML2,1475);(95,C0026896|C0154199|C0494501|C0495465,Brain white matter_CLEC12A,1473);(95,C0026896|C0154199|C0494501|C0495465,Macrophage_MEPE,1424);(95,C0026896|C0154199|C0494501|C0495465,Endometrial tissue_PODN,1416);(97,C0027531|C0037929|C0411063|C0452049|C0452050|C0452051|C0478224|C0478257|C0694460|C1331979,Whole_blood_AKIRIN2,2911);(97,C0027531|C0037929|C0411063|C0452049|C0452050|C0452051|C0478224|C0478257|C0694460|C1331979,Monocyte_PER2,2255);(97,C0027531|C0037929|C0411063|C0452049|C0452050|C0452051|C0478224|C0478257|C0694460|C1331979,Colon_RAMP2,2035);(97,C0027531|C0037929|C0411063|C0452049|C0452050|C0452051|C0478224|C0478257|C0694460|C1331979,Unknown_CD86,1905);(97,C0027531|C0037929|C0411063|C0452049|C0452050|C0452051|C0478224|C0478257|C0694460|C1331979,Monocyte_RILP,1893);(97,C0027531|C0037929|C0411063|C0452049|C0452050|C0452051|C0478224|C0478257|C0694460|C1331979,Monocyte_SNX9,1875);(97,C0027531|C0037929|C0411063|C0452049|C0452050|C0452051|C0478224|C0478257|C0694460|C1331979,Endometrial tissue_TVP23CP2,1868);(97,C0027531|C0037929|C0411063|C0452049|C0452050|C0452051|C0478224|C0478257|C0694460|C1331979,Monocyte_EIF3G,1705);(97,C0027531|C0037929|C0411063|C0452049|C0452050|C0452051|C0478224|C0478257|C0694460|C1331979,Endometrial tissue_SMC2,1699);(97,C0027531|C0037929|C0411063|C0452049|C0452050|C0452051|C0478224|C0478257|C0694460|C1331979,Temporal_cortex_TRIM37,1687);(97,C0027531|C0037929|C0411063|C0452049|C0452050|C0452051|C0478224|C0478257|C0694460|C1331979,Colorectum_VPS37B,1656);(97,C0027531|C0037929|C0411063|C0452049|C0452050|C0452051|C0478224|C0478257|C0694460|C1331979,Brain white matter_STON1-GTF2AlL,1647);(97,C0027531|C0037929|C0411063|C0452049|C0452050|C0452051|C047822 4|C0478257|C0694460|C1331979,Endometrial tissue_TMEM159,1635);(97,C0027531|C0037929|C0411063|C0452049|C0452050|C0452051|C0478224|C0478257|C0694460|C1331979,Endometrial tissue_GLB1,1550);(97,C0027531|C0037929|C0411063|C0452049|C0452050|C0452051|C0478224|C0478257|C0694460|C1331979,Monocyte_PDHX,1494);(97,C0027531|C0037929|C0411063|C0452049|C0452050|C0452051|C0478224|C0478257|C0694460|C1331979,Bladder_THEM6,1490);(97,C0027531|C0037929|C0411063|C0452049|C0452050|C0452051|C0478224|C0478257|C0694460|C1331979,Temporal_cortex_MYADML2,1475);(97,C0027531|C0037929|C0411063|C0452049|C0452050|C0452051|C0478224|C0478257|C0694460|C1331979,Brain white matter_CLEC12A,1473);(97,C0027531|C0037929|C0411063|C0452049|C0452050|C0452051|C0478224|C0478257|C0694460|C1331979,Macrophage_MEPE,1424);(97,C0027531|C0037929|C0411063|C0452049|C0452050|C0452051|C0478224|C0478257|C0694460|C1331979,Endometrial tissue_PODN,1416);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Whole_blood_AKIRIN2,2911);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Monocyte_PER2,2255);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Colon_RAMP2,2035) ;(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Unknown_CD86,1905);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Monocyte_RILP,1893);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Monocyte_SNX9,1875);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Endometrial tissue_TVP23CP2,1868);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Monocyte_EIF3G,1705);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Endometrial tissue_SMC2,1699);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Temporal_cortex_TRIM37,1687);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Colorectum_VPS37B,1656);(100,C0029456|C0029458|C0029694|C0031707|C0042870|C0152256|C0158447|C0264115|C0451868|C0451869|C0451870|C0451871|C0451872|C0451873|C0451881|C045 1882|C0451883|C0451884|C0477673|C0477674|C0494808|C0494958|C0494964|C0494997|C0494998|C0495002|C0495020|C0495120|C0495123|C0521170|C0694460|C0694523,Brain white matter_STONI-GTF2A1L,1647);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Endometrial tissue_TMEM159,1635);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Endo metrial tissue_GLB1,1550);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Monocyte_PDHX,1494);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Bladder_THEM6,1490);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Temporal_cortex_MYADML2,1475);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Brain white matter_CLEC12A,1473);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Macrophage_MEPE,1424);(100,C0029456|C0029458|C0029694|C0031707|C0042870|C0152256|C0158447|C0264115|C0451868|C0451869|C0451870|C0451871|C0451872|C0451873|C0451881|C0451882|C0451883|C0451884|C0477673|C0477674|C0494808|C0494958|C0494964|C0494997|C0494998|C0495002|C0495020|C0495120|C0495123|C0521170|C0694460|C0694523,Endometrial tissue_PODN,1416);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Whole_blood_AKIRIN2,2911);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Monocyte_PER2,2255);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Colon_RAMP2,2035);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Unknown_CD86,1905);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Monocyte_RILP,1893);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Monocyte_SNX9,1875);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Endometrial tissue_TVP23CP2,1868);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Monocyte_EIF3G,1705);(101 ,C0029882|C0029888|C0152874|C0155415|C0155421|C0155440|C0155441|C0155460|C0161744|C0271431|C0271446|C0395849|C0477443|C0477444|C0477445|C0477446|C0477447|C04774 48|C0494087|C0694493|C1455742,Endometrial tissue_SMC2,1699);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Temporal_cortex_TRIM37,1687);( 101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Colorectum_VPS37B,1656);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C069449 3|C1455742,Brain white matter_STON1-GTF2A1L,1647);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Endometrial tissue_TMEM159,1635);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Endometrial tissue_GLB1,1550);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Monocyte_PDHX,1494);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Bladder_THEM6,1490);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Temporal_cortex_MYADML2,1475);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Brain white matter_CLEC12A,1473);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Macrophage_MEPE,1424);(101,C0029882|C0029888|C0152874|C0155415|C0155421|C0155440|C0155441|C0155460|C0161744|C0271431|C0271446|C0395849|C0477443|C0477444|C0477445|C0477446|C0477447|C0477448|C0494087|C0694493|C1455742,Endometrial tissue_PODN,1416);(103,C0030567|C1828079,Whole_blood_AKIRIN2,2911);(103,C0030567|C1828079,Monocyte_PER2,2255);(103,C0030567|C1828079,Colon_RAMP2,2035);(103,C0030567|C1828079,Unknown_CD86,1905);(103,C0030567|C1828079,Monocyte_RILP,1893);(103,C0030567|C1828079,Monocyte_SNX9,1875);(103,C0030567|C1828079,Endometrial tissue_TVP23CP2,1868);(103,C0030567|C1828079,Monocyte_EIF3G,1705);(103,C0030567|C1 828079,Endometrial tissue_SMC2,1699);(103,C0030567|C1828079,Temporal_cortex_TRIM37,1687);(103,C0030567|C1828079,Colorectum_VPS37B,1656);(103,C0030567|C1828079,Brain white matter_STON1-GTF2A1L,1647);(103,C0030567|C1828079,Endometrial tissue_TMEM159,1635);(103,C0030567|C1828079, Endometrial tissue_GLB1,1550);(103,C0030567|C1828079,Monocyte_PDHX,1494);(103,C0030567|C1828079,Bladder_THEM6,1490);(103,C0030567|C1828079,Temporal_cortex_MYADML2,1475);(103,C0030567|C1828079,Brain white matter_CLEC12A,1473);(103,C0030567|C1828079,Macrophage_MEPE,1424);(103,C0030567| C1828079,Endometrial tissue_PODN,1416);(104,C0030809,Whole_blood_AKIRIN2,2911);(104,C0030809,Monocyte_PER2,2255);(104,C0030809,Colon_RAMP2,2035);(104,C0030809,Unknown_CD86,1905);(104,C0030809,Monocyte_RILP,1893);(104,C0030809,Monocyte_SNX9,1875);(104,C0030809,Endometrial tissue_TVP23CP2,1868);(104,C0030809,Monocyte_EIF3G,1705);(104,C0030809, Endometrial tissue_SMC2,1699);(104,C0030809,Temporal_cortex_TRIM37,1687);(104,C0030809,Colorectum_VPS37B,1656);(104,C0030809,Brain white matter_STON1-GTF2A1L,1647);(104,C0030809,Endometrial tissue_TMEM159,1635);(104,C0030809,Endometrial tissue_GLBl,1550);(104,C0030809,Monocyte_PDHX,1494);(104,C0030809,Bladder_THEM6,1490);(104,C0030809,Temporal_cortex_MYADML2,1475);(104,C0030809,Brain white matter_CLEC12A,1473);(104,C0030809,Macrophage_MEPE,1424);(104,C0030809,Endometria l tissue_PODN,1416);(105,C0031090|C1314006,Whole_blood_AKIRIN2,2911);(105,C0031090|C1314006,Monocyte_PER2,2255);(105,CO031090|C1314006,Colon_RAMP2,2035);(105,C0031090|C1314006,Unknown_CD86,1905);(105,C0031090|C1314006,Monocyte_RILP,1893);(105,C0031090|C1314006,Monocyte_SNX9,1875);(105,C0031090|C1314006,Endometrial tissue_TVP23CP2,1868);(105,C0031090|C1314006,Monocyte_EIF3G,1705);(105,C0031090|C1 314006,Endometrial tissue_SMC2,1699);(105,C0031090|C1314006,Temporal_cortex_TRIM37,1687);(105,C003109 0|C1314006,Colorectum_VPS37B,1656);(105,C0031090|C1314006,Brain white matter_STON1-GTF2A1L,1647);(105,C0031090|C1314006,Endometrial tissue_TMEM159,1635);(105,C0031090|C1314006,Endometrial tissue_GLB1,1550);(105,C0031090|C1314006,Monocyte_PDHX,1494);(105,C0031090|C13140 06,Bladder_THEM6,1490);(105,C0031090|C1314006,Temporal_cortex_MYADML2,1475);(10 5,C0031090|C1314006,Brain white matter_CLEC12A,1473);(105,C0031090|C1314006,Macrophage_MEPE,1424);(105,C0031090| C1314006,Endometrial tissue_PODN,1416);(106,C0035435,Whole_blood_AKIRIN2,2911);(106,C0035435,Monocyte_ PER2,2255);(106,C0035435,Colon_RAMP2,2035);(106,C0035435,Unknown_CD86,1905);(106 ,C0035435,Monocyte _RILP,1893);(106,C0035435,Monocyte_SNX9,1875);(106,C0035435,End ometrial tissue_TVP23CP2,1868);(106,C0035435,Monocyte_EIF3G,1705);(106,C0035435, Endometrial tissue_SMC2,1699);(106,C0035435,Temporal_cortex_TRIM37,1687);(106,C0035435,Colorectu m_VPS37B,1656);(106,C0035435,Brain white matter_STON1-GTF2A1L,1647);(106,C0035435,Endometrial tissue_TMEM159,1635);(106,C0035435,Endometrial tissue_GLBl,1550);(106,C0035435,Monocyte_PDHX,1494);(106,C0035435,Bladder_THEM6, 1490);(106,C0035435,Temporal_cortex_MYADML2,1475);(106,C0035435,Brain white matter_CLEC12A,1473);(106,C0035435,Macrophage_MEPE,1424);(106,C0035435,Endometria 1 tissue_PODN,1416);(109,C0035854|C0477490|C0477510,Whole_blood_AKIRIN2,2911);(109, C0035854|C0477490|C0477510,Monocyte_PER2,2255);(109,C0035854|C0477490|C0477510,C olon_RAMP2,2035);(109,C0035854|C0477490|C0477510,Unknown_CD86,1905);(109,C00358 54|C0477490|C0477510,Monocyte_RILP,1893);(109,C0035854|C0477490|C0477510,Monocyte _SNX9,1875);(109,C0035854|C0477490|C0477510,Endometrial tissue_TVP23CP2,1868);(109,C0035854|C0477490|C0477510,Monocyte_EIF3G,1705);(109,C0 035854|C0477490|C0477510,Endometrial tissue_SMC2,1699);(109,C0035854|C0477490|C0477510,Temporal_cortex_TRIM37,1687);(10 9,C0035854|C0477490|C0477510,Colorectum_VPS37B,1656);(109,C0035854|C0477490|C0477 510,Brain white matter_STONl-GTF2A1L,1647);(109,C0035854|C0477490|C0477510,Endometrial tissue_TMEM159,1635);(109,C0035854|C0477490|C0477510,Endometrial tissue_GLB1,1550);(109,C0035854|C0477490|C0477510,Monocyte_PDHX,1494);(109,C00358 54|C0477490|C0477510,Bladder_THEM6,1490);(109,C0035854|C0477490|C0477510,Temporal _cortex_MYADML2,1475);(109,C0035854|C0477490|C0477510,Brain white matter_CLEC12A,1473);(109,C0035854|C0477490|C0477510,Macrophage_MEPE,1424);(109, C0035854|C0477490|C0477510,Endometrial tissue_PODN,1416);(114,C0036690|C0152486|C0152946|C0152964|C0152965|C0152966|C015 2967|C0269294|C0269936|C0275685|C0276029|C0276063|C0343453|C0348131|C0348133|C03 48152|C0348970|C0349003|C0349004|C0349005|C0349006|C0349008|C0349009|C0392110|C0 452115|C0452197|C0452198|C0452199|C0477713|C0477736|C0477851|C0477919|C0477920|C 0477924|C0494048|C0494082|C0495304|C0495408|C0496128|C0496134|C0600327|C0868755| C0870076|C3665339,Whole_blood_AKIRIN2,2911);(114,C0036690|C0152486|C0152946|C015 2964|C0152965|C0152966|C0152967|C0269294|C0269936|C0275685|C0276029|C0276063|C03 43453|C0348131|C0348133|C0348152|C0348970|C0349003|C0349004|C0349005|C0349006|C0 349008|C0349009|C0392110|C0452115|C0452197|C0452198|C0452199|C0477713|C0477736|C 0477851|C0477919|C0477920|C0477924|C0494048|C0494082|C0495304|C0495408|C0496128| C0496134|C0600327|C0868755|C0870076|C3665339,Monocyte_PER2,2255);(114,C0036690|C 0152486|C0152946|C0152964|C0152965|C0152966|C0152967|C0269294|C0269936|C0275685| C0276029|C0276063|C0343453|C0348131|C0348133|C0348152|C0348970|C0349003|C034900 4|C0349005|C0349006|C0349008|C0349009|C0392110|C0452115|C0452197|C0452198|C04521 99|C0477713|C0477736|C0477851|C0477919|C0477920|C0477924|C0494048|C0494082|C0495 304|C0495408|C0496128|C0496134|C0600327|C0868755|C0870076|C3665339,Colon_RAMP2, 2035);(114,C0036690|C0152486|C0152946|C0152964|C0152965|C0152966|C0152967|C026929 4|C0269936|C0275685|C0276029|C0276063|C0343453|C0348131|C0348133|C0348152|C03489 70|C0349003|C0349004|C0349005|C0349006|C0349008|C0349009|C0392110|C0452115|C0452 197|C0452198|C0452199|C0477713|C0477736|C0477851|C0477919|C0477920|C0477924|C049 4048|C0494082|C0495304|C0495408|C0496128|C0496134|C0600327|C0868755|C0870076|C36 65339,Unknown_CD86,1905);(114,C0036690|C0152486|C0152946|C0152964|C0152965|C015 2966|C0152967|C0269294|C0269936|C0275685|C0276029|C0276063|C0343453|C0348131|C03 48133|C0348152|C0348970|C0349003|C0349004|C0349005|C0349006|C0349008|C0349009|C0 392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Monocyte_RILP,1893);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Monocyte_SNX9,1875);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Endometrial tissue_TVP23CP2,1868);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Monocyte_EIF3G,1705);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Endometrial tissue_SMC2,1699);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Temporal_cortex_TRIM37,1687);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Colorectum_VPS37B,1656);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Brain white matter_STON1-GTF2A1L,1647);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Endometrial tissue_TMEM159,1635);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Endometrial tissue_GLB1,1550);(114,C0036690|C0152486|C0152946|C0152964|C0152965|C0152966|C0152967|C0269294|C0269936|C0275685|C0276029|C0276063|C0343453|C0348131|C0348133|C0348152|C0348970|C0349003|C0349004|C0349005|C0349006|C0349008|C0349009|C0392110|C0452115|C0452197|C0452198|C0452199|C0477713|C0477736|C0477851|C0477919|C0477920|C0477924|C0494048|C0494082|C0495304|C0495408|C0496128|C0496134|C0600327|C0868755|C0870076|C3665339,Monocyte_PDHX,1494);(114,C0036690|C0152486|C0152946|C0152964|

C0152965|C0152966|C0152967|C0269294|C0269936|C0275685|C0276029|C0276063|C034345 3|C0348131|C0348133|C0348152|C0348970|C0349003|C0349004|C0349005|C0349006|C03490 08|C0349009|C0392110|C0452115|C0452197|C0452198|C0452199|C0477713|C0477736|C0477 851|C0477919|C0477920|C0477924|C0494048|C0494082|C0495304|C0495408|C0496128|C049 6134|C0600327|C0868755|C0870076|C3665339,Bladder_THEM6,1490);(114,C0036690|C0152 486|C0152946|C0152964|C0152965|C0152966|C0152967|C0269294|C0269936|C0275685|C027 6029|C0276063|C0343453|C0348131|C0348133|C0348152|C0348970|C0349003|C0349004|C03 49005|C0349006|C0349008|C0349009|C0392110|C0452115|C0452197|C0452198|C0452199|C0 477713|C0477736|C0477851|C0477919|C0477920|C0477924|C0494048|C0494082|C0495304|C 0495408|C0496128|C0496134|C0600327|C0868755|C0870076|C3665339,Temporal_cortex_MY ADML2,1475);(114,C0036690|C0152486|C0152946|C0152964|C0152965|C0152966|C0152967| C0269294|C0269936|C0275685|C0276029|C0276063|C0343453|C0348131|C0348133|C034815 2|C0348970|C0349003|C0349004|C0349005|C0349006|C0349008|C0349009|C0392110|C04521 15|C0452197|C0452198|C0452199|C0477713|C0477736|C0477851|C0477919|C0477920|C0477 924|C0494048|C0494082|C0495304|C0495408|C0496128|C0496134|C0600327|C0868755|C087 0076|C3665339,Brain                                                              white matter_CLEC12A,1473);(114,C0036690|C0152486|C0152946|C0152964|C0152965|C0152966| C0152967|C0269294|C0269936|C0275685|C0276029|C0276063|C0343453|C0348131|C034813 3|C0348152|C0348970|C0349003|C0349004|C0349005|C0349006|C0349008|C0349009|C03921 10|C0452115|C0452197|C0452198|C0452199|C0477713|C0477736|C0477851|C0477919|C0477 920|C0477924|C0494048|C0494082|C0495304|C0495408|C0496128|C0496134|C0600327|C086 8755|C0870076|C3665339,Macrophage_MEPE,1424);(114,C0036690|C0152486|C0152946|C01 52964|C0152965|C0152966|C0152967|C0269294|C0269936|C0275685|C0276029|C0276063|C0 343453|C0348131|C0348133|C0348152|C0348970|C0349003|C0349004|C0349005|C0349006|C 0349008|C0349009|C0392110|C0452115|C0452197|C0452198|C0452199|C0477713|C0477736| C0477851|C0477919|C0477920|C0477924|C0494048|C0494082|C0495304|C0495408|C049612 8|C0496134|C0600327|C0868755|C0870076|C3665339,Endometrial tissue_PODN,1416);(115,C0038013|C0348540|C0348715|C0409675|C0477606,Whole_blood_ AKIRIN2,2911);(115,C0038013|C0348540|C0348715|C0409675|C0477606,Monocyte_PER2,22 55);(115,C0038013|C0348540|C0348715|C0409675|C0477606,Colon_RAMP2,2035);(115,C003 8013|C0348540|C0348715|C0409675|C0477606,Unknown_CD86,1905);(115,C0038013|C0348 540|C0348715|C0409675|C0477606,Monocyte_RILP,1893);(115,C0038013|C0348540|C034871 5|C0409675|C0477606,Monocyte_SNX9,1875);(115,C0038013|C0348540|C0348715|C0409675| C0477606,Endometrial              tissue_TVP23CP2,1868);(115,C0038013|C0348540|C0348715|C0409675|C0477606,Monocyte_ EIF3G,1705);(115,C0038013|C0348540|C0348715|C0409675|C0477606,Endometrial tissue_SMC2,1699);(115,C0038013|C0348540|C0348715|C0409675|C0477606,Temporal_corte x_TRIM37,1687);(115,C0038013|C0348540|C0348715|C0409675|C0477606,Colorectum_VPS3 7B,1656);(115,C0038013|C0348540|C0348715|C0409675|C0477606,Brain           white          matter_STONI- GTF2A1L,1647);(115,C0038013|C0348540|C0348715|C0409675|C0477606,Endometrial tissue_TMEM159,1635);(115,C0038013|C0348540|C0348715|C0409675|C0477606,Endometria 1 tissue_GLB1,1550);(115,C0038013|C0348540|C0348715|C0409675|C0477606,Monocyte_PDH X,1494);(115,C0038013|C0348540|C0348715|C0409675|C0477606,Bladder_THEM6,1490);(11 5,C0038013|C0348540|C0348715|C0409675|C0477606,Temporal_cortex_MYADML2,1475);(1 15,C0038013|C0348540|C0348715|C0409675|C0477606,Brain                            white matter_CLEC12A,1473);(115,C0038013|C0348540|C0348715|C0409675|C0477606,Macrophag e_MEPE,1424);(115,C0038013|C0348540|C0348715|C0409675|C0477606,Endometrial tissue_PODN,1416);(118,C0040156|C0270222|C0477553|C0494276|C0494505,Whole_blood_ AKIRIN2,2911);(118,C0040156|C0270222|C0477553|C0494276|C0494505,Monocyte_PER2,22 55);(118,C0040156|C0270222|C0477553|C0494276|C0494505,Colon_RAMP2,2035);(118,C004 0156|C0270222|C0477553|C0494276|C0494505,Unknown_CD86,1905);(118,C0040156|C0270 222|C0477553|C0494276|C0494505,Monocyte_RILP,1893);(118,C0040156|C0270222|C047755 3|C0494276|C0494505,Monocyte_SNX9,1875);(118,C0040156|C0270222|C0477553|C04942761  C0494505,Endometrial              tissue_TVP23CP2,1868);(118,C0040156|C0270222|C0477553|C0494276|C0494505,Monocyte_ EIF3G,1705);(118,C0040156|C0270222|C0477553|C0494276|C0494505,Endometrial tissue_SMC2,1699);(118,C0040156|C0270222|C0477553|C0494276|C0494505,Temporal_corte x_TRIM37,1687);(118,C0040156|C0270222|C0477553|C0494276|C0494505,Colorectum_VPS3 7B,1656);(118,C0040156|C0270222|C0477553|C0494276|C0494505,Brain           white          matter_STONI- GTF2A1L,1647);(118,C0040156|C0270222|C0477553|C0494276|C0494505,Endometrial tissue_TMEM159,1635);(118,C0040156|C0270222|C0477553|C0494276|C0494505,Endometria tissue_GLB1,1550);(118,C0040156|C0270222|C0477553|C0494276|C0494505,Monocyte_PDH X,1494);(118,C0040156|C0270222|C0477553|C0494276|C0494505,Bladder_THEM6,1490);(11 8,C0040156|C0270222|C0477553|C0494276|C0494505,Temporal_cortex_MYADML2,1475);(1 18,C0040156|C0270222|C0477553|C0494276|C0494505,Brain white matter_CLEC12A,1473);(118,C0040156|C0270222|C0477553|C0494276|C0494505,Macrophag e_MEPE,1424);(118,C00401561C02702221C04775531C04942761C0494505,Endometrial tissue_PODN,1416);(119,C0040592|C0153107|C0153108|C0348303,Whole_blood_AKIRIN2,2 911);(119,C0040592|C0153107|C0153108|C0348303,Monocyte_PER2,2255);(119,C0040592|C 0153107|C0153108|C0348303,Colon_RAMP2,2035);(119,C0040592|C0153107|C0153108|C034 8303,Unknown_CD86,1905);(119,C0040592|C0153107|C0153108|C0348303,Monocyte_RILP, 1893);(119,C0040592|C0153107|C0153108|C0348303,Monocyte_SNX9,1875);(119,C0040592| C0153107|C0153108|C0348303,Endometrial tissue_TVP23CP2,1868);(119,C0040592|C0153107|C0153108|C0348303,Monocyte_EIF3G,170 5);(119,C0040592|C0153107 |C0153108|C0348303,Endometrial tissue_SMC2,1699);(119,C0040592|C0153107|C0153108|C0348303,Temporal_cortex_TRIM37 ,1687);(119,C00405 92|C0153107|C0153108|C0348303,Colorectum_VPS37B,1656);(119,C0040 592|C0153107|C0153108|C0348303,Brain white matter_STONI- GTF2A1L,1647);(119,C0040592|C0153107|C0153108|C0348303,Endometrial tissue_TMEM159,1635);(119,C0040592|C0153107|C0153108|C0348303,Endometrial tissue_GLB1,1550);(119,C0040592|C0153107|C0153108|C0348303,Monocyte_PDHX,1494);(1 19,C00405921C01531071C01531081C0348303,Bladder_THEM6,1490);(119,C0040592|C015310 7|C0153108|C0348303,Temporal_cortex_MYADML2,1475);(119,C0040592|C0153107|C01531 08|C0348303,Brain white matter_CLEC12A,1473);(119,C0040592|C0153107|C0153108|C0348303,Macrophage_MEPE,1 424);(119,C0040592|C0153107|C0153108|C0348303,Endometrial tissue_PODN,1416);(121,C0041333|C0242172|C0348147|C0348149|C0348905|C0451784|C045 1785|C0452162|C0452163|C0494056,Whole_blood_AKIRIN2,2911);(121,C0041333|C0242172| C0348147|C0348149|C0348905|C0451784|C0451785|C0452162|C0452163|C0494056,Monocyt e_PER2,2255);(121,C0041333|C0242172|C0348147|C0348149|C0348905|C0451784|C0451785| C0452162|C0452163|C0494056,Colon_RAMP2,2035);(121,C0041333|C0242172|C0348147|C0 348149|C0348905|C0451784|C0451785|C0452162|C0452163|C0494056,Unknown_CD86,1905) ;(121,C0041333|C0 242172|C0348147|C0348149|C0348905|C0451784|C0451785|C0452162|C04 52163|C0494056,Monocyte_RILP,1893);(121,C0041333|C0242172|C0348147|C0348149|C0348 905|C0451784|C0451785|C0452162|C0452163|C0494056,Monocyte_SNX9,1875);(121,C00413 33|C0242172|C0348147|C0348149|C0348905|C0451784|C0451785|C0452162|C0452163|C0494 056,Endometrial tissue_TVP23CP2,1868);(121,C0041333|C0242172|C0348147|C0348149|C0348905|C0451784| C0451785|C0452162|C0452163|C0494056,Monocyte_EIF3G,1705);(121,C0041333|C0242172| C0348147|C0348149|C0348905|C0451784|C0451785|C0452162|C0452163|C0494056,Endometr ial tissue_SMC2,1699);(121,C0041333|C0242172|C0348147|C0348149|C0348905|C0451784|C045 1785|C0452162|C0452163|C0494056,Temporal_cortex_TRIM37,1687);(121,C0041333|C02421 72|C0348147|C0348149|C0348905|C0451784|C0451785|C0452162|C0452163|C0494056,Colore ctum_VPS37B,1656);(121,C0041333|C0242172|C0348147|C0348149|C0348905|C0451784|C04 51785|C0452162|C0452163|C0494056,Brain white matter_STONI- GTF2A1L,1647);(121,C0041333|C0242172|C0348147|C0348149|C0348905|C0451784|C04517 85|C0452162|C0452163|C0494056,Endometrial tissue_TMEM159,1635);(121,C0041333|C0242172|C0348147|C0348149|C0348905|C0451784| C0451785|C0452162|C0452163|C0494056,Endometrial tissue_GLB1,1550);(121,C0041333|C0242172|C0348147|C0348149|C0348905|C0451784|C045 1785|C0452162|C0452163|C0494056,Monocyte_PDHX,1494);(121,C0041333|C0242172|C0348 147|C0348149|C0348905|C0451784|C0451785|C0452162|C0452163|C0494056,Bladder_THEM 6,1490);(121,C0041333|C0242172|C0348147|C0348149|C0348905|C0451784|C0451785|C0452 162|C0452163|C0494056,Temporal_cortex_MYADML2,1475);(121,C0041333|C0242172|C034 8147|C0348149|C0348905|C0451784|C0451785|C0452162|C0452163|C0494056,Brain white matter_CLEC12A,1473);(121,C0041333|C0242172|C0348147|C0348149|C0348905|C0451784| C0451785|C0452162|C0452163|C0494056,Macrophage_MEPE,1424);(121,C0041333|C024217 2|C0348147|C0348149|C0348905|C0451784|C0451785|C0452162|C0452163|C0494056,Endom etrial tissue_PODN,1416);(123,C0042900|C0348510,Whole_blood_AKIRIN2,2911);(123,C0042900| C0348510,Monocyte_PER2,2255);(123,C0042900|C0348510,Colon_RAMP2,2035);(123,C0042 900|C0348510,Unknown_CD86,1905);(123,C0042900|C0348510,Monocyte_RILP,1893);(123,C0042900|C0348510,Monocyte_SNX9,1875);(123,C0042900|C0348510,Endometrial tissue_TVP23CP2,1868);(123,C0042900|C0348510,Monocyte_EIF3G,1705);(123,C0042900|C0348510,Endometrial tissue_SMC2,1699);(123,C0042900|C0348510,Temporal_cortex_TRIM37,1687);(123,C0042900|C0348510,Colorectum_VPS37B,1656);(123,C0042900|C0348510,Brain white matter_STON1-GTF2A1L,1647);(123,C0042900|C0348510,Endometrial tissue_TMEM159,1635);(123,C0042900|C0348510,Endometrial tissue_GLB1,1550);(123,C0042900|C0348510,Monocyte_PDHX,1494);(123,C0042900|C0348510,Bladder_THEM6,1490);(123,C0042900|C0348510,Temporal_cortex_MYADML2,1475);(123,C0042900|C0348510,Brain white matter_CLEC12A,1473);(123,C0042900|C0348510,Macrophage_MEPE,1424);(123,C0042900|C0348510,Endometrial tissue_PODN,1416);(126,C0085207|C0158915|C0477897|C1455664,Whole_blood_AKIRIN2,2911);(126,C0085207|C0158915|C0477897|C1455664,Whole_blood_AKIRIN2,2911);(126,C0085207|C0158915|C0477897|C1455664,Monocyte_PER2,2255);(126,C0085207|C0158915|C0477897|C1455664,Monocyte_PER2,2255);(126,C0085207|C0158915|C0477897|C1455664,Colon_RAMP2,2035);(126,C0085207|C0158915|C0477897|C1455664,Colon_RAMP2,2035);(126,C0085207|C0158915|C0477897|C1455664,Unknown_CD86,1905);(126,C0085207|C0158915|C0477897|C1455664,Unknown_CD86,1905);(126,C0085207|C0158915|C0477897|C1455664,Monocyte_RILP,1893);(126,C0085207|C0158915|C0477897|C1455664,Monocyte_RILP,1893);(126,C0085207|C0158915|C0477897|C1455664,Monocyte_SNX9,1875);(126,C0085207|C0158915|C0477897|C1455664,Monocyte_SNX9,1875);(126,C0085207|C0158915|C0477897|C1455664,Endometrial tissue_TVP23CP2,1868);(126,C0085207|C0158915|C0477897|C1455664,Endometrial tissue_TVP23CP2,1868);(126,C0085207|C0158915|C0477897|C1455664,Monocyte_EIF3G,1705);(126,C0085207|C0158915|C0477897|C1455664,Monocyte_EIF3G,1705);(126,C0085207|C0158915|C0477897|C1455664,Endometrial tissue_SMC2,1699);(126,C0085207|C0158915|C0477897|C1455664,Endometrial tissue_SMC2,1699);(126,C0085207|C0158915|C0477897|C1455664,Temporal_cortex_TRIM37 ,1687);(126,C0085207|C0158915|C0477897|C1455664,Temporal_cortex_TRIM37,1687);(132,C0155709,Whole_blood_AKIRIN2,2911);(132,C0155709,Whole_blood_AKIRIN2,2911);(132,C0155709,Whole_blood_AKIRIN2,2911);(132,C0155709,Whole_blood_AKIRIN2,2911);(132,C0155709,Monocyte_PER2,2255);(132,C0155709,Monocyte_PER2,2255);(132,C0155709,Monocyte_PER2,2255);(132,C0155709,Monocyte_PER2,2255);(132,C0155709,Colon_RAMP2,2035) ;(132,C0155709,Colon_RAMP2,2035);(132,C0155709,Colon_RAMP2,2035);(132,C0155709,Colon_RAMP2,2035);(132,C0155709,Unknown_CD86,1905);(132,C0155709,Unknown_CD86,I905);(132,C0155709,Unknown_CD86,1905);(132,C0155709,Unknown_CD86,1905);(132,C0155709,Monocyte_RILP,1893);(132,C0155709,Monocyte_RILP,1893);(132,C0155709,Monocyte_RILP,1893);(132,C0155709,Monocyte_RILP,1893);(133,C0156258|C0178288|C0451641,Whole_blood_AKIRIN2,2911);(133,C0156258|C0178288|C0451641,Monocyte_PER2,2255);(133,C0156258|C0178288|C0451641,Colon_RAMP2,2035);(133,C0156258|C0178288|C0451641,Unknown_CD86,1905);(133,C0156258|C0178288|C0451641,Monocyte_RILP,1893);(133,C0156258|C0178288|C0451641,Monocyte_SNX9,1875);(133,C0156258|C0178288|C0451641,Endometrial tissue_TVP23CP2,1868);(133,C0156258|C0178288|C0451641,Monocyte_EIF3G,1705);(133,C0156258|C0178288|C0451641,Endometrial tissue_SMC2,1699);(133,C0156258|C0178288|C0451641,Temporal_cortex_TRIM37,1687);(133,C0156258|C0178288|C0451641,Colorectum_VPS37B,1656);(133,C0156258|C0178288|C0451641,Brain white matter_STONl-GTF2A1L,1647);(133,C0156258|C0178288|C0451641,Endometrial tissue_TMEM159,1635);(133,C0156258|C0178288|C0451641,Endometrial tissue_GLB1,1550);(133,C0156258|C0178288|C0451641,Monocyte_PDHX,1494);(133,C0156258|C0178288|C0451641,Bladder_THEM6,1490);(133,C0156258|C0178288|C0451641,Temporal_cortex_MYADML2,1475);(133,C0156258|C0178288|C0451641,Brain white matter_CLEC12A,1473);(133,C0156258|C0178288|C0451641,Macrophage_MEPE,1424);(133,C0156258|C0178288|C0451641,Endometrial tissue_PODN,1416);(135,C0178283,Whole_blood_AKIRIN2,1018);(135,C0178283,Monocyte_EIF3G,818);(135,C0178283,Monocyte_PER2,817);(135,C0178283,Bladder_TBC1D27,782);(135,C0178283,Breast_PARP9,781);(135,C0178283,Whole_blood_AMNI,721);(135,C0178283,Skin_DPP3,712);(135,C0178283,Whole_blood_CRISP3,700);(135,C0178283,Colorectum_USP2,678);(135,C0178283,Skin_GUSBPI,678);(135,C0178283,Whole_blood_ANXA3,673);(135,C0178283,Whole_blood_CLEC4D,670);(135,C0178283,Monocyte_ANXAII,654);(135,C0178283,Whole_blood_S100A12,654);(135,C0178283,Bladder_MRPL55,650);(135,C0178283,Monocyte _PIBFl,650);(135,C0178283,Colorectum_ADCY10,649);(135,C0178283,Adipose tissue_CASS4,649);(135,C0178283,Whole_blood_HNRNPDL,648);(135,C0178283,Colorectum _LINC01207,639);(137,C0238990,Whole_blood_AKIRIN2,2911);(137,C0238990,Monocyte_P ER2,2255);(137,C0238990,Colon_RAMP2,2035);(137,C0238990,Unknown_CD86,1905);(137, C0238990,Monocyte_RILP,1893);(137,C0238990,Monocyte_SNX9,1875);(137,C0238990,End ometrial tissue_TVP23CP2,1868);(137,C0238990,Monocyte_EIF3G,1705);(137,C0238990, Endometrial tissue_SMC2,1699);(137,C0238990,Temporal_cortex_TRIM37,1687);(137,C0238990,Colorectu m_VPS37B,1656);(137,C0238990,Brain white matter_STON1-GTF2A1L,1647);(137,C0238990,Endometrial tissue_TMEM159,1635);(137,C0238990,Endometrial tissue_GLB1,1550);(137,C0238990,Monocyte_PDHX,1494);(137,C0238990,Bladder_THEM6, 1490);(137,C0238990,Temporal_cortex_MYADML2,1475);(137,C0238990,Brain white matter_CLEC12A,1473);(137,C0238990,Macrophage_MEPE,1424);(137,C0238990,Endometria 1 tissue_PODN,1416);(143,C0276609|C0276610|C0348199|C0400913|C0400918|C0451704|C045 1705|C0494092|C0494093,Whole_blood_AKIRIN2,2911);(143,C0276609|C0276610|C0348199| C0400913|C0400918|C0451704|C0451705|C0494092|C0494093,Monocyte_PER2,2255);(143,C 0276609|C0276610|C0348199|C0400913|C0400918|C0451704|C0451705|C0494092|C0494093, Colon_RAMP2,2035);(143,C0276609|C0276610|C0348199|C0400913|C0400918|C0451704|C0 451705|C0494092|C0494093,Unknown_CD86,1905);(143,C0276609|C0276610|C0348199|C04 00913|C0400918|C0451704|C0451705|C0494092|C0494093,Monocyte_RILP,1893);(143,C0276 609|C0276610|C0348199|C0400913|C0400918|C0451704|C0451705|C0494092|C0494093,Mon ocyte_SNX9,1875);(143,C0276609|C0276610|C0348199|C0400913|C0400918|C0451704|C045 1705|C0494092|C0494093,Endometrial tissue_TVP23CP2,1868);(143,C0276609|C0276610|C0348199|C0400913|C0400918|C0451704| C0451705|C0494092|C0494093,Monocyte_EIF3G,1705);(143,C0276609|C0276610|C0348199| C0400913|C0400918|C0451704|C0451705|C0494092|C0494093,Endometrial tissue_SMC2,1699);(143,C0276609|C0276610|C0348199|C0400913|C0400918|C0451704|C045 1705|C0494092|C0494093,Temporal_cortex_TRIM37,1687);(143,C0276609|C0276610|C03481 99|C0400913|C0400918|C0451704|C0451705|C0494092|C0494093,Colorectum_VPS37B,1656) ;(143,C0276609|C0 276610|C0348199|C0400913|C0400918|C0451704|C0451705|C0494092|C04 94093,Brain white matter_STON1- GTF2A1L,1647);(143,C0276609|C0276610|C0348199|C0400913|C0400918|C0451704|C04517 05|C0494092|C0494093,Endometrial tissue_TMEM159,1635);(143,C0276609|C0276610|C0348199|C0400913|C0400918|C0451704| C0451705|C0494092|C0494093,Endometrial tissue- GLB1,1550);(143,C0276609|C0276610|C0348199|C0400913|C0400918|C0451704|C045 1705|C0494092|C0494093,Monocyte_PDHX,1494);(143,C0276609|C0276610|C0348199|C0400 913|C0400918|C0451704|C0451705|C0494092|C0494093,Bladder_THEM6,1490);(143,C02766 09|C0276610|C0348199|C0400913|C0400918|C0451704|C0451705|C0494092|C0494093,Temp oral_cortex_MYADML2,1475);(143,C0276609|C0276610|C0348199|C0400913|C0400918|C04 51704|C0451705|C0494092|C0494093,Brain white matter_CLEC12A,1473);(143,C0276609|C0276610|C0348199|C0400913|C0400918|C0451704| C0451705|C0494092|C0494093,Macrophage_MEPE,1424);(143,C0276609|C0276610|C034819 9|C0400913|C0400918|C0451704|C0451705|C0494092|C0494093,Endometrial tissue PODN,1416);(151,C0348694,Whole blood AKIRIN2,2911);(151,C0348694,Monocyte_ PER2,2255);(151,C0348694,Colon_RAMP2,2035);(151,C0348694,Unknown_CD86,1905);(151 ,C0348694,Monocyte _RILP,1893);(151,C0348694,Monocyte_SNX9,1875);(151,C0348694,End ometrial tissue_TVP23CP2,1868);(151,C0348694,Monocyte_EIF3G,1705);(151,C0348694,Endometrial tissue_SMC2,1699);(151,C0348694,Temporal_cortex_TRIM37,1687);(151,C0348694,Colorectu m_VPS37B,1656);(151,C0348694,Brain white matter_STON1-GTF2A1L,1647);(151,C0348694,Endometrial tissue_TMEM159,1635);(151,C0348694,Endometrial tissue_GLB1,1550);(151,C0348694,Monocyte_PDHX,1494);(151,C0348694,Bladder_THEM6, 1490);(151,C0348694,Temporal_cortex_MYADML2,1475);(151,C0348694,Brain white matter_CLEC12A,1473);(151,C0348694,Macrophage_MEPE,1424);(151,C0348694,Endometria 1 tissue_PODN,1416);(153,C0348723|C0451753|C0477411|C0478155|C0494697|C1527336,Whol e_blood_AKIRIN2,2911);(153,C0348723|C0451753|C0477411|C0478155|C0494697|C1527336, Whole_blood_AKIRIN2,2911);(153,C0348723|C0451753|C0477411|C0478155|C0494697|C152 7336,Monocyte_PER2,2255);(153,C0348723|C0451753|C0477411|C0478155|C0494697|C1527 336,Monocyte_PER2,2255);(153,C0348723|C0451753|C0477411|C0478155|C0494697|C15273 36,Colon_RAMP2,2035);(153,C0348723|C0451753|C0477411|C0478155|C0494697|C1527336, Colon_RAMP2,2035);(153,C0348723|C0451753|C0477411|C0478155|C0494697|C1527336,Un known_CD86,1905);(153,C0348723|C0451753|C0477411|C0478155|C0494697|C1527336,Unk nown_CD86,1905);(153,C0348723|C0451753|C0477411|C0478155|C0494697|C1527336,Mono cyte_RILP,1893);(153,C0348723|C0451753|C0477411|C0478155|C0494697|C1527336,Monocy te_RILP,1893);(153,C0348723|C0451753|C0477411|C0478155|C0494697|C1527336,Monocyte _SNX9,1875);(153,C0348723|C0451753|C0477411|C0478155|C0494697|C1527336,Monocyte_ SNX9,1875);(153,C0348723|C0451753|C0477411|C0478155|C0494697|C1527336,Endometrial tissue_TVP23CP2,1868);(153,C0348723|C0451753|C0477411|C0478155|C0494697|C1527336, Endometrial tissue_TVP23CP2,1868);(153,C0348723|C0451753|C0477411|C0478155|C0494697|C1527336, Monocyte_EIF3G,1705);(153,C0348723|C0451753|C0477411|C0478155|C0494697|C1527336, Monocyte_EIF3G,1705);(153,C0348723|C0451753|C0477741|C0478155|C0494697|C1527336, Endometrial tissue_SMC2,1699);(153,C0348723|C0451753|C0477411|C0478155|C0494697|C1527336,Endo metrial tissue_SMC2,1699);(153,C0348723|C0451753|C0477411|C0478155|C0494697|C1527336,Temp oral_cortex_TRIM37,1687);(153,C0348723|C0451753|C0477411|C0478155|C0494697|C15273 36,Temporal_cortex_TRIM37,1687);(156,C0349349|C0579986,Whole_blood_AKIRIN2,2911); (156,C0349349|C0579986,Whole_blood_AKIRIN2,2911);(156,C0349349|C0579986,Whole_bl ood_AKIRIN2,2911);(156,C0349349|C0579986,Monocyte_PER2,2255);(156,C0349349|C0579 986,Monocyte_PER2,2255);(156,C0349349|C0579986,Monocyte_PER2,2255);(156,C0349349| C0579986,Colon_RAMP2,2035);(156,C0349349|C0579986,Colon_RAMP2,2035);(156,C03493 49|C0579986,Colon_RAMP2,2035);(156,C0349349|C0579986,Unknown_CD86,1905);(156,C0 349349|C0579986,Unknown_CD86,1905);(156,C0349349|C0579986,Unknown_CD86,1905);(1 56,C0349349|C0579986,Monocyte_RILP,1893);(156,C0349349|C0579986,Monocyte_RILP,189 3);(156,C0349349|C0579986,Monocyte_RILP,1893);(156,C0349349|C0579986,Monocyte_SN X9,1875);(156,C0349349|C0579986,Monocyte_SNX9,1875);(156,C0349349|C0579986,Monoc yte_SNX9,1875);(156,C0349349|C0579986,Endometrial tissue_TVP23CP2,1868);(156,C0349349|C0579986,Endometrial tissue_TVP23CP2,1868);(157,C0400914|C0524910,Whole_blood_AKIRIN2,2911);(157,C0400 914|C0524910,Monocyte_PER2,2255);(157,C0400914|C0524910,Colon_RAMP2,2035);(157,C 0400914|C0524910,Unknown_CD86,1905);(157,C0400914|C0524910,Monocyte_RILP,1893);( 157,C0400914|C052 4910,Monocyte_SNX9,1875);(157,C0400914|C0524910,Endometrial tissue_TVP23CP2,1868);(157,C0400914|C0524910,Monocyte_EIF3G,1705);(157,C0400914|C0 524910,Endometrial tissue_SMC2,1699);(157,C0400914|C0524910,Temporal_cortex_TRIM37,1687);(157,C040091 4|C0524910,Colorectum_VPS37B,1656);(157,C0400914|C0524910,Brain white matter_STON1- GTF2A1L,1647);(157,C0400914|C0524910,Endometrial tissue_TMEM159,1635);(157,C0400914|C0524910, Endometrial tissue_GLB1,1550);(157,C0400914|C0524910,Monocyte_PDHX,1494);(157,C0400914|C05249 10,Bladder_THEM6,1490);(157,C0400914|C0524910,Temporal_cortex_MYADML2,1475);(15 7,C0400914|C0524910,Brain white matter_CLEC12A,1473);(157,C0400914|C0524910,Macrophage_MEPE,1424);(157,C0400914| C0524910,Endometrial tissue_PODN,1416);(158,C0404572,Whole_blood_AKIRIN2,2911);(158,C0404572,Monocyte_ PER2,2255);(158,C0404572,Colon_RAMP2,2035);(158,C0404572,Unknown_CD86,1905);(158 ,C0404572,Monocyte _RILP,1893);(158,C0404572,Monocyte_SNX9,1875);(158,C0404572,End ometrial tissue_TVP23CP2,1868);(158,C0404572,Monocyte_EIF3G,1705);(158,C0404572,Endometrial tissue_SMC2,1699);(158,C0404572,Temporal_cortex_TRIM37,1687);(158,C0404572,Colorectu m_VPS37B,1656);(158,C0404572,Brain white matter_STON1-GTF2A1L,1647);(158,C0404572,Endometrial tissue_TMEM159,1635);(158,C0404572,Endometrial tissue_GLB1,1550);(158,C0404572,Monocyte_PDHX,1494);(158,C0404572,Bladder_THEM6, 1490);(158,C0404572,Temporal_cortex_MYADML2,1475);(158,C0404572,Brain white matter_CLEC12A,1473);(158,C0404572,Macrophage_MEPE,1424);(158,C0404572,Endometria l tissue_PODN,1416);(159,C0433856|C0452047,Whole_blood_AKIRIN2,2911);(159,C0433856| C0452047,Monocyte_PER2,2255);(159,C0433856|C0452047,Colon_RAMP2,2035);(159,C0433 856|C0452047,Unknown_CD86,1905);(159,C0433856|C0452047,Monocyte_RILP,1893);(159, C0433856|C0452047,Monocyte_SNX9,1875);(159,C0433856|C0452047,Endometrial tissue_TVP23CP2,1868);(159,C0433856|C0452047,Monocyte_EIF3G,1705);(159,C0433856|C0 452047,Endometrial tissue_SMC2,1699);(159,C0433856|C0452047,Temporal_cortex_TRIM37,1687);(159,C043385 6|C0452047,Colorectum_VPS37B,1656);(159,C0433856|C0452047,Brain white matter_STON1- GTF2A1L,1647);(159,C0433856|C0452047,Endometrial tissue_TMEM159,1635);(159,C0433856|C0452047,Endometrial tissue_GLB1,1550);(159,C0433856|C0452047,Monocyte_PDHX,1494);(159,C0433856|C04520 47,Bladder_THEM6,1490);(159,C0433856|C0452047,Temporal_cortex_MYADML2,1475);(15 9,C0433856|C0452047,Brain white matter_CLEC12A,1473);(159,C0433856|C0452047,Macrophage_MEPE,1424);(159,C0433856| C0452047,Endometrial tissue_PODN,1416);(160,C0451707|C0728889,Whole_blood_AKIRIN2,2911);(160,C0451707| C0728889,Monocyte_PER2,2255);(160,C0451707|C0728889,Colon_RAMP2,2035);(160,C0451 707|C0728889,Unknown_CD86,1905);(160,C0451707|C0728889,Monocyte_RILP,1893);(160, C0451707|C0728889,Monocyte_SNX9,1875);(160,C0451707|C0728889,Endometrial tissue_TVP23CP2,1868);(160,C0451707|C0728889,Monocyte_EIF3G,1705);(160,C0451707|C0 728889,Endometrial tissue_SMC2,1699);(160,C0451707|C0728889,Temporal_cortex_TRIM37,1687);(160,C045170 7|C0728889,Colorectum_VPS37B,1656);(160,C0451707|C0728889,Brain white matter_STON1-GTF2A1L,1647);(160,C0451707|C0728889,Endometrial tissue_TMEM159,1635);(160,C0451707|C0728889,Endometrial tissue_GLB1,1550);(160,C0451707|C0728889,Monocyte_PDHX,1494);(160,C0451707|C07288 89,Bladder_THEM6,1490);(160,C0451707|C0728889,Temporal_cortex_MYADML2,1475);(16 0,C0451707|C0728889,Brain white matter_CLEC12A,1473);(160,C0451707|C0728889,Macrophage_MEPE,1424);(160,C0451707| C0728889,Endometrial tissue_PODN,1416);(161,C0451708|C0451710|C0451711|C0451712|C0494794,Whole_blood_ AKIRIN2,2911);(161,C0451708|C0451710|C0451711|C0451712|C0494794,Monocyte_PER2,22 55);(161,C0451708|C0451710|C0451711|C0451712|C0494794,Colon_RAMP2,2035);(161,C045 1708|C0451710|C0451711|C0451712|C0494794,Unknown_CD86,1905);(161,C0451708|C0451 710|C0451711|C0451712|C0494794,Monocyte_RILP,1893);(161,C0451708|C0451710|C045171 1|C0451712|C0494794,Monocyte_SNX9,1875);(161,C0451708|C0451710|C0451711|C0451712| C0494794,Endometrial tissue_TVP23CP2,1868);(161,C0451708|C0451710|C0451711|C0451712|C0494794,Monocyte_ EIF3G,1705);(161,C0451708|C0451710|C0451711|C0451712|C0494794,Endometrial tissue_SMC2,1699);(161,C0451708|C0451710|C0451711|C0451712|C0494794,Temporal_corte x_TRIM37,1687);(161,C0451708|C0451710|C0451711|C0451712|C0494794,Colorectum_VPS3 7B,1656);(161,C0451708|C0451710|C0451711|C0451712|C0494794,Brain white matter_STON1-GTF2A1L,1647);(161,C0451708|C0451710|C0451711|C0451712|C0494794,Endometrial tissue_TMEM159,1635);(161,C0451708|C0451710|C0451711|C0451712|C0494794,Endometrial tissue_GLB1,1550);(161,C0451708|C0451710|C0451711|C0451712|C0494794,Monocyte_PDH X,1494);(161,C0451708|C0451710|C0451711|C0451712|C0494794,Bladder_THEM6,1490);(16 1,C0451708|C0451710|C0451711|C0451712|C0494794,Temporal_cortex_MYADML2,1475);(1 61,C0451708|C0451710|C0451711|C0451712|C0494794,Brain white matter_CLEC12A,1473);(161,C0451708|C0451710|C0451711|C0451712|C0494794,Macrophag e_MEPE,1424);(161,C0451708|C0451710|C0451711|C0451712|C0494794,Endometrial tissue_PODN,1416);(165,C0477491,Whole_blood_AKIRIN2,2911);(165,C0477491,Whole_blo od_AKIRIN2,2911);(165,C0477491,Monocyte_PER2,2255);(165,C0477491,Monocyte_PER2,2 255);(165,C0477491,Colon_RAMP2,2035);(165,C0477491,Colon_RAMP2,2035);(165,C04774 91,Unknown_CD86,1905);(165,C0477491,Unknown_CD86,1905);(165,C0477491,Monocyte_R ILP,1893);(165,C0477491,Monocyte_RILP,1893);(165,C0477491,Monocyte_SNX9,1875);(165, C0477491,Monocyte_SNX9,1875);(165,C0477491,Endometrial tissue_TVP23CP2,1868);(165,C0477491,Endometrial tissue_TVP23CP2,1868);(165,C0477491,Monocyte_EIF3G,1705);(165,C0477491,Monocyte_EI F3G,1705);(165,C0477491,Endometrial tissue_SMC2,1699);(165,C0477491,Endometrial tissue_SMC2,1699);(165,C0477491,Temporal_cortex_TRIM37,1687);(165,C0477491,Temporal _cortex_TRIM37,1687);(166,C0494026,Whole_blood_AKIRIN2,2911);(166,C0494026,Monoc yte_PER2,2255);(166,C0494026,Colon_RAMP2,2035);(166,C0494026,Unknown_CD86,1905); (166,C0494026,Monocyte_RILP,1893);(166,C0494026,Monocyte_SNX9,1875);(166,C0494026 ,Endometrial tissue_TVP23CP2,1868);(166,C0494026,Monocyte_EIF3G,1705);(166,C0494026, Endometrial tissue_SMC2,1699);(166,C0494026,Temporal_cortex_TRIM37,1687);(166,C0494026,Colorectu m_VPS37B,1656);(166,C0494026,Brain white matter_STON1-GTF2A1L,1647);(166,C0494026,Endometrial tissue_TMEM159,1635);(166,C0494026,Endometrial tissue_GLB1,1550);(166,C0494026,Monocyte_PDHX,1494);(166,C0494026,Bladder_THEM6, 1490);(166,C0494026,Temporal_cortex_MYADML2,1475);(166,C0494026,Brain white matter_CLEC12A,1473);(166,C0494026,Macrophage_MEPE,1424);(166,C0494026,Endometria 1 tissue_PODN,1416);(169,C0494650|C0494660,Whole_blood_AKIRIN2,2911);(169,C0494650| C0494660,Monocyte_PER2,2255);(169,C0494650|C0494660,Colon_RAMP2,2035);(169,C0494 650|C0494660,Unknown_CD86,1905);(169,C0494650|C0494660,Monocyte_RILP,1893);(169, C0494650|C0494660,Monocyte_SNX9,1875);(169,C0494650|C0494660,Endometrial tissue_TVP23CP2,1868);(169,C0494650|C0494660,Monocyte_EIF3G,1705);(169,C0494650|C0 494660,Endometrial tissue_SMC2,1699);(169,C0494650|C0494660,Temporal_cortex_TRIM37,1687);(169,C049465 0|C0494660,Colorectum_VPS37B,1656);(169,C0494650|C0494660,Brain white matter_STON1-GTF2A1L,1647);(169,C0494650|C0494660,Endometrial tissue_TMEM159,1635);(169,C0494650|C0494660,Endometrial tissue_GLB1,1550);(169,C0494650|C0494660,Monocyte_PDHX,1494);(169,C0494650|C0494660,Bladder_THEM6,1490);(169,C0494650|C0494660,Temporal_cortex_MYADML2,1475);(169,C0494650|C0494660,Brain white matter_CLEC12A,1473);(169,C0494650|C0494660,Macrophage_MEPE,1424);(169,C0494650| C0494660,Endometrial tissue_PODN,1416);(171,C0494697|C0494887|C0495047|C3495801,Whole_blood_AKIRIN2,2911);(171,C0494697|C0494887|C0495047|C3495801,Whole_blood_AKIRIN2,2911);(171,C0494697|C0494887|C0495047|C3495801,Monocyte_PER2,2255);(171,C0494697|C0494887|C0495047|C3495801,Monocyte_PER2,2255);(171,C0494697|C0494887|C0495047|C3495801,Colon_RAMP2,2035);(171,C0494697|C0494887|C0495047|C3495801,Colon_RAMP2,2035);(171,C0494697|C0494887|C0495047|C3495801,Unknown_CD86,1905);(171,C0494697|C0494887|C0495047|C3495801,Unknown_CD86,1905);(171,C0494697|C0494887|C0495047|C3495801,Monocyte_RILP,1893);(171,C0494697|C0494887|C0495047|C3495801,Monocyte_RILP,1893);(171,C0494697|C0494887|C0495047|C3495801,Monocyte_SNX9,1875);(171,C0494697|C0494887|C0495047|C3495801,Monocyte_SNX9,1875);(171,C0494697|C0494887|C0495047|C3495801,Endometrial tissue_TVP23CP2,1868);(171,C0494697|C0494887|C0495047|C3495801,Endometrial tissue_TVP23CP2,1868);(171,C0494697|C0494887|C0495047|C3495801,Monocyte_EIF3G,1705);(171,C0494697|C0494887|C0495047|C3495801,Monocyte_EIF3G,1705);(171,C0494697|C0494887|C0495047|C3495801,Endometrial tissue_SMC2,1699);(171,C0494697|C0494887|C0495047|C3495801,Endometrial tissue_SMC2,1699);(171,C0494697|C0494887|C0495047|C3495801,Temporal_cortex_TRIM37 ,1687);(171,C0494697|C0494887|C0495047|C3495801,Temporal_cortex_TRIM37,1687);(172,C0494783,Whole_blood_AKIRIN2,2911);(172,C0494783,Monocyte_PER2,2255);(172,C0494783,Colon_RAMP2,2035);(172,C0494783,Unknown_CD86,1905);(172,C0494783,Monocyte_RILP,1893);(172,C0494783,Monocyte_SNX9,1875);(172,C0494783,Endometrial tissue_TVP23CP2,1868);(172,C0494783,Monocyte_EIF3G,1705);(172,C0494783,Endometrial tissue_SMC2,1699);(172,C0494783,Temporal_cortex_TRIM37,1687);(172,C0494783,Colorectum_VPS37B,1656);(172,C0494783,Brain white matter_STON1-GTF2A1L,1647);(172,C0494783,Endometrial tissue_TMEM159,1635);(172,C0494783,Endometrial tissue_GLB1,1550);(172,C0494783,Monocyte_PDHX,1494);(172,C0494783,Bladder_THEM6,1490);(172,C0494783,Temporal_cortex_MYADML2,1475);(172,C0494783,Brain white matter_CLEC12A,1473);(172,C0494783,Macrophage_MEPE,1424);(172,C0494783,Endometrial tissue_PODN,1416);(173,C0494831,Whole_blood_AKIRIN2,2911);(173,C0494831,Whole_blood AKIRIN2,2911);(173,C0494831,Whole blood AKIRIN2,2911);(173,C0494831,Monocyte_PER2,2255);(173,C0494831,Monocyte_PER2,2255);(173,C0494831,Monocyte_PER2,2255);(173,C0494831,Colon_RAMP2,2035);(173,C0494831,Colon_RAMP2,2035);(173,C0494831,Colon_RAMP2,2035);(173,C0494831,Unknown_CD86,1905);(173,C0494831,Unknown_CD86,1905 );(173,C0494831,Unknown_CD86,1905);(173,C0494831,Monocyte_RILP,1893);(173,C0494831,Monocyte_RILP,1893);(173,C0494831,Monocyte_RILP,1893);(173,C0494831,Monocyte_SNX9,1875);(173,C0494831,Monocyte_SNX9,1875);(173,C0494831,Monocyte_SNX9,1875);(173 ,C0494831,Endometrial tissue_TVP23CP2,1868);(173,C0494831,Endometrial tissue_TVP23CP2,1868);(175,C0496594,Whole_blood_AKIRIN2,2911);(175,C0496594,Monocyte_PER2,2255);(175,C0496594,Colon_RAMP2,2035);(175,C0496594,Unknown_CD86,1905);(175,C0496594,Monocyte_RILP,1893);(175,C0496594,Monocyte_SNX9,1875);(175,C0496594 ,Endometrial tissue_TVP23CP2,1868);(175,C0496594,Monocyte_EIF3G,1705);(175,C0496594,Endometrial tissue_SMC2,1699);(175,C0496594,Temporal_cortex_TRIM37,1687);(175,C0496594,Colorectum_VPS37B,1656);(175,C0496594,Brain white matter_STON1-GTF2A1L, 1647);(175,C0496594,Endometrial tissue_TMEM159,1635);(175,C0496594, Endometrial tissue_GLB1,1550);(175,C0496594,Monocyte_PDHX,1494);(175,C0496594,Bladder_THEM6, 1490);(175,C0496594,Temporal_cortex_MYADML2,1475);(175,C0496594,Brain white matter_CLEC12A,1473);(175,C0496594,Macrophage_MEPE,1424);(175,C0496594,Endometrial tissue_PODN,1416);(176,C0496856,Whole_blood_AKIRIN2,2911);(176,C0496856,Monocyte_PER2,2255);(176,C0496856,Colon_RAMP2,2035);(176,C0496856,Unknown_CD86,1905);(176 ,C0496856,Monocyte_RILP,1893);(176,C0496856,Monocyte_SNX9,1875);(176,C0496856,Endometrial tissue_TVP23CP2,1868);(176,C0496856,Monocyte_EIF3G,1705);(176,C0496856, Endometrial tissue_SMC2,1699);(176,C0496856,Temporal_cortex_TRIM37,1687);(176,C0496856,Colorectum_VPS37B,1656);(176,C0496856,Brain white matter_STON1-GTF2A1L,1647);(176,C0496856,Endometrial tissue_TMEM159,1635);(176,C0496856,Endometrial tissue_GLB1,1550);(176,C0496856,Monocyte_PDHX,1494);(176,C0496856,Bladder_THEM6, 1490);(176,C0496856,Temporal_cortex_MYADML2,1475);(176,C0496856,Brain white matter_CLEC12A,1473);(176,C0496856,Macrophage_MEPE,1424);(176,C0496856,Endometria tissue_PODN,1416);(179,C1265999,Whole_blood_AKIRIN2,2911);(179,C1265999,Whole_blo od_AKIRIN2,2911);(179,C1265999,Monocyte_PER2,2255);(179,C1265999,Monocyte_PER2,2 255);(179,C1265999,Colon_RAMP2,2035);(179,C1265999,Colon_RAMP2,2035);(179,C12659 99,Unknown_CD86,1905);(179,C1265999,Unknown_CD86,1905);(179,C1265999,Monocyte_R ILP,1893);(179,C1265999,Monocyte_RILP,1893);(179,C1265999,Monocyte_SNX9,1875);(179, C1265999,Monocyte_SNX9,1875);(179,C1265999,Endometrial tissue_TVP23CP2,1868);(179,C1265999,Endometrial tissue_TVP23CP2,1868);(179,C1265999,Monocyte_EIF3G,1705);(179,C1265999,Monocyte_EI F3G,1705);(179,C1265999,Endometrial tissue_SMC2,1699);(179,C1265999,Endometrial tissue_SMC2,1699);(179,C1265999,Temporal_cortex_TRIM37,1687);(179,C1265999,Temporal _cortex_TRIM37,1687);(186,barrett oesophagus, Whole_blood_AKIRIN2,2911);(186,barrett oesophagus,Monocyte_PER2,2255);(186,barrett oesophagus,Colon_RAMP2,2035);(186,barrett oesophagus,Unknown_CD86,1905);(186,barrett oesophagus,Monocyte_RILP,1893);(186,barrett oesophagus,Monocyte_SNX9,1875);(186,barrett oesophagus,Endometrial tissue_TVP23CP2,1868);(186,barrett oesophagus,Monocyte_EIF3G,1705);(186,barrett oesophagus,Endometrial tissue_SMC2,1699);(186,barrett oesophagus,Temporal_cortex_TRIM37,1687);(186,barrett oesophagus,Colorectum_VPS37B,1656);(186,barrett oesophagus,Brain white matter_STON1-GTF2A1L,1647);(186,barrett oesophagus,Endometrial tissue_TMEM159,1635);(186,barrett oesophagus,Endometrial tissue_GLB1,1550);(186,barrett oesophagus,Monocyte_PDHX,1494);(186,barrett oesophagus,Bladder_THEM6,1490);(186,barrett oesophagus,Temporal_cortex_MYADML2,1475);(186,barrett oesophagus,Brain white matter_CLEC12A,1473);(186,barrett oesophagus,Macrophage_MEPE,1424);(186,barrett oesophagus,Endometrial tissue_PODN,1416);(191,dandruff,Whole_blood_AKIRIN2,2911);(191,dandruff,Monocyte_PE R2,2255);(191,dandruff,Colon_RAMP2,2035);(191,dandruff,Unknown_CD86,1905);(191,dandr uff,Monocyte_RILP,1893);(191,dandruff,Monocyte_SNX9,1875);(191,dandruff,Endometrial tissue_TVP23CP2,1868);(191,dandruff ,Monocyte_EIF3G,1705);(191,dandruff,Endometrial tissue_SMC2,1699);(191,dandruff,Temporal_cortex_TRIM37,1687);(191,dandruff,Colorectum_ VPS37B,1656);(191,dandruff,Brain white matter_STON1-GTF2A1L,1647);(191,dandruff,Endometrial tissue_TMEM159,1635);(191,dandruff,Endometrial tissue_GLB 1,1550);(191,dandruff,Monocyte_PDHX,1494);(191,dandruff,Bladder_THEM6,149 0);(191,dandruff,Temporal_cortex_MYADML2,1475);(191,dandruff,Brain white matter_CLEC 12 A, 1473);(191,dandruff ,Macrophage_MEPE, 1424);(191,dandruff,Endometrial tissue_PODN,1416);(200,juvenile idiopathic arthritis,Whole_blood_AKIRIN2,2911);(200,juvenile idiopathic arthritis,Monocyte_PER2,2255);(200,juvenile idiopathic arthritis,Colon_RAMP2,2035);(200,juvenile idiopathic arthritis,Unknown_CD86,1905);(200,juvenile idiopathic arthritis,Monocyte_RILP,1893);(200,juvenile idiopathic arthritis,Monocyte_SNX9, 1875) ;(200,juvenile idiopathic arthritis,Endometrial tissue_TVP23CP2,1868);(200,juvenile idiopathic arthritis,Monocyte_EIF3G,1705);(200,juvenile idiopathic arthritis,Endometrial tissue_SMC2,1699);(200,juvenile idiopathic arthritis,Temporal_cortex_TRIM37,1687);(200,juvenile idiopathic arthritis,Colorectum_VPS37B,1656);(200,juvenile idiopathic arthritis,Brain white matter_STON1-GTF2A1L,1647);(200,juvenile idiopathic arthritis,Endometrial tissue_TMEM159,1635);(200,juvenile idiopathic arthritis,Endometrial tissue GLB1,1550);(200,juvenile idiopathic arthritis,Monocyte_PDHX,1494);(200,juvenile idiopathic arthritis,Bladder_THEM6,1490);(200,juvenile idiopathic arthritis,Temporal_cortex_MYADML2,1475);(200,juvenile idiopathic arthritis,Brain white matter_CLEC12A,1473);(200,juvenile idiopathic arthritis,Macrophage_MEPE,1424);(200,juvenile idiopathic arthritis,Endometrial tissue_PODN,1416);(209,prostate cancer,Whole_blood_AKIRIN2,2911);(209,prostate cancer,Monocyte_PER2,225 5); (209,pro state cancer,Colon_RAMP2,203 5); (209,pro state cancer,Unknown_CD86,1905);(209,prostate cancer,Monocyte_RILP,1893);(209,prostate cancer,Monocyte_SNX9,1875);(209,prostate cancer,Endometrial tissue_TVP23CP2,1868);(209,prostate cancer,Monocyte_EIF3G,1705);(209,prostate cancer,Endometrial tissue_SMC2,1699);(209,prostate cancer,Temporal_cortex_TRIM37,1687);(209,prostate cancer,Colorectum_VPS37B,1656);(209,prostate cancer,Brain white matter_STON1-GTF2A1L,1647);(209,prostate cancer,Endometrial tissue_TMEM159,1635);(209,prostate cancer,Endometrial tissue_GLB1,1550);(209,prostate cancer,Monocyte_PDHX,1494);(209,prostate cancer,Bladder_THEM6,1490);(209,prostate cancer,Temporal_cortex_MYADML2,1475);(209,prostate cancer,Brain white matter_CLEC12A,1473);(209,prostate cancer,Macrophage_MEPE,1424);(209,prostate cancer,Endometrial tissue_PODN, 1416);(210,reflux esophagitis,Whole_blood_AKIRIN2,2911);(210,reflux esophagitis,Monocyte_PER2,2255);(210,reflux esophagitis,Colon_RAMP2,2035);(210,reflux esophagitis,Unknown_CD86,1905);(210,reflux esophagitis,Monocyte_RILP,1893);(210,reflux esophagitis,Monocyte_SNX9,1875);(210,reflux esophagitis,Endometrial tissue_TVP23CP2,1868);(210,reflux esophagitis,Monocyte_EIF3G,1705);(210,reflux esophagitis,Endometrial tissue_SMC2,1699);(210,reflux esophagitis,Temporal_cortex_TRIM37,1687);(210,reflux esophagitis,Colorectum_VPS37B,1656);(210,reflux esophagitis,Brain white matter_STON1-GTF2A1L,1647);(210,reflux esophagitis,Endometrial tissue_TMEM159,1635);(210,reflux esophagitis,Endometrial tissue_GLB 1,1550);(210,reflux esophagitis,Monocyte_PDHX,1494);(210,reflux esophagitis,Bladder_THEM6,1490);(210,reflux esophagitis,Temporal_cortex_MYADML2,1475);(210,reflux esophagitis,Brain white matter_CLEC 12A,1473);(210,reflux esophagitis,Macrophage_MEPE,1424);(210,reflux esophagitis,Endometrial tissue_POD N, 1416); (212, sjogren syndrome,Whole_blood_AKIRIN2,2911);(212,sjogren syndrome,Monocyte_PER2,2255);(212,sjogren syndrome,Colon_RAMP2,2035);(212,sjogren syndrome,Unknown_CD86,1905);(212,sjogren syndrome,Monocyte_RILP,1893);(212,sjogren syndrome,Monocyte_SNX9,1875);(212,sjogren syndrome,Endometrial tissue_TVP23CP2,1868);(212,sjogren syndrome,Monocyte_EIF3G,1705);(212,sjogren syndrome,Endometrial tissue_SMC2,1699);(212,sjogren syndrome,T emporal_cortex_TRIM37,1687); (212, sjogren syndrome,Colorectum_VPS37B,1656);(212,sjogren syndrome,Brain white matter_STON1-GTF2A1L,1647);(212,sjogren syndrome,Endometrial tissue_TMEM159,1635);(212,sjogren syndrome,Endometrial tissue_GLB1,1550);(212,sjogren syndrome,Monocyte_PDHX,1494);(212,sjogren syndrome,Bladder_THEM6,1490);(212,sjogren syndrome,Temporal_cortex_MYADML2,1475);(212,sjogren syndrome,Brain white matter_CLEC12A,1473);(212,sjogren syndrome,Macrophage_MEPE,1424);(212,sjogren syndrome,Endometrial tissue_PODN,1416);(213,stevens-johnson syndrome,Whole_blood_AKIRIN2,2911);(213,stevens-johnson syndrome,Monocyte_PER2,2255);(213,stevens-johnson syndrome,Colon_RAMP2,2035);(213,stevens-johnson syndrome,Unknown_CD86,1905);(213,stevens-johnson syndrome,Monocyte_RILP,1893);(213,stevens-johnson syndrome,Monocyte_SNX9,1875);(213,stevens-johnson syndrome, Endometrial tissue_TVP23CP2,1868);(213,stevens-johnson syndrome,Monocyte_EIF3G,1705);(213,stevens-johnson syndrome, Endometrial tissue_SMC2,1699);(213,stevens-johnson syndrome,Temporal_cortex_TRIM37,1687);(213,stevens-johnson syndrome,Colorectum_VPS37B,1656);(213,stevens-johnson syndrome,Brain white matter_STON1-GTF2A1L,1647);(213,stevens-johnson syndrome, Endometrial tissue_TMEM159,1635);(213,stevens-johnson syndrome,Endometrial tissue_GLB1,1550);(213,stevens-johnson syndrome,Monocyte_PDHX,1494);(213,stevens-johnson syndrome,Bladder_THEM6,1490);(213,stevens-johnson syndrome,Temporal_cortex_MYADML2,1475);(213,stevens-johnson syndrome,Brain white matter_CLEC 12 A, 1473) ;(213, stevens-johnson syndrome,Macrophage_MEPE,1424);(213,stevens-johnson syndrome,Endometrial tissue_PODN,1416)

Reference Signs List

10: search device
100: control unit
1042: search program

**Claims**

1. A method of searching for novel diagnostic biomarkers and/or therapeutic targets, comprising

   performing a topic analysis using a topic model on an omics database derived from two or more biofeatures linked to information indicating a disease name to estimate missing biofeatures for a particular disease, wherein the estimated missing biofeatures suggest the novel diagnostic biomarkers and/or therapeutic targets.

2. The method according to claim 1, wherein the two or more biofeatures are selected from single nucleotide polymorphisms, microbiota, drug targets, transcriptome, proteome and disease biomarkers.

3. The method according to claim 1, wherein the topic model is based on Latent Dirichlet Allocation model.

4. The method according to claim 1, further comprising verifying the reliability of the extracted missing biofeatures.

5. A device of searching for novel diagnostic biomarkers and/or therapeutic targets provided with a control unit, wherein

   the control unit performs a topic analysis using a topic model on an omics database derived from two or more biofeatures linked to information indicating a disease name to estimate missing biofeatures for a particular disease, wherein

the estimated missing biofeatures suggest the novel diagnostic biomarkers and/or therapeutic targets.

6. A program of searching for novel diagnostic biomarkers and/or therapeutic targets that, when executed by a computer, causes the computer to execute a step of performing a topic analysis using a topic model on an omics database derived from two or more biofeatures linked to information indicating a disease name to estimate missing biofeatures for a particular disease, wherein
the estimated missing biofeatures suggest the novel diagnostic biomarkers and/or therapeutic targets.

Fig. 1

omics database (biofeature: feature)

SNPs database
data identifier/information indicating disease name/omics data (SNP data: element)

microbiota database
data identifier/information indicating disease name/omics data (microbial data: element)

drug target database
data identifier/information indicating disease name/drug determination information/omics data (drug target data: element)

disease-associated tissue/organ transcriptome database
data identifier/ information indicating disease name/omics data (transcriptome data: element)

disease-associated tissue/organ proteome database
data identifier/ information indicating disease name/omics data (proteome data: element)

disease biomarker database
data identifier/ information indicating disease name/omics data (disease biomarker data: element)

.
.
.

MD

Fig. 2

Fig. 3

Fig. 4

```
                    ( start )
                        │
                        ▼
  ┌──────────────────────────────────────────────┐
  │  accept a selection request for an omics      │   S11
  │  database and acquire a corresponding database│
  └──────────────────────────────────────────────┘
                        │
                        ▼
  ┌──────────────────────────────────────────────┐
  │ estimate missing biofeatures for a particular │   S12
  │ disease                                        │
  └──────────────────────────────────────────────┘
                        │
                        ▼
  ┌──────────────────────────────────────────────┐
  │         output the missing biofeatures         │   S13
  └──────────────────────────────────────────────┘
                        │
                        ▼
                    ( end )
```

Fig. 5

## Fig. 6-1

Fig. 6-2

Fig. 6-3

Fig. 6-4

Fig. 6-5

Fig. 6-6

Fig. 6-7

Fig. 6-8

Fig. 6-9

## INTERNATIONAL SEARCH REPORT

| International application No. |
|---|
| **PCT/JP2022/020744** |

**A. CLASSIFICATION OF SUBJECT MATTER**

*G16B 40/30*(2019.01)i
FI: G16B40/30

According to International Patent Classification (IPC) or to both national classification and IPC

**B. FIELDS SEARCHED**

Minimum documentation searched (classification system followed by classification symbols)

G16B40/30

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); PubMed

**C. DOCUMENTS CONSIDERED TO BE RELEVANT**

| Category* | Citation of document, with indication, where appropriate, of the relevant passages | Relevant to claim No. |
|---|---|---|
| A | US 2016/0225048 A1 (FAIR ISAAC CORPORATION) 04 August 2016 (2016-08-04) <br> entire text, all drawings | 1-6 |
| A | 松谷 太郎　外3名, トピックモデルを用いたがんゲノムの変異シグネチャー解析, 電子情報通信学会技術研究報告, 16 June 2017, vol. 117, no. 110, pp. 105-110, ISSN: 0913-5685 <br> entire text, all drawings, (MATSUTANI, Taro et al. IEICE Technical Report.), non-official translation (Mutational signature analysis of cancer genomes using topic models) | 1-6 |
| A | LI, Ning. HE, Huixin. Topic Evolution Analysis for Omics Data Integration in Cancers. Frontiers in Cell and Developmental Biology. 07 April 2021, pp. 1-8 <br> entire text, all drawings | 1-6 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 August 2022** | **16 August 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2022/020744**

| Patent document cited in search report | Publication date (day/month/year) | Patent family member(s) | Publication date (day/month/year) |
|---|---|---|---|
| US 2016/0225048 A1 | 04 August 2016 | WO 2016/126867 A1 | |
| | | EP 3254248 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GYORI, B.M et al.** *Molecular systems biology,* 2017, vol. 13, 954
- **HAGGERTY, R.A. ; PURVIS, J.E.** *Molecular systems biology,* 2017, vol. 13, 958
- **JANSSENS et al.** Disbiome database: linking the microbiome to disease. *BMC Microbiol,* 2018, vol. 18, 50
- **LOVE et al.** Moderated estimation of fold change and dispersion for RNA-seq data with DESeq2. *Genome biology,* 2014, vol. 15, 550
- **LAMY et al.** *Stud Health Technol Inform,* 2015, vol. 210, 924-928
- **BLEI et al.** *Journal of Machine Learning Research,* 2003, vol. 3, 993-1022